(19) Europäisches Patentamt / European Patent Office / Office européen des brevets

(11) **EP 2 594 589 A1**

(12) **EUROPEAN PATENT APPLICATION**
published in accordance with Art. 153(4) EPC

(43) Date of publication:
**22.05.2013 Bulletin 2013/21**

(51) Int Cl.:
*C07K 16/28* (2006.01)   *A61K 39/395* (2006.01)
*A61K 45/00* (2006.01)   *C12N 5/10* (2006.01)
*C12N 15/09* (2006.01)   *C12P 21/08* (2006.01)

(21) Application number: **11792536.2**

(22) Date of filing: **09.06.2011**

(86) International application number:
**PCT/JP2011/063294**

(87) International publication number:
**WO 2011/155579 (15.12.2011 Gazette 2011/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.06.2010 US 353430 P**

(71) Applicants:
• **Sapporo Medical University**
  **Hokkaido 060-0061 (JP)**
• **Kyowa Hakko Kirin Co., Ltd.**
  **Tokyo 100-8185 (JP)**

(72) Inventors:
• **YAMAGUCHI, Miki**
  **Sapporo-shi**
  **Hokkaido 060-8556 (JP)**

• **KATO, Kazunori**
  **Sapporo-shi**
  **Hokkaido 060-8556 (JP)**
• **HAMADA, Hirofumi**
  **Sapporo-shi**
  **Hokkaido 060-8556 (JP)**
• **NAKAMURA, Kazuyasu**
  **Tokyo 100-8185 (JP)**
• **SUGIMOTO, Yoshiyuki**
  **Tokyo 100-8185 (JP)**
• **KUBOTA, Tsuguo**
  **Tokyo 100-8185 (JP)**
• **IKEDA, Masahiro**
  **Tokyo 100-8185 (JP)**

(74) Representative: **HOFFMANN EITLE**
**Patent- und Rechtsanwälte**
**Arabellastrasse 4**
**81925 München (DE)**

(54) **ANTI-Trop-2 ANTIBODY**

(57) The present invention relates to a monoclonal antibody or an antibody fragment thereof, which binds to the extracellular region of human Trop-2 with high affinity and exhibits high ADCC activity and high antitumor activity; a hybridoma which produces the antibody; a DNA which encodes the antibody; a vector which comprises the DNA; a transformant obtainable by introducing the vector; a process for producing an antibody or an antibody fragment thereof using the hybridoma or the transformant; and a therapeutic agent or a diagnostic agent using the antibody fragment thereof.

EP 2 594 589 A1

**Description**

Technical Field

The present invention relates to a monoclonal antibody or an antibody fragment thereof, which binds to the extracellular region of human Trop-2 with high affinity and exhibits high antibody-dependent cellular cytotoxicity (hereinafter referred to as "ADCC activity") and high antitumor activity; a hybridoma which produces the antibody; a DNA which encodes the antibody; a vector which comprises the DNA; a transformant obtainable by introducing the vector; a process for producing an antibody or an antibody fragment thereof using the hybridoma or the transformant; and a therapeutic agent or a diagnostic agent using the antibody or the antibody fragment thereof.

Background Art

Trop-2 is also known as Tumor associated calcium signal transducer 2 (TACSTD2), GA733-1, gp50 and T16 and is a type I transmembrane protein comprising 323 amino acids. Trop-2 was found as a protein recognized by monoclonal antibodies 162-25.3 and 162-46.2 which react with human trophoblast cells (Non-patent Literature 1).

The gene of Trop-2 was cloned in 1989 (Non-patent Literature 2). DNA sequences, amino acid sequences and three-dimensional structures of Trop-2 are disclosed in a public database and therefore can be referred to accession numbers such as NP_002344 and NM_002353 (NCBI). The amino acid sequence of Trop-2 comprises a signal sequence consisting of N-terminal 26 amino acids, an extracellular region of 248 amino acids, a transmembrane domain consisting of 23 amino acids and an intracellular domain consisting of 26 amino acids. Although the theoretically calculated molecular weight of Trop-2 is 35 kDa, it is known that the actual molecular weight of Trop-2 increased by 10 kDa or so due to the addition of sugar chains.

Since Trop-2 has the same number and location of cysteine residues as the family molecule, EpCAM (also known as Trop-1), their structures are estimated to be similar to each other (Non-patent Literature 2). The extracellular region of EpCAM comprises three domains: two cysteine-rich domains at the N-terminus and one cysteine-poor domain at the C terminus (Non-patent Literature 15). Regarding EpCAM, it is known that first domain from the N-terminus is involved in the mutual adhesion of EpCAMs between cells and second domain is involved in the interaction of EpCAMs on the same cell membrane (Non-patent Literature 15).

Trop-2 has been known to be expressed on a variety of cancers and the correlation of Trop-2 expression with poor prognosis has also been reported (Non-patent Literatures 3 to 9). Also, the involvement of Trop-2 in anchorage-independent growth was reported in colon cancer (Non-patent Literature 10). It has been reported that the expression of Trop-2 in normal tissues are limited to epithelial cells in several tissues.

Since 162-25.3 and 162-46.2 were reported, several monoclonal antibodies against Trop-2 have been established (Non-patent Literatures 11 to 13; Patent Literatures 1 to 3). Some anti-Trop-2 monoclonal antibodies such as 77220 are commercially available as reagents. Some of these established anti-Trop-2 monoclonal antibodies are being under investigation for treating cancers.

Since one of anti-Trop-2 monoclonal antibodies, BR110 (Patent Literature 1), is known to bind to Trop-2 on the cell surface and internalize within the cells, a cytotoxic drug-conjugated antibody was examined and reported. It was disclosed that the above conjugate shows cellular cytotoxicity for cancer cell lines such as H2987, H3396, H3619 and MCF-7.

Since RS7 (Patent Literature 2; Non-patent Literatures 11, 14 and 16), one of anti-Trop-2 monoclonal antibodies, binds to Trop-2 on the cell surface and internalizes within the cells, the example of cytotoxic drug-conjugated antibody has been reported. It has been disclosed that the conjugate of the humanized antibody hRS7 of the above antibody with a radioactive isotope ($^{131}$I, $^{131}$I-IMR-R4) shows antitumor activity in a xenograft model of human cancer cell using a breast cancer cell line.

Anti-Trop-2 monoclonal antibodies, AR47A6.4.2 (Patent Literature 3) and AR52A301.5 (Patent Literature 4) are antibodies which specifically injure Trop-2-expressing cancer cell as a target cell to exhibit effects. Patent Literatures 3 and 4 disclose that these antibodies kill cancer cells directly.

It has also been disclosed that huAR47A6.4.2 (Patent Literature 5) which is the humanized antibody of AR47A6.4.2 inhibits the activation of intracellular extracellular-signal regulated kinase (ERK). Patent Literature 5 discloses that huAR47A6.4.2 showed complement-dependent cytotoxicity (hereinafter referred to as "CDC activity") on Trop-2-positive human cell lines when rabbit serum having a high complement titer was used.

[Citation List]

[Patent Literature]

[Patent Literature 1] WO97/14796
[Patent Literature 2] WO2003/074566
[Patent Literature 3] WO2007/095748
[Patent Literature 4] WO2007/095749
[Patent Literature 5] WO2008/144891

[Non-patent Literature]

[Non-patent Literature 1] Proc. Natl. Acad. Sci. USA, 8, 5147 (1981)
[Non-patent Literature 2] Int. J. Cancer, 62, 610 (1995)
[Non-patent Literature 3] Mod. Pathol., 21, 186 (2008)
[Non-patent Literature 4] Clin. Cancer Res., 12, 3057 (2006)
[Non-patent Literature 5] Br. J. Cancer, 99, 1290 (2008)
[Non-patent Literature 6] J. Clin. Pathol., 62, 152 (2009)
[Non-patent Literature 7] Eur. J. Cancer, 46, 944 (2010)
[Non-patent Literature 8] Cancer Res., 62, 5325 (2002)
[Non-patent Literature 9] Lab. Invest. 84, 320 (2004)
[Non-patent Literature 10] Mol. Cancer Ther., 7, 280 (2008)
[Non-patent Literature 11] Int. J. Cancer, 39, 297 (1987)
[Non-patent Literature 12] Cancer Res., 50, 1330 (1990)
[Non-patent Literature 13] Hybridoma, 11, 539 (1992)
[Non-patent Literature 14] Breast Cancer Res. Treat., 84, 173, (2004)
[Non-patent Literature 15] Mol. Cell. Biol., 21, 2570 (2001)
[Non-patent Literature 16] Clin. Cancer Res., 17, 3157 (2011)

Disclosure of Invention

Problems to be Solved by the Invention

However, BR110 disclosed in Patent Literature 1 has no cellular cytotoxicity when it is not conjugated with a cytotoxic drug. In addition, when RS7 disclosed in Patent Literature 2 and Non-patent Literatures 11, 14 and 16 is not conjugated with a cytotoxic drug, it has no cellular cytotoxicity. There is no report on ADCC activity of huAR47A6.4.2 disclosed in Patent Literature 5.

In addition, there is no report on an anti-Trop-2 monoclonal antibody which binds to the extracellular region of human Trop-2 with high affinity. An anti-Trop-2 monoclonal antibody which binds to the extracellular region of human Trop-2 is thought to have high ADCC activity and antitumor activity for human Trop-2 positive human cell lines.

Therefore, the present invention has been made in an effort to a monoclonal antibody or an antibody fragment thereof, which binds to the extracellular region of human Trop-2 with high affinity; a hybridoma which produces the antibody; a DNA which encodes the antibody; a vector which comprises the DNA; a transformant obtainable by introducing the vector; a process for producing an antibody or an antibody fragment thereof using the hybridoma or the transformant; or a therapeutic agent or a diagnostic agent using the antibody or the antibody fragment thereof.

Means for Solving the Problems

The present invention relates to the followings.

1. A monoclonal antibody or an antibody fragment thereof against human Trop-2, which binds to at least domain I in an extracellular region of human Trop-2.
2. The monoclonal antibody or the antibody fragment thereof described in the above 1, which binds to at least one amino acid in amino acids at positions 34 to 72 in the amino acid sequence represented by SEQ ID NO:1.

3. The monoclonal antibody or the antibody fragment thereof described in the above 1 or 2, which has a dissociation constant ($K_D$) of $5 \times 10^{-10}$M or less against an antigen of an antibody.

4. The monoclonal antibody or the antibody fragment thereof described in the above any one of 1 to 3, which has high ADCC activity and antitumor activity.

5. The monoclonal antibody or the antibody fragment thereof described in the above any one of 1 to 4, wherein complementary determining regions (hereinafter referred as "CDR") 1 to 3 of a heavy chain (hereinafter referred as "H chain") of the antibody comprise the amino acid sequences represented by SEQ ID NOs:13 to 15, respectively; and CDR1 to 3 of a light chain (hereinafter referred as "L chain") of the antibody comprise the amino acid sequences represented by SEQ ID NOs:16 to 18, respectively.

6. A monoclonal antibody or an antibody fragment thereof, which binds to an epitope which is the same as an epitope in an extracellular region of Trop-2 to which a monoclonal antibody comprising an H chain variable region (hereinafter referred to as "VH") comprising the amino acid sequence represented by SEQ ID NO:10 and an L chain variable region (hereinafter referred to as "VL") comprising the amino acid sequence represented by SEQ ID NO:12 binds.

7. A monoclonal antibody or an antibody fragment thereof, which binds to an extracellular region of Trop-2 while competing with the antibody described in the above 5 or 6.

8. The monoclonal antibody or the antibody fragment thereof described in any one of the above 1 to 7, which is a recombinant antibody.

9. The monoclonal antibody or the antibody fragment thereof described in the above 8, which is a recombinant antibody selected from a human chimeric antibody, a humanized antibody and a human antibody.

10. The monoclonal antibody or the antibody fragment thereof described in the above 9, which is a humanized antibody and comprises the following (a) VH and (b) VL:

(a) VH which comprises the amino acid sequence represented by SEQ ID NO:24 or an amino acid sequence in which at least one modification in amino acid modifications for substituting Ala at position 9 with Pro, Lys at position 12 with Val, Val at position 20 with Ile, Arg at position 38 with Lys, Met at position 48 with Ile, Arg at position 67 with Lys, Val at position 68 with Ala, Ile at position 70 with Leu, Tyr at position 95 with Phe and Val at position 112 with Leu is introduced in the amino acid sequence represented by SEQ ID NO:24,

(b) VL which comprises the amino acid sequence represented by SEQ ID NO:26 or an amino acid sequence in which at least one modification in amino acid modifications for substituting Leu at position 15 with Ala, Ala at position 19 with Val, Ile at position 21 with Met and Leu at position 84 with Val is introduced in the amino acid sequence represented by SEQ ID NO:26.

11. The monoclonal antibody or the antibody fragment thereof described in the above 10, which is selected from the following (1) to (5):

(1) a humanized antibody which comprises at least one of VH of the antibody comprising the amino acid sequence represented by SEQ ID NO:28 and VL of the antibody comprising the amino acid sequence represented by SEQ ID NO:26,

(2) a humanized antibody which comprises at least one of VH of the antibody comprising the amino acid sequence represented by SEQ ID NO:30 and VL of the antibody comprising the amino acid sequence represented by SEQ ID NO:26,

(3) a humanized antibody which comprises at least one of VH of the antibody comprising the amino acid sequence represented by SEQ ID NO:32 and VL of the antibody comprising the amino acid sequence represented by SEQ ID NO:26,

(4) a humanized antibody which comprises at least one of VH of the antibody comprising the amino acid sequence represented by SEQ ID NO:34 and VL of the antibody comprising the amino acid sequence represented by SEQ ID NO:36,

(5) a humanized antibody which comprises at least one of VH of the antibody comprising the amino acid sequence represented by SEQ ID NO:34 and VL of the antibody comprising the amino acid sequence represented by SEQ ID NO:38.

12. The antibody fragment described in any one of the above 1 to 11, which is an antibody fragment selected from Fab, Fab', F(ab')$_2$, a single chain antibody (scFv), a dimerized V region (diabody), a disulfide stabilized V region (dsFv) and a peptide comprising CDR.

13. A DNA which encodes the monoclonal antibody or the antibody fragment described in any one of the above 1 to 12.

14. A recombinant vector comprising the DNA described in the above 13.

15. A transformant obtainable by introducing the recombinant vector described in the above 14 into a host cell.

16. A process for producing the monoclonal antibody or the antibody fragment thereof described in any one of the above 1 to 12, which comprises culturing the transformant described in the above 14 in a medium to thereby form and accumulate the antibody or the antibody fragment thereof described in any one of the above 1 to 12 in culture, and recovering the antibody or the antibody fragment thereof from the culture.

17. A reagent for detecting or measuring human Trop-2, which comprises the monoclonal antibody or the antibody fragment thereof described in any one of the above 1 to 12.

18. A diagnostic agent for a disease relating to human Trop-2-positive cells, which comprises the monoclonal antibody or the antibody fragment thereof described in any one of the above 1 to 12 as an active ingredient.

19. The diagnostic agent described in the above 18, wherein the disease relating to human Trop-2-positive cells is a cancer.

20. A therapeutic agent for a disease relating to human Trop-2-positive cells, which comprises the monoclonal antibody or the antibody fragment thereof described in any one of the above 1 to 12 as an active ingredient.

21. The therapeutic agent described in the above 20, wherein the disease relating to human Trop-2-positive cells is a cancer.

22. A method for diagnosing a disease relating to human Trop-2-positive cells, which comprises using the monoclonal antibody or the antibody fragment thereof described in any one of the above 1 to 12 to detect or measure human Trop-2-positive cells.

23. A method for diagnosing a disease relating to human Trop-2-positive cells, which comprises using the monoclonal antibody or the antibody fragment thereof described in any one of the above 1 to 12 to detect or measure human Trop-2.

24. The diagnosing method described in the above 22 or 23, wherein the disease relating to human Trop-2-positive cells is a cancer.

25. Use of the monoclonal antibody or the antibody fragment thereof described in any one of the above 1 to 12 for manufacture of a diagnostic agent for a disease relating to human Trop-2-positive cells.

26. The use of the monoclonal antibody or the antibody fragment thereof described in the above 25, wherein the disease relating to human Trop-2-positive cells is a cancer.

27. Use of the monoclonal antibody or the antibody fragment thereof described in any one of the above 1 to 12 for manufacture of a therapeutic agent for a disease relating to human Trop-2-positive cells.

28. The use of the monoclonal antibody or the antibody fragment thereof described in the above 27, wherein the disease relating to human Trop-2-positive cells is a cancer.

Effects of Invention

A monoclonal antibody or an antibody fragment thereof against human Trop-2 in the present invention can recognize specifically at least domain I in the extracellular region of human Trop-2 and bind to the extracellular region with high affinity. Accordingly, a monoclonal antibody or an antibody fragment thereof in the present invention can have high ADCC activity and antitumor activity on human Trop-2-positive cells. Therefore, a monoclonal antibody or an antibody fragment thereof against human Trop-2 in the present invention is very useful for the treatment and diagnosis of diseases relating to human Trop-2-positive cells.

The present invention can provide a hybridoma which produces the antibody; a DNA which encodes the antibody; a vector which comprises the DNA; a transformant obtainable by introducing the vector; a process for producing an antibody or an antibody fragment thereof using the hybridoma or the transformant; and a therapeutic agent or a diagnostic agent using the antibody or the antibody fragment thereof.

Brief Description of the Drawings

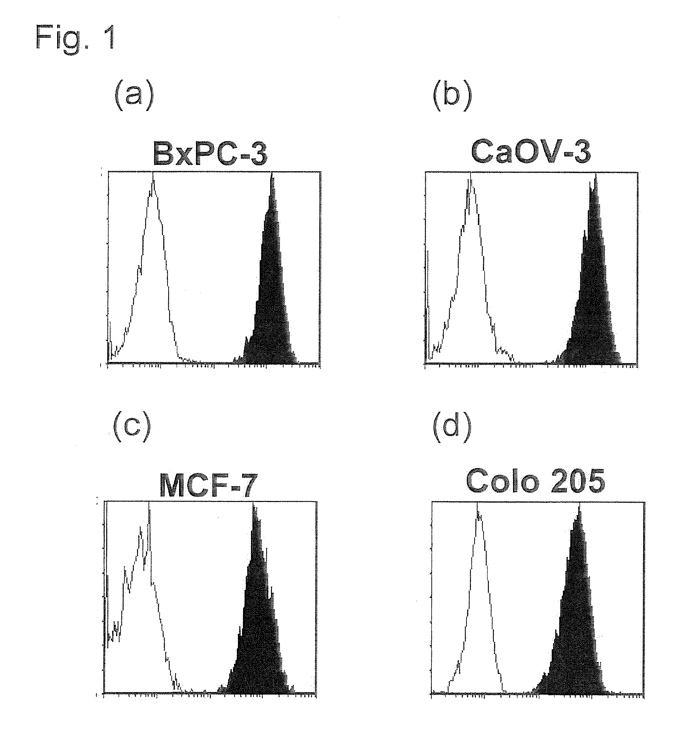

[Fig. 1(a)] to [Fig. 1(d)] Fig. 1(a) shows results obtained by analyzing the reactivity of KM4097 to human pancreatic carcinoma cell line BxPC-3 using FCM. Fig. 1(b) shows results obtained by analyzing the reactivity of KM4097 to human ovarian cancer cell line CaOV-3 using FCM. Fig. 1(c) shows results obtained by analyzing the reactivity of KM4097 to human breast cancer cell line MCF-7 using FCM. Fig. 1(d) shows results obtained by analyzing the reactivity of KM4097 to human colon cancer cell line Colo205 using FCM. In Fig. 1(a) to Fig. 1(d), the black area represents histograms when 10 μg/ml of KM4097 was added and the white area represents histograms when a negative control antibody was used.

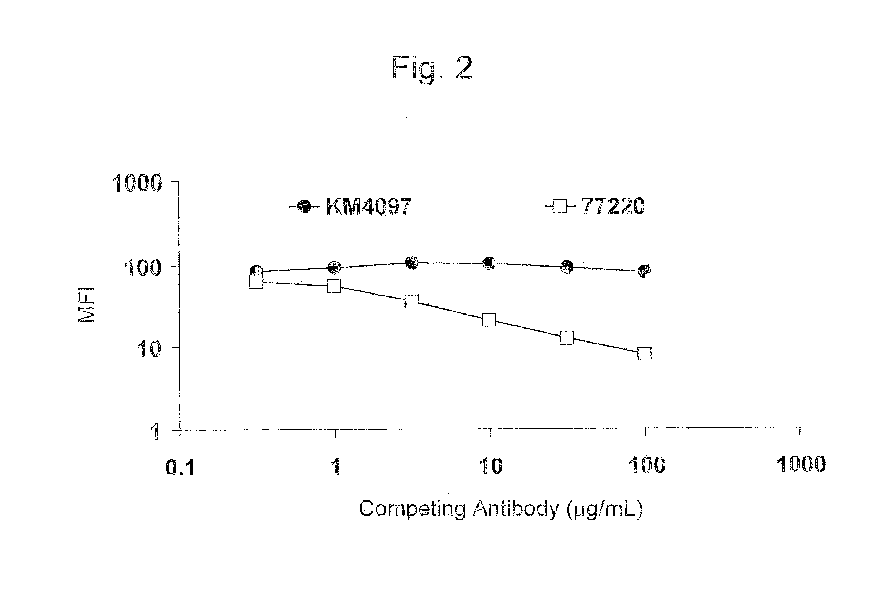

[Fig. 2] Fig. 2 shows results obtained by analyzing the antagonism of an anti-Trop-2 mouse monoclonal antibody for the binding of cAR47A6.4.2 to human pancreatic carcinoma cell line BxPC-3 using FCM. The abscissa shows the concentration of each antibody and the ordinate shows the MFI value which represents the binding of cAR47A6.4.2. ● represents the MFI value in the coexistence of KM4097 and □ represents the MFI value in the coexistence of 77220.

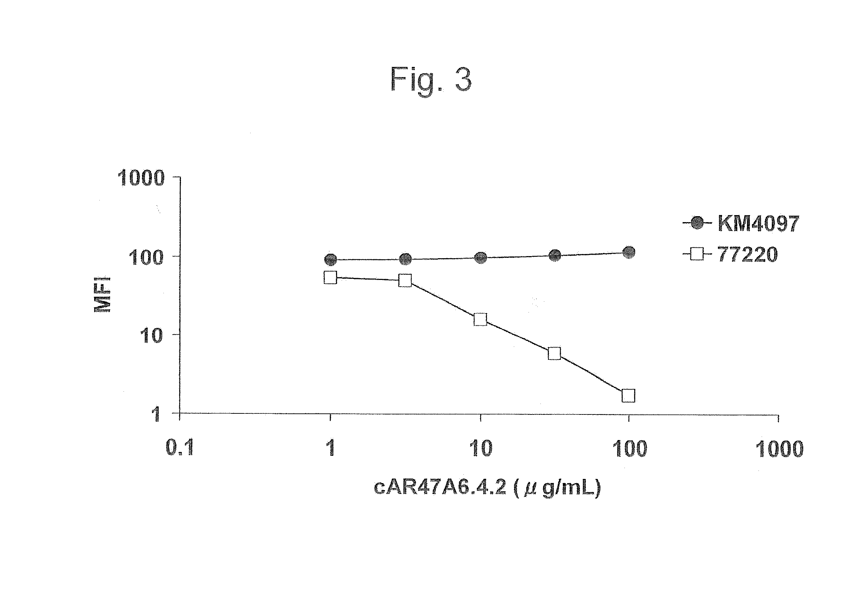

[Fig. 3] Fig. 3 shows results obtained by analyzing the antagonism of cAR47A6.4.2 for the binding of each anti-Trop-2 mouse monoclonal antibody to human pancreatic carcinoma cell line BxPC-3 using FCM. The abscissa shows the concentration of cAR47A6.4.2 and the ordinate shows the MFI value which represents the binding of each competitive antibody. ● represents the MFI value in the coexistence of KM4097 and □ represents the MFI value in the coexistence of 77220.

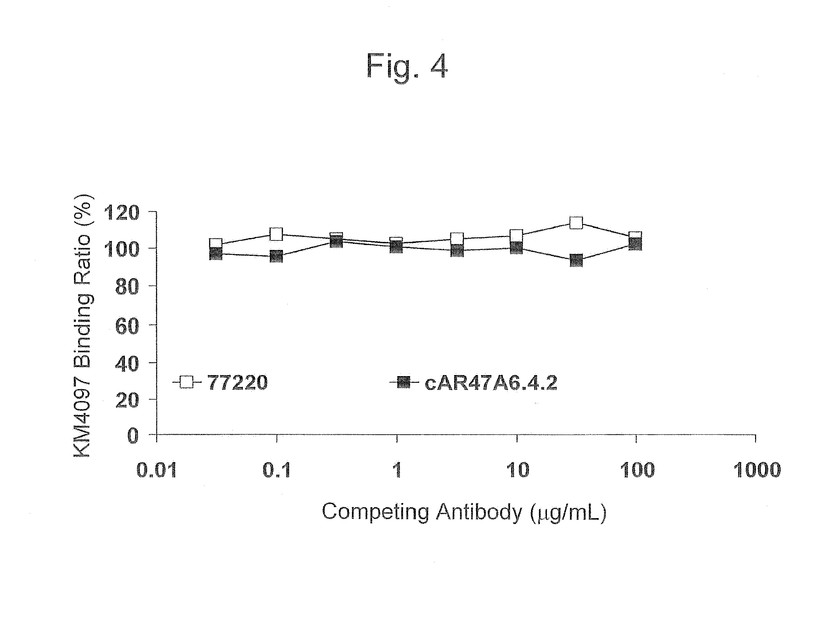

[Fig. 4] Fig. 4 shows obtained by analyzing the antagonism of an anti-Trop-2 monoclonal antibody for the binding of KM4097 to recombinant Trop-2 using binding ELISA. The abscissa shows the concentration of each antibody and the ordinate shows binding rate (%) of KM4097. ■ represents the binding rate (%) of KM4097 in the coexistence of cAR47A6.4.2 and □ represents the binding rate (%) of KM4097 in the coexistence of 77220.

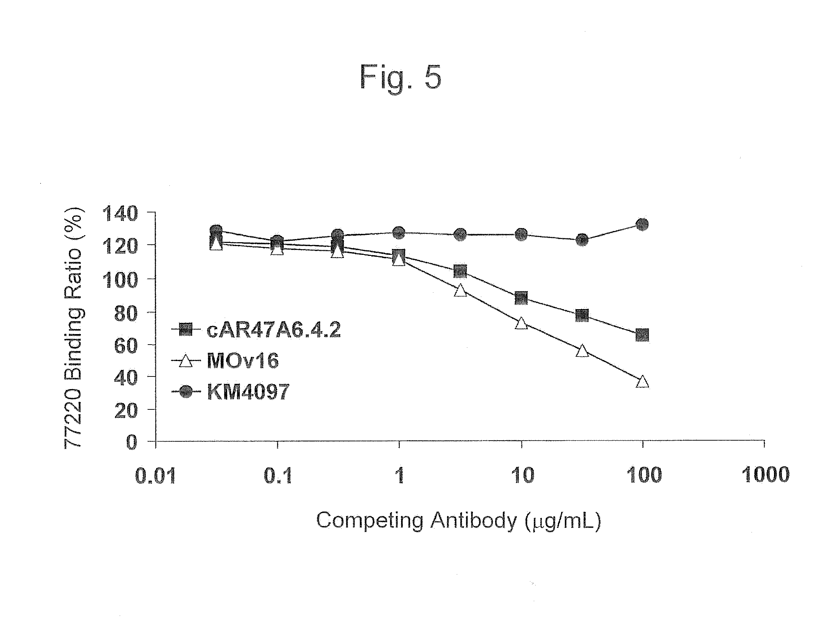

[Fig. 5] Fig. 5 shows results obtained by analyzing the antagonism of an anti-Trop-2 monoclonal antibody for the binding of 77220 to recombinant Trop-2 using binding ELISA. The abscissa shows the concentration of each antibody and the ordinate shows binding rate (%) of 77220. ●, ■ and ∆ represent the binding rate (%) of 77220 in the coexistence of KM4097, cAR47A6.4.2 and MOv16, respectively.

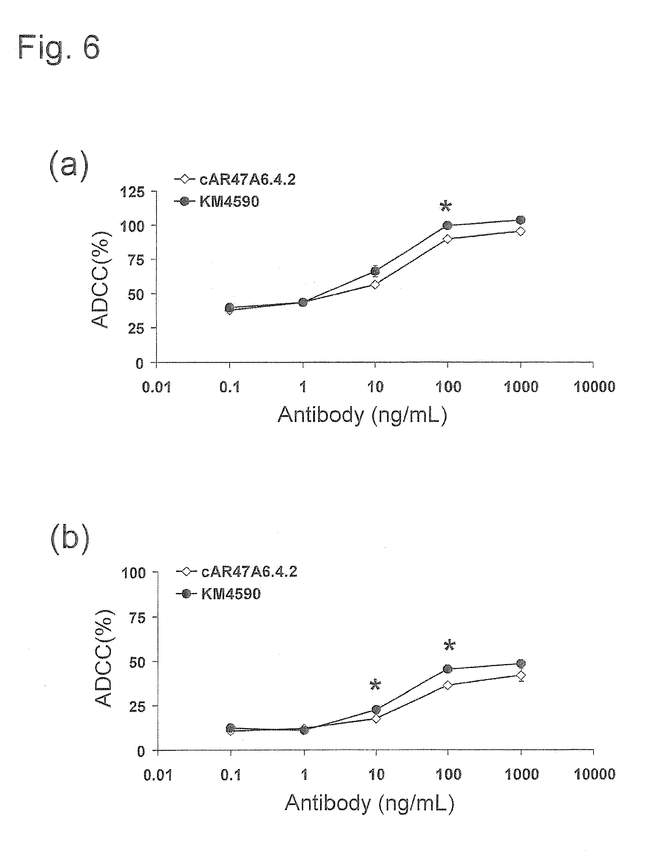

[Fig. 6(a)] and [Fig. 6(b)] Fig. 6(a) and Fig. 6(b) show antibody-dependent cell cytotoxicity (ADCC activity) of an anti-Trop-2 antibody for human breast cancer cell line MCF-7 cells. Fig. 6(a) shows the result of donor 1 and Fig. 6(b) shows the result of donor 2. The abscissa shows the concentration of each antibody and the ordinate shows cellular cytotoxicity rate (%). ● and ◇ represent KM4590 and cAR47A6.4.2, respectively. The results are expressed as a mean value ± standard error of three wells. * means significantly different (p<0.05) from cAR47A6.4.2 according to Student's t-test.

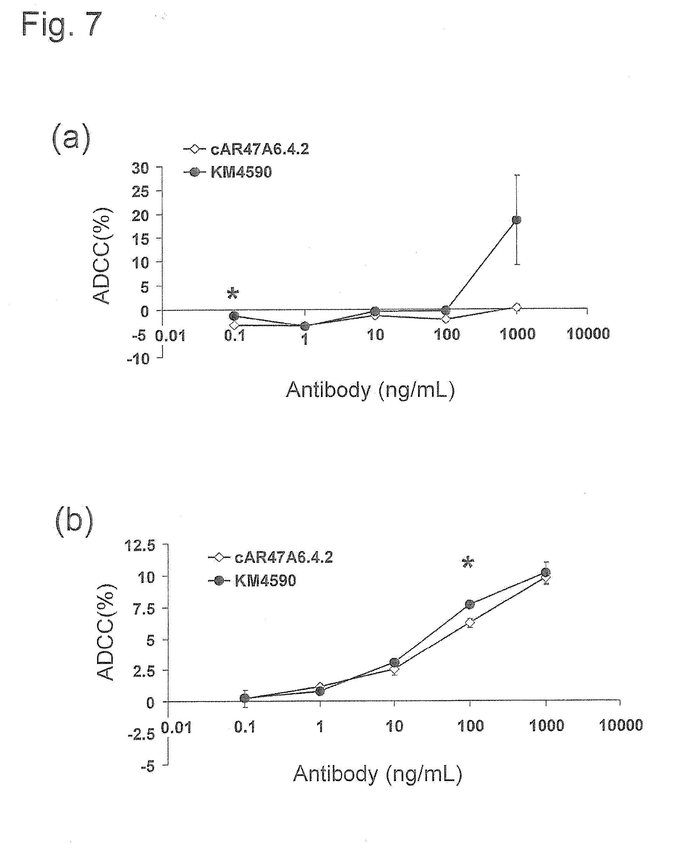

[Fig. 7(a)] and [Fig. 7(b)] Fig. 7(a) and Fig.7(b) show antibody-dependent cell cytotoxicity (ADCC activity) of an anti-Trop-2 antibody for human colon cancer cell line Colo205 cells. Fig. 7(a) shows the result of donor 1 and Fig. 7(b) shows the result of donor 2. The abscissa shows the concentration of each antibody and the ordinate shows cellular cytotoxicity rate (%). ● and ◇ represent KM4590 and cAR47A6.4.2, respectively. The results are expressed as a mean value ± standard error of three wells. * means significantly different (p < 0.05) from cAR47A6.4.2 according to Student's t-test.

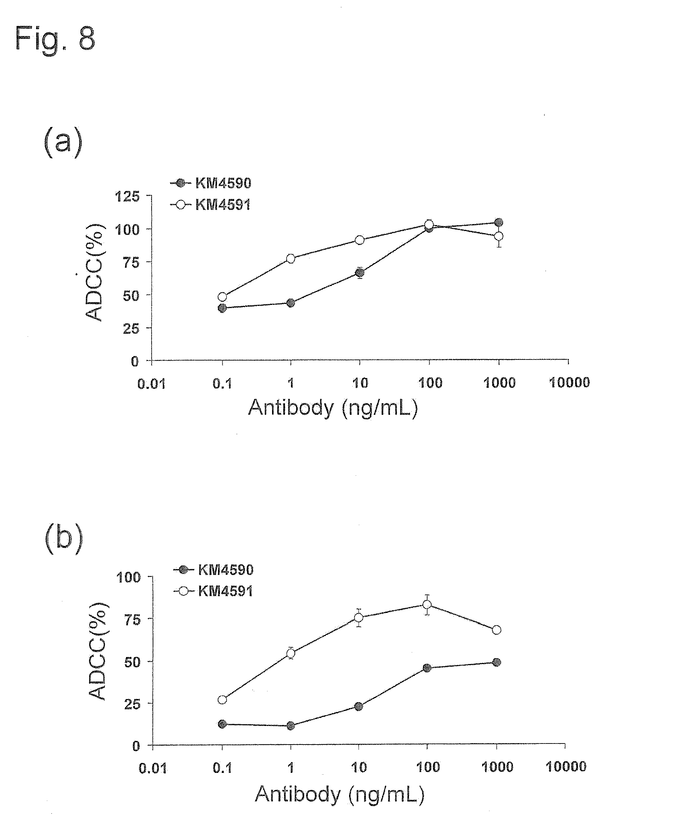

[Fig. 8(a)] and [Fig. 8(b)] Fig. 8(a) and Fig. 8(b) show antibody-dependent cell cytotoxicity (ADCC activity) of an anti-Trop-2 antibody for human breast cancer cell line MCF-7 cells. Fig. 8(a) shows the result of donor 1 and Fig. 8(b) shows the result of donor 2. The abscissa shows the concentration of each antibody and the ordinate shows cellular cytotoxicity rate (%). ● and ○ represent KM4590 and KM4591, respectively. The results are shown as a mean value ± standard error of three wells.

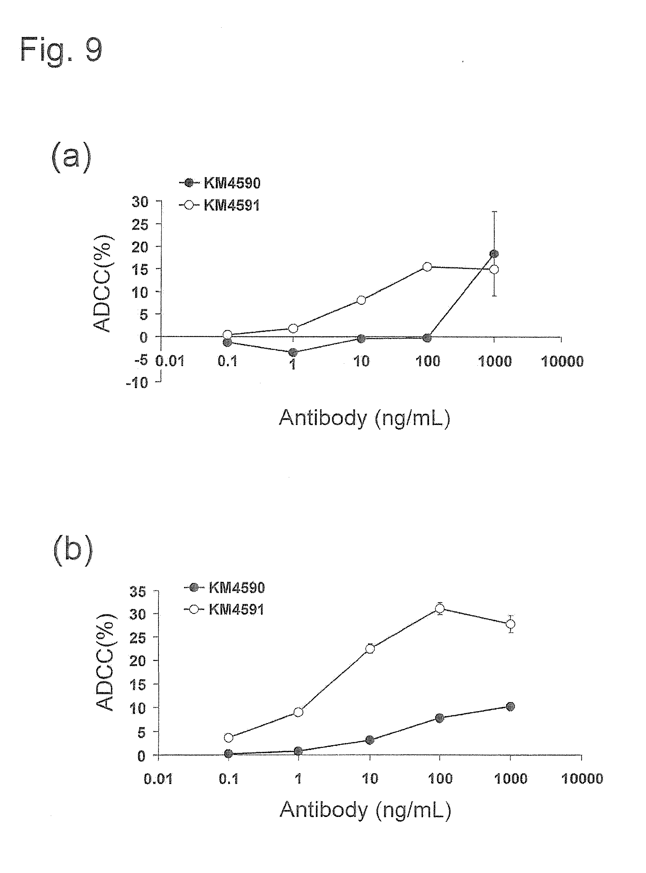

[Fig. 9(a)] and [Fig. 9(b)] Fig. 9 (a) and 9 (b) show antibody-dependent cell cytotoxicity (ADCC activity) for human colon cancer cell line Colo205 cells. Fig. 9 (a) shows the result of donor 1 and Fig. 9 (b) shows the result of donor 2. The abscissa shows the concentration of each antibody and the ordinate shows cellular cytotoxicity rate (%). ● and ○ represent KM4590 and KM4591, respectively. The results are expressed shown as a mean value ± standard error of three wells.

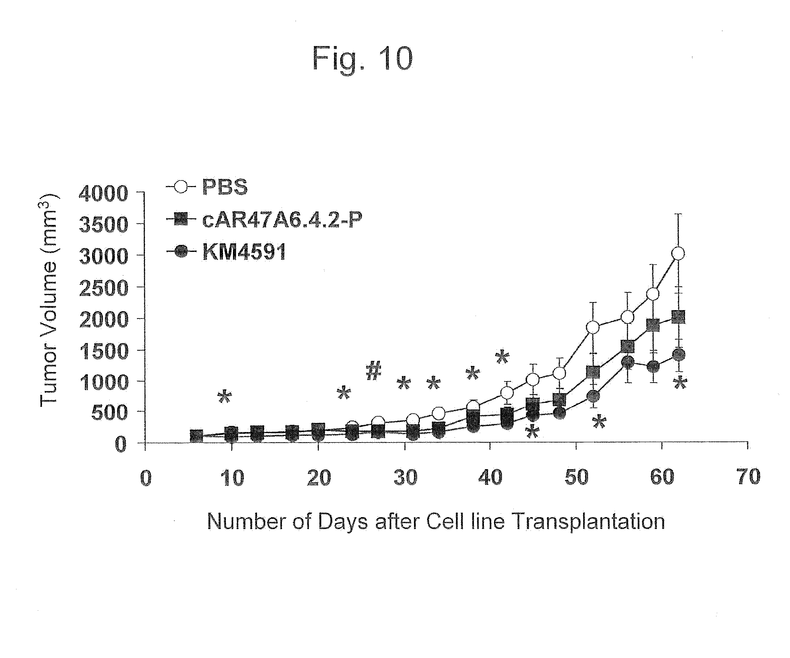

[Fig. 10] Fig. 10 shows antitumor effects of an anti-Trop-2 chimeric antibody on a human pancreatic carcinoma cell line BxPC-3 xenograft mouse. The ordinate shows tumor volume and the abscissa shows the number of days after cell plantation. ○, ■ and ● represent the PBS administration group, the cAR47A6.4.2-P administration group and the KM4591 administration group, respectively. The results are shown as a mean value ± standard error of six animals. * means that the KM4591 administration group is significantly different (p<0.05) from the PBS administration group according to Student's t-test. # means that the cAR47A6.4.2-P administration group is significantly different (p<0.05) from the PBS administration group according to Student's t-test.

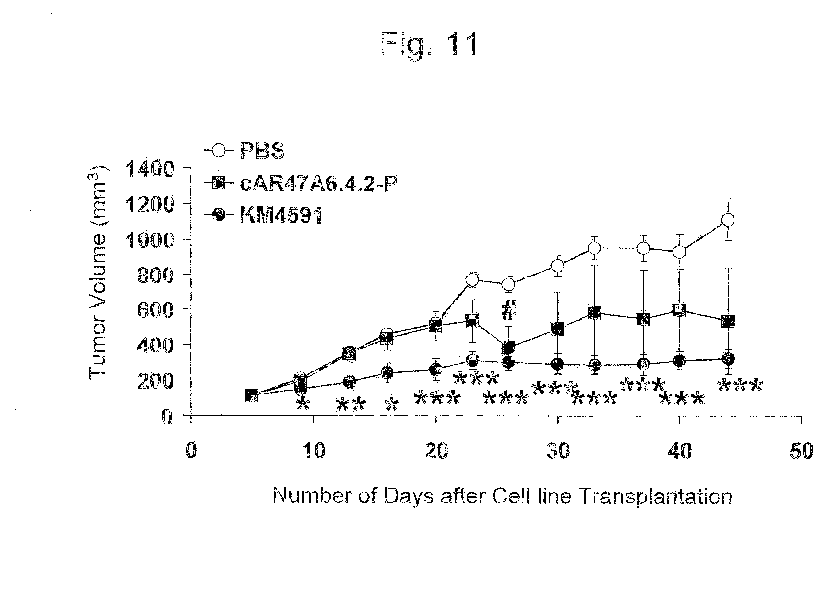

[Fig. 11] Fig. 11 shows antitumor effects of an anti-Trop-2 chimeric antibody on a human colon cancer cell line Colo205 xenograft mouse. The ordinate shows tumor volume and the abscissa shows the number of days after cell transplantation. ○, ■ and ● represent the PBS administration group, the cAR47A6.4.2-P administration group and the KM4591 administration group, respectively. The results are shown as a mean value ± standard error of five animals. *, ** and *** mean that the KM4591 administration group is significantly different (p<0.05, p<0.01 and p<0.001, respectively) from the PBS administration group according to Student's t-test. # means that the cAR47A6.4.2-P administration group is significantly different (p<0.05) from the PBS administration group according to Student's t-test.

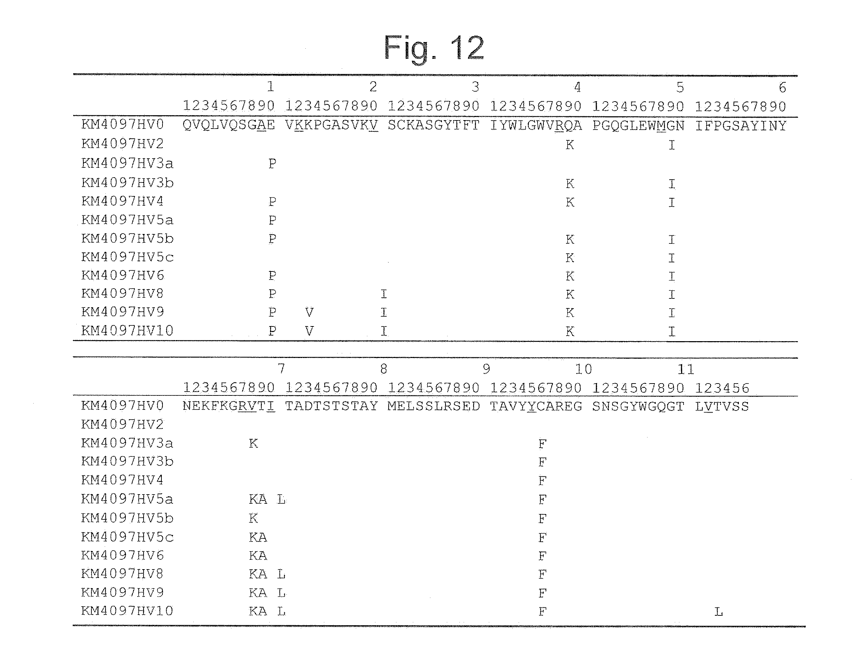

[Fig. 12] Fig. 12 shows amino acid sequences of HV0, HV2, HV3a, HV3b, HV4, HV5a, HV5b, HV5c, HV6, HV8, HV9 and HV10 which are an H chain variable regions of designed KM4097 humanized antibody.

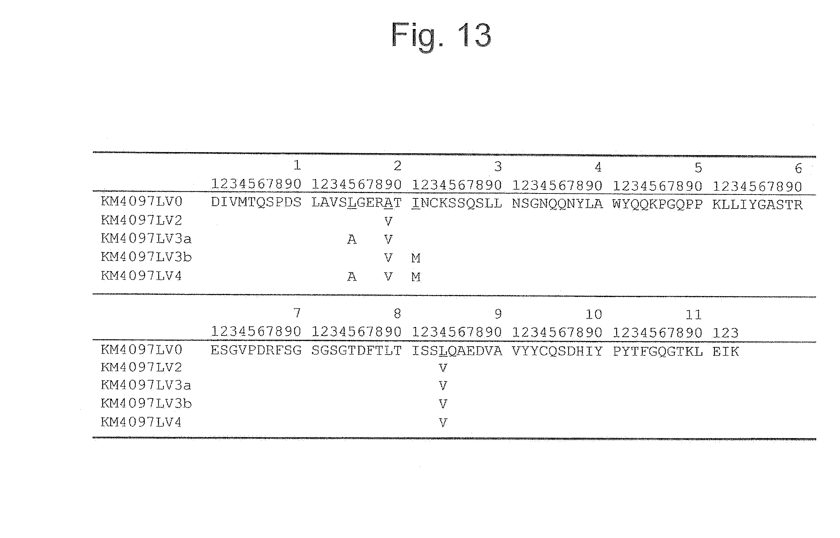

[Fig. 13] Fig. 13 shows amino acid sequences of LV0, LV2, LV3a, LV3b and LV4, which are an L chain variable regions of designed KM4097 humanized antibody.

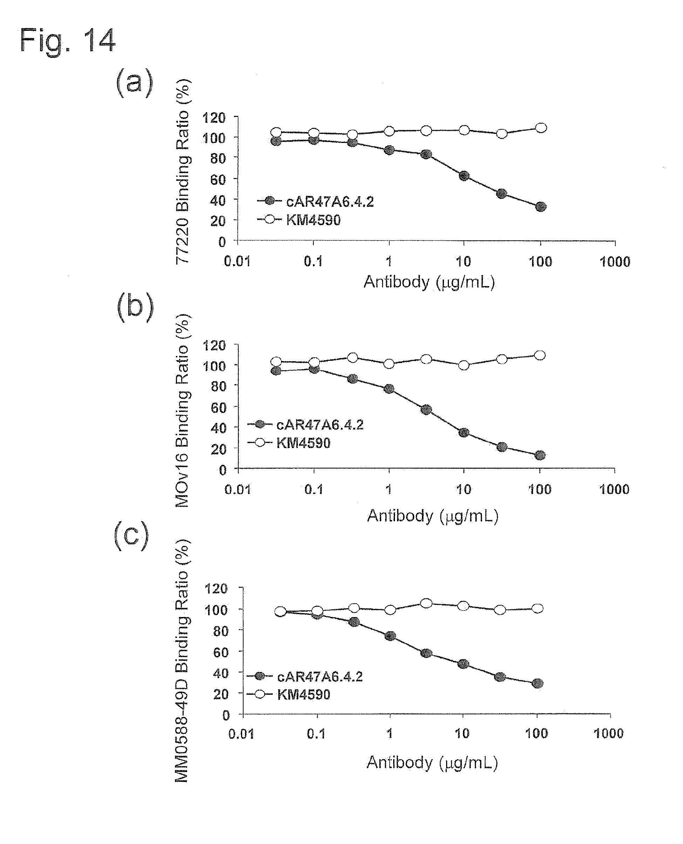

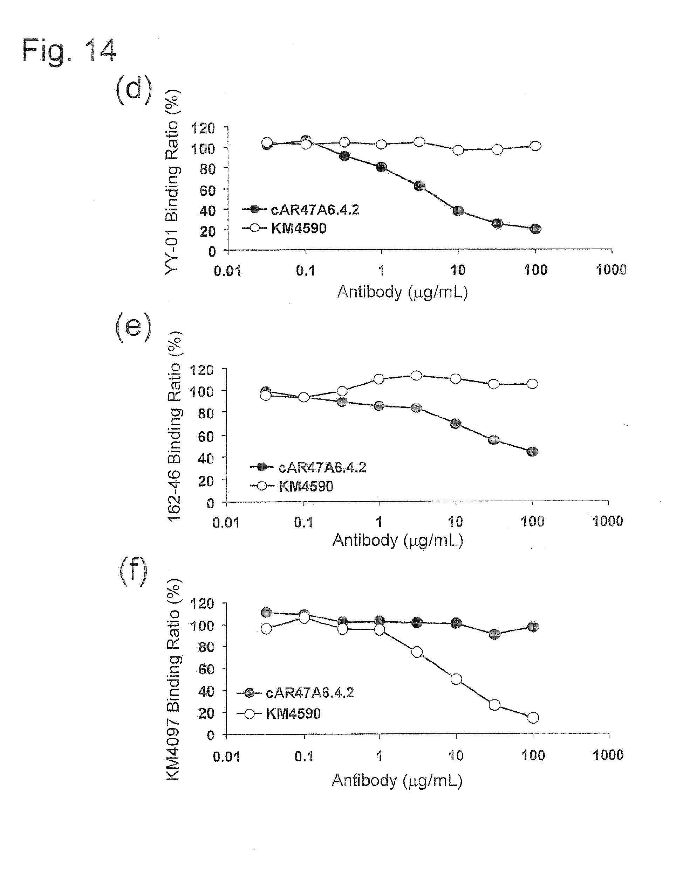

[Fig. 14(a)] to [Fig.14(f)] Fig. 14(a) to Fig. 14(f) show results obtained by analyzing the antagonism of KM4590 or cAR47A6.4.2 for the binding of each anti-Trop-2 mouse monoclonal antibody to recombinant Trop-2 using binding ELISA. The abscissa shows the concentration of each chimeric antibody and the ordinate shows binding rate (%) of each mouse monoclonal antibody. ● represents the binding rate (%) of mouse monoclonal antibody in the coexistence of cAR47A6.4.2 and ○ represents the binding rate (%) of mouse monoclonal antibody in the coexistence KM4590.

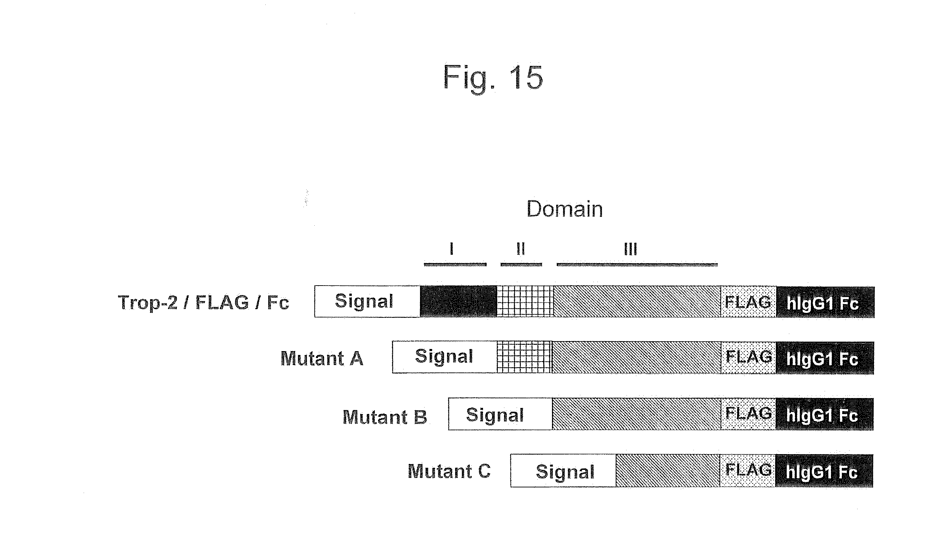

[Fig. 15] Fig. 15 shows schematic view of structures of Trop-2/FLAG/Fc, Mutant A, Mutant B and Mutant C.

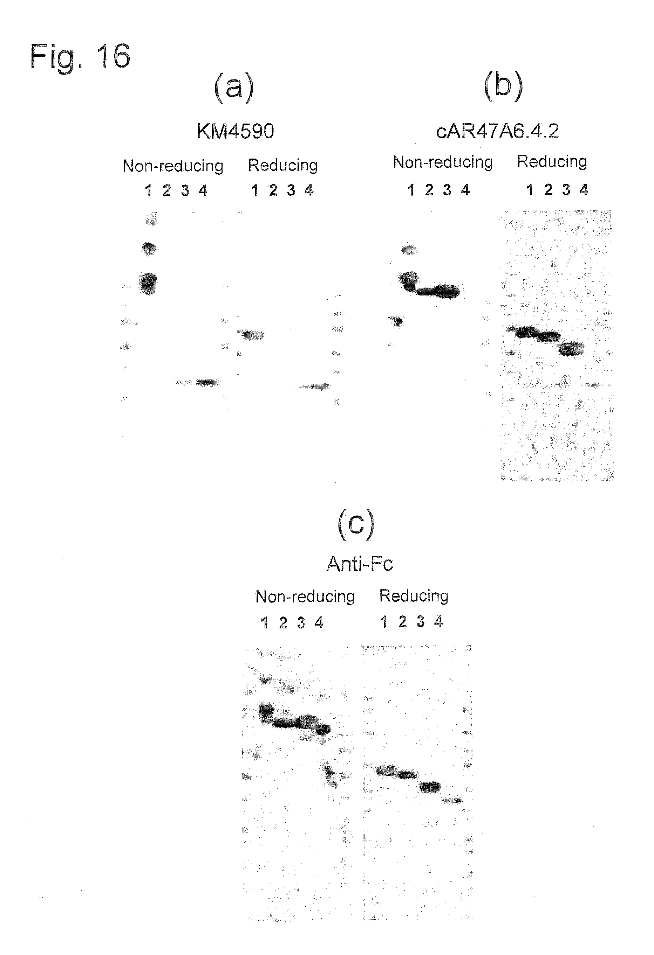

[Fig. 16(a)] to [Fig. 16(c)] Fig. 16(a) to 16(c) show results obtained by reacting KM4590 (Fig. 16(a)), cAR47A6.4.2 (Fig. 16(b)), or anti-IgG (Anti-Fc) (Fig. 16(c)) with Trop-2 extracellular region proteins or the domain deletion mutant by Western blotting. Lanes 1, 2, 3 and 4 represent Trop-2/FLAG/Fc, Mutant A, Mutant B and Mutant C, respectively.

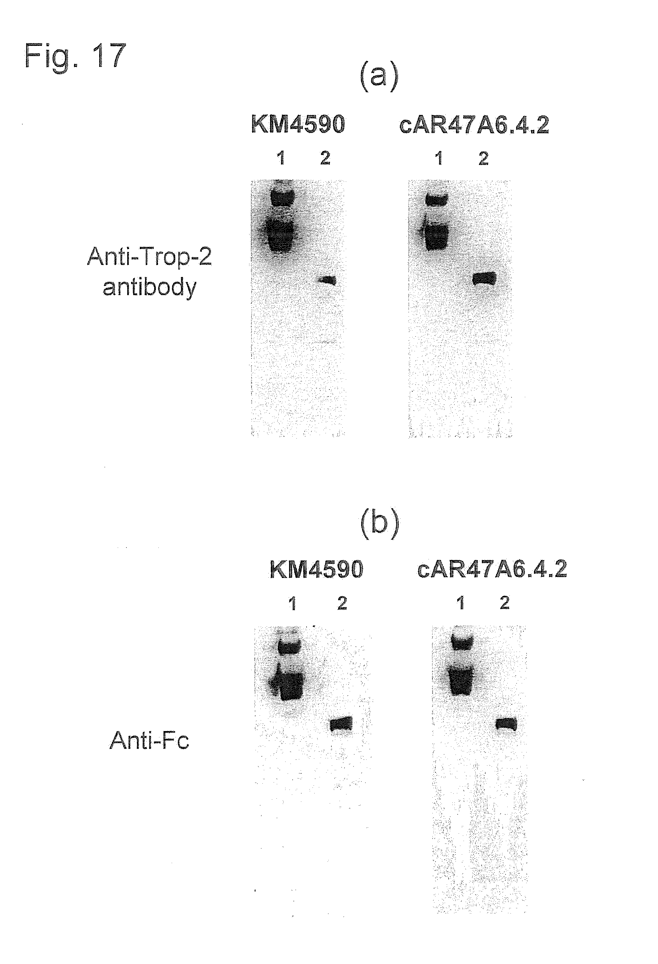

[Fig. 17(a)] and [Fig. 17(b)] Fig. 17(a) and Fig. 17(b) show results obtained by comparing the reactivity of KM4590 or cAR47A6.4.2 to Trop-2/FLAG/Fc with or without reducing treatment by Western blotting. Fig. 17(a) shows results obtained by reacting KM4590 or cAR47A6.4.2 with Trop-2/FLAG/Fc under non-reducing conditions and reducing conditions. Fig. 17(b) shows results obtained by detaching an antibody from each PDVF membrane of Fig. 17(a) and reacting the antibody with anti-human IgG (Anti-Fc). In both figures, lanes 1 and 2 represent Trop-2/FLAG/Fc under non-reducing conditions and Trop-2/FLAG/Fc under reducing conditions, respectively.

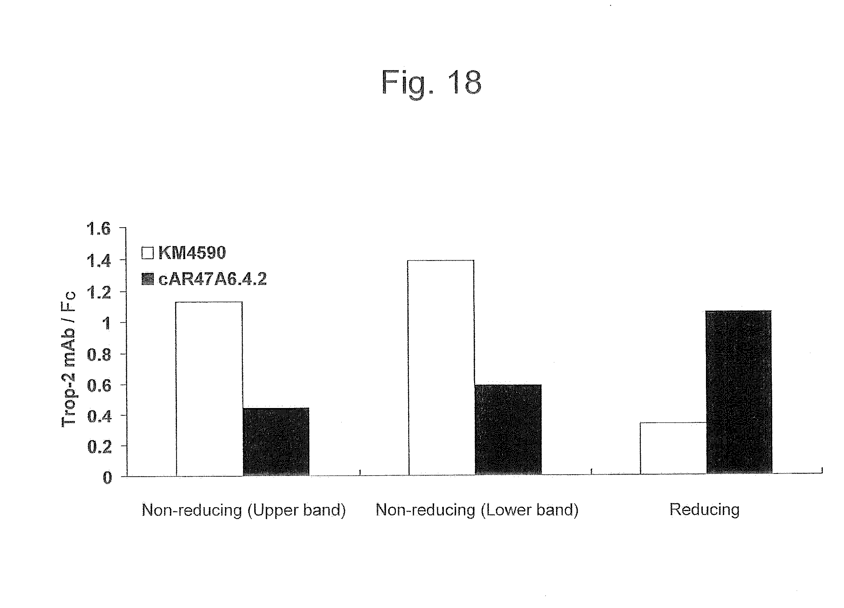

[Fig. 18] Fig. 18 shows results obtained by quantifying the signal intensity of three types of bands (the upper and lower bands in non-reducing conditions and a band in reducing conditions) on each PDVF membrane and calculating the ratio of the signal intensity quantified when KM4590 or cAR47A6.4.2 reacts to the signal intensity quantified when an anti-Fc reacts.

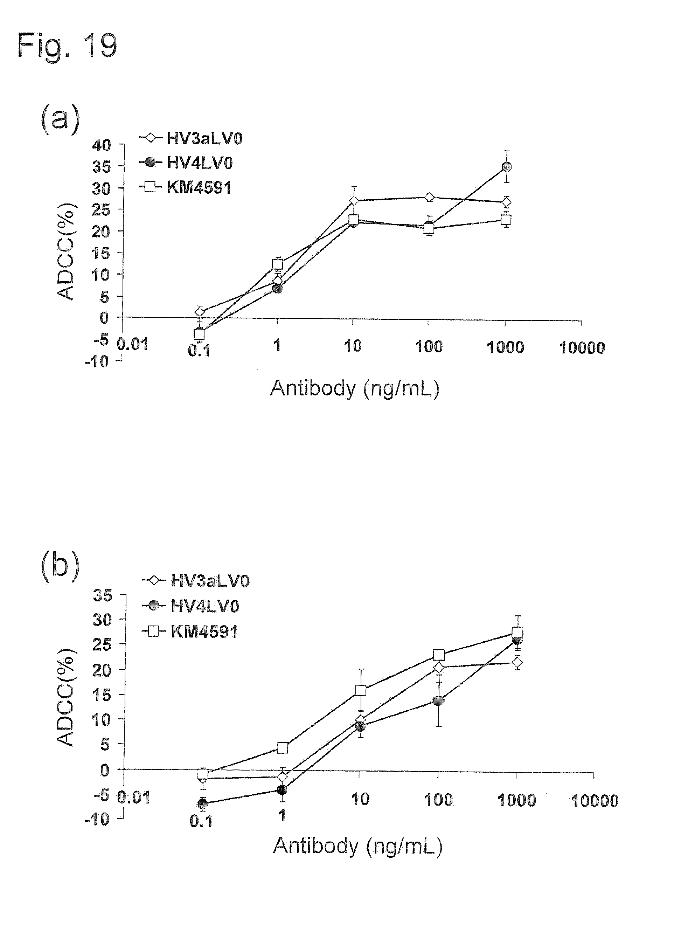

[Fig. 19(a)] and [Fig. 19(b)] Fig. 19(a) and Fig. 19(b) show the ADCC activity for human Trop-2-expressing CHO cells. The abscissa shows the concentration of each antibody and the ordinate shows the cellular cytotoxicity rate (%). Fig. 19(a) shows the result of donor 1 and Fig. 19(b) shows the result of donor 2. ◇ represents HV3aLV0, ● represents HV4LV0 and □ represents KM4591. The results are expressed as a mean value ± standard error of three wells.

Embodiments for Carrying Out the Invention

The present invention relates to a monoclonal antibody against human Trop-2, which specifically binds to the extracellular region of human Trop-2 (hereinafter referred to as "a monoclonal antibody of the present invention") or an antibody fragment thereof. Since the monoclonal antibody of the present invention binds to the extracellular region of human Trop-2 with high affinity, it has high ADCC activity and antitumor activity.

The human Trop-2 of the present invention include a polypeptide comprising the amino acid sequence represented by SEQ ID NO:1 or NCBI Accession No. P_002344; a polypeptide comprising an amino acid sequence in which at least one amino acid is deleted, substituted or added in the amino acid sequence represented by SEQ ID NO:1 or NCBI Accession No. NP_002344, and having a function of Trop-2; a polypeptide comprising an amino acid sequence having at least 60% homology, preferably at least 80% homology, more preferably at least 90% homology, and most preferably at least 95% homology, with the amino acid sequence represented by SEQ ID NO:1 or NCBI Accession No. NP_002344, and having a function of Trop-2; and the like.

The polypeptide comprising an amino acid sequence wherein one or more amino acid residue(s) is/are deleted, substituted and/or added in the amino acid sequence represented by SEQ ID NO:1 or NCBI Accession No. NP_002344 can be obtained, for example, by introducing a site-directed mutation into DNA encoding the polypeptide comprising the amino acid sequence represented by SEQ ID NO:1 by site-directed mutagenesis described in Molecular Cloning, A Laboratory Manual, Second Edition Cold Spring Harbor Laboratory Press, (1989), Current Protocols in Molecular Biology (John Wiley & Sons, 1987-1997), Nucleic Acids Research, 10, 6487 (1982), Proc. Natl. Acad. Sci. USA, 79, 6409 (1982), Gene, 34, 315 (1985), Nucleic Acids Research, 13, 4431 (1985), Proc. Natl. Acad. Sci. USA, 82, 488 (1985), or the like.

The number of amino acid residues which are deleted, substituted or added is not particularly limited, and the number is preferably, 1 to dozens, such as 1 to 20, and more preferably 1 to several, such as 1 to 5.

As a gene which encodes human Trop-2 antibody, examples include the nucleotide sequence represented by SEQ ID NO:2 or NCBI Accession No. NM_002353. Examples also include a nucleotide sequence comprising a gene in which at least one nucleotide is deleted, substituted or added in the nucleotide sequence represented by SEQ ID NO: 2 or NCBI Accession No. NM_002353 and comprising a DNA encoding a polypeptide having a function of Trop-2; a gene comprising an nucleotide sequence having at least 60% homology, preferably at least 80% homology, more preferably at least 90% homology, and most preferably at least 95% homology, with the nucleotide sequence represented by SEQ ID NO:2 or NCBI Accession No. NM_002353 and comprising a DNA encoding a polypeptide having a function of Trop-2; a gene which hybridizes with the DNA consisting of the nucleotide sequence represented by SEQ ID NO:2 or NCBI Accession No. NM_002353 under stringent conditions and encodes a polypeptide having a function of Trop-2; and the like.

The DNA which hybridizes under stringent conditions refers to a DNA which is obtained by colony hybridization, plaque hybridization, Southern hybridization or the like using a DNA consisting of the nucleotide sequence represented by SEQ ID NO:2 or NCBI Accession No. NM_002353 as a probe.

A specific example of such DNA is a DNA which can be identified by performing hybridization [Molecular Cloning, A Laboratory Manual, Second Edition, Cold Spring Harbor Lab. Press (1989), Current Protocols in Molecular Biology, John Wiley & Sons (1987-1997); DNA Cloning 1: Core Techniques, A Practical Approach, Second Edition, Oxford University (1995)] at 65°C in the presence of 0.7 to 1.0 mol/l sodium chloride using a filter with colony- or plaque-derived DNA immobilized thereon, and then washing the filter at 65°C with a 0.1 to 2-fold concentration SSC solution (1-fold concentration SSC solution: 150 mmol/l sodium chloride and 15 mmol/l sodium citrate).

Specifically, the DNA capable of hybridization under stringent conditions includes DNA having at least 60% or more homology, preferably 80% or more homology, more preferably 90% or more homology, and most preferably 95% or more homology with the nucleotide sequence represented by SEQ ID NO:2 or NCBI Accession No. NM_002353.

In the nucleotide sequence of the gene encoding a protein of a eukaryote, genetic polymorphism is often recognized. The Trop-2 gene used in the present invention also includes a gene in which small modification is generated in the nucleotide sequence by such polymorphism.

The number of the homology described in the present invention may be a number calculated by using a homology search program known by the skilled person, unless otherwise indicated. Regarding the nucleotide sequence, the number may be calculated by using a default parameter in BLAST [J. Mol. Biol., 215, 403 (1990)] or the like, and regarding the amino acid sequence, the number may be calculated by using a default parameter in BLAST2 [Nucleic Acids Res., 25, 33 89 (1997); Genome Res., 7, 649 (1997); http://www.ncbi.nlm.nih.gov/Education/BLASTinfo/information3.html] or the like.

As the default parameter, G (Cost to open gap) is 5 for the nucleotide sequence and 11 for the amino acid sequence; -E (Cost to extend gap) is 2 for the nucleotide sequence and 1 for the amino acid sequence; -q (Penalty for nucleotide mismatch) is -3; -r (reward for nucleotide match) is; -e (expect value) is 10; -W (wordsize) is 11 residues for the nucleotide sequence and 3 residues for the amino acid sequence; -y (Dropoff (X) for blast extensions in bits) is 20 for blastn and 7 for a program other than blastn; -X (X dropoff value for gapped alignment in bits) is 15; and -Z (final X dropoff value for gapped alignment in bits) is 50 for blastn and 25 for a program other than blastn (http://www.ncbi.nlm.nih.gov/blast/html/blastcgihelp.html).

The polypeptide comprising a partial sequence of the amino acid sequence represented by SEQ ID NO:1 or NCBI Accession No. NP_002344 can be prepared according to a method known by the skilled person. For example, it can be prepared by deleting a part of DNA encoding the amino acid sequence represented by SEQ ID NO:1 and culturing a transformant into which an expression vector containing the DNA is introduced.

Also, based on the thus prepared polypeptide or DNA, a polypeptide comprising an amino acid sequence in which one or more amino acid(s) is/are deleted, substituted or added in a partial sequence of the amino acid sequence represented by SEQ ID NO:1 or NCBI Accession No. NP_002344 can be prepared in the same manner as described above.

The polypeptide comprising the amino acid sequence represented by SEQ ID NO:1 or NCBI Accession No. NP_002344; or the polypeptide comprising an amino acid sequence in which at least one amino acid is deleted, substituted or added in the amino acid sequence represented by SEQ ID NO:1 or NCBI Accession No. NP_002344 can also be produced by a chemical synthesis method such as fluorenylmethoxycarbonyl (Fmoc) method or t-butyloxycarbonyl (t-Boc) method.

The extracellular region of human Trop-2 of the present invention includes, for example, regions predicted by using the amino acid sequence of the polypeptide represented by SEQ ID NO:1 with conventionally known transmembrane region prediction program SOSUI (http://bp.nuap.nagoya-u.ac.jp/SOSUI/SOSUI_submit.), prediction program TMHMM ver. 2 (http://www.cbs.dtu.dk/services/TMHMM-2.0/) or ExPASy Proteomics Server (http://Ca.expasy.org/). Examples of the extracellular region include regions corresponding to position 274 from N-terminus of the extracellular region predicted by SOSUI.

The extracellular region of human Trop-2 of the present invention is not limited, so long as it has a structure equivalent to the possible structure naturally formed by the extracellular region of Trop-2 comprising the amino acid sequence represented by SEQ ID NO:1 or NCBI Accession No. NP_002344. The possible structure naturally formed by the extracellular region of Trop-2 means a native conformation of Trop-2 expressing on a cell.

In the present invention, the extracellular region of human Trop-2 is divided into three groups and these are called as domains I, II, and III starting from the N-terminus. Namely, in the extracellular region of human Trop-2, domains which have a homology with two cysteine-rich domains in the extracellular region of EpCAM are called by domains I and II from the N-terminal and a cysteine-poor domain at the C-terminal is called as domain III. Specifically, in the amino acid sequence of human Trop-2 represented by SEQ ID NO:1, domain I corresponds to positions 34 to 72, domain II corresponds to positions 73 to 145 and domain III corresponds to position 146 to 274. The monoclonal antibody or antibody fragment thereof of the present invention binds to at least domain I in the extracellular region of human Trop-2.

Binding of the antibody or antibody fragment of the present invention to the extracellular region of human Trop-2 can be confirmed by a method in which the binding ability of a cell expressing a specified antigen and an antibody for the specific antigen is confirmed, for example, by a conventionally known immunological immunological detection method such as a radioimmuno assay using a solid phase sandwich method and the like and enzyme-linked immunosorbent assay (ELISA), preferably a fluorescent cell staining method or the like.

Specific examples include a fluorescent antibody staining method using an FMAT8100HTS System (manufactured by Applied Biosystems) [Cancer Immunol. Immunother., 36, 373 (1993)] or the like, a fluorescent cell staining method using flow cytometry, surface plasmon resonance using a Biacore System (manufactured by GE Healthcare) or the like, and the like.

Furthermore, a known immunological detection methods [Monoclonal Antibodies - Principles and practice, Third edition, Academic Press (1996), Antibodies - A Laboratory Manual, Cold Spring Harbor Laboratory (1988), Monoclonal Antibody Experimental Manual, Kodan-sha Scientific (1987)] and the like can be combined to confirm the binding.

The cell expressing human Trop-2 may be any cell, so long as it expresses the Trop-2, and examples include a cell which is naturally present in the human body, a cell line established from the cell which is naturally present in the human body, a cell obtained by gene recombination technique and the like.

The cell which is naturally present in the human body includes a cell expressing the polypeptide in the body of a cancer patient, such as a cell expressing human Trop-2 among tumor cells obtained by biopsy or the like.

Example of a cell line established from the cell which is naturally present in the human body include a cell line expressing the Trop-2, among cell lines prepared by establishing the Trop-2-expressing cells obtained from the above cancer patients, and examples include human pancreas cancer cell line BxPC-3 (ATCC Accession No: CRL-1687), human breast cancer cell line MCF-7 (ATCC Accession No: HTB-22), which are established from a human cell and the like.

Specific examples of the cell obtained by gene recombination techniques may include a human Trop-2-expressing cell obtained by introducing an expression vector comprising a human Trop-2-encoding cDNA into an insect cell, an animal cell or the like, and the like.

The monoclonal antibody of the present invention is preferably an antibody which binds to Trop-2 with high affinity. Examples of the antibody which binds to Trop-2 with high affinity include an antibody which has sufficient affinity as a therapeutic antibody and which has a dissociation constant (hereinafter referred to as $K_D$) against Trop-2 of preferably $1 \times 10^{-9}$ M or less, more preferably $7 \times 10^{-10}$ M or less, furthermore preferably $5 \times 10^{-10}$ M or less, and especially preferably $1 \times 10^{-10}$ M or less.

By setting $K_D$ at $1 \times 10^{-9}$ M or less, the antibody can bind to the extracellular region of human Trop-2 with sufficient affinity as a therapeutic antibody and have high ADCC activity and antitumor activity. The dissociation constant $K_D$ against Trop-2 can be calculated using such as Biacore T100 (manufactured by GE Healthcare) and specifically can be measured using the following method described in Examples. The monoclonal antibody of the present invention exhibits lower value than that of anti-Trop-2 antibody AR47A6.4.2 when this measuring method is applied.

The affinity is obtained through the analysis of chemical kinetics and, for example, can be calculated using such as Biacore T100 (manufactured by GE Healthcare).

In the present invention, when the dissociation is slow, the dissociation rate constant kd of an antibody calculated using Biacore T100 exhibits smaller value. The dissociation rate constant kd can be measured using Biacore T100 and then calculated by the attached software, Biacore T100 evaluation software (manufactured by GE Healthcare).

In addition, the monoclonal antibody of the present invention has preferably high ADCC activity and high antitumor activity and more preferably further has CDC activity.

In the present invention, an antibody having a high ADCC activity means an antibody which exhibits higher ADCC activity than anti-Trop-2 antibody AR47A6.4.2 when the ADCC activity of plural antibodies on a Trop-2-expressing cell are measured at the same time using a known method [Cancer Immunol. Immunother., 36, 373 (1993)].

In the present invention, an antibody having a high antitumor activity means an antibody which exhibits higher antitumor activity than anti-Trop-2 antibody AR47A6.4.2 when the antitumor activity of plural antibodies in a tumor-bearing animal model having a Trop-2-expressing cancer cell are measured at the same time. The antitumor activity can be measured using the following method described in Examples.

The monoclonal antibody of the present invention includes an antibody produced by a hybridoma and a recombinant antibody produced by a transformant transformed with an expression vector containing a gene encoding an antibody.

The monoclonal antibody is an antibody secreted by a single clone of antibody-producing cell, and recognizes only one epitope (also called antigen determinant) and has the uniformity in the amino acid sequence (primary structure).

Examples of the epitope include a single amino acid sequence, a three-dimensional structure comprising the amino acid sequence, a sugar chain-bound amino acid sequence, a three-dimensional structure comprising a sugar chain-bound amino acid sequence, and the like, recognized and bound by a monoclonal antibody.

The epitope which the monoclonal antibody of the present invention recognizes can be determined by carrying out an antibody binding assay using a mutant in which a part of domain of Trop-2 is deleted or is substituted with a domain derived from another protein.

The epitope recognized by the monoclonal antibody of the present invention has an amino acid sequence or a conformation different from that of anti-Trop-2 antibody AR47A6.4.2. It is disclosed that AR47A6.4.2 recognizes the amino acid sequence at position 179 to 187 and the amino acid sequence at position 252 to 260 as an epitope and binds to them (WO2008/144891).

Examples of the monoclonal antibody of the present invention include a monoclonal antibody which binds to a site different from epitopes to which anti-Trop-2 antibody AR47A6.4.2 and 77220 binds, and the antibody fragment thereof.

Examples of the monoclonal antibody of the present invention include a monoclonal antibody in which CDR1, CDR2 and CDR3 of a heavy chain variable region (hereinafter referred to as VH) of the antibody comprise the amino acid sequences represented by SEQ ID NOs:13, 14 and 15, respectively, and CDR1, CDR2 and CDR3 of a light chain variable region (hereinafter referred to as VL) of the antibody comprise the amino acid sequences represented by SEQ ID NOs:16, 17 and 18, respectively.

In addition, more specific examples of the monoclonal antibody of the present invention include an antibody in which VH of the antibody comprises the amino acid sequence represented by SEQ ID NO:10 and VL of the antibody comprises the amino acid sequence represented by SEQ ID No:12.

In addition, examples of the monoclonal antibody of the present invention include the above-described monoclonal antibody, a monoclonal antibody which binds to an epitope which is the same as an epitope in the extracellular region of Trop-2 to which the above-described monoclonal antibody binds, and the like. Specifically, examples include an antibody which recognizes at least domain I in the extracellular region of human Trop-2, and antibody fragment thereof.

The hybridoma can be prepared, for example, by preparing the above cell expressing Trop-2 as an antigen, inducing an antibody-producing cell having antigen specificity from an animal immunized with the antigen, and fusing the antigen-producing cell with a myeloma cell. The anti-Trop-2 antibody can be obtained by culturing the hybridoma or administering the hybridoma cell into an animal to cause ascites tumor in the animal and separating and purifying the culture or the ascites.

The animal immunized with an antigen may be any animal, so long as a hybridoma can be prepared, and mouse, rat, hamster, chicken, rabbit or the like is suitably used. Also, the antibody of the present invention includes an antibody produced by a hybridoma generated by obtaining an antibody producing cell from such an animal, in vitro immunizing by the cell and fusing the cell with a myeloma cell..

In the present invention, the recombinant antibody includes an antibody produced by gene recombination, such as a human chimeric antibody, a humanized antibody, a human antibody and an antibody fragment thereof. Among the recombinant antibodies, one having character of a common monoclonal antibody, low immunogenecity and prolonged half-life in blood is preferable as a therapeutic agent. Examples of the recombinant antibody include an antibody which is prepared by modifying the above monoclonal antibody of the present invention using a recombinant technique.

The human chimeric antibody is an antibody comprising VH and VL of an antibody of a non-human animal, and a heavy chain constant region (hereinafter referred to as CH) and a light chain constant region (hereinafter referred to as CL) of a human antibody. Specifically, the human chimeric antibody of the present invention can be produced by obtaining cDNAs encoding VH and VL from a hybridoma which produces a monoclonal antibody which specifically recognizes Trop-2 and binds to the extracellular region, inserting each of them into an expression vector for animal cell comprising DNAs encoding CH and CL of human antibody to thereby construct a expression vector of human chimeric antibody, and then introducing the vector into an animal cell to express the antibody.

As the CH of the human chimeric antibody, any CH can be used, so long as it belongs to human immunoglobulin (hereinafter referred to as "hIg"), and those belonging to the hIgG class are preferred, and any one of the subclasses belonging to the hIgG class, such as hIgG1, hIgG2, hIgG3 and hIgG4, can be used. As the CL of the human chimeric antibody, any CL can be used, so long as it belongs to the hIg class, and those belonging to κ class or λ class can be used.

Examples of the chimeric antibody of the present invention include a chimeric antibody in which VH of the antibody comprises the amino acid sequences represented by SEQ ID NO:10, and VL of the antibody comprises the amino acid sequences represented by SEQ ID NO:12.

In addition, examples of the chimeric antibody of the present invention include a chimeric antibody which binds to an extracellular region of Trop-2 while competing with the above chimeric antibody and a chimeric antibody which binds an epitope in an extracellular region of Trop-2 to which the above chimeric antibody binds.

A humanized antibody includes a humanized Complementarity Determining Region (hereinafter referred to as CDR) grafted antibody.

The humanized antibody of the present invention can be produced by constructing cDNAs encoding an antibody V region in which the amino acid sequences of CDRs of VH and VL of an antibody derived from a non-human animal produced by a hybridoma which produces a monoclonal antibody which specifically recognizes three-dimensional structure of Trop-2 and binds to the extracellular region are grafted into frameworks (hereinafter referred to as "FR") of VH and VL of a suitable human antibody, inserting each of them into an expression vector for animal cell comprising genes encoding CH and CL of a human antibody to thereby construct an expression vector of a humanized antibody, and introducing it into an animal cell to thereby express and produce the humanized antibody.

As the amino acid sequences of FRs of VH and VL of a humanized antibody, any amino acid sequences can be used, so long as they are amino acid sequences of FRs of VH and VL, respectively, derived from a human antibody. Examples include amino acid sequences of FRs of VH and VL of human antibodies registered in database such as Protein Data Bank, common amino acid sequences of each sub group of FRs of VH and VL of human antibodies described in, for example, Sequences of Proteins of Immunological Interest, US Dept. Health and Human Services (1991), and the like.

Example of a humanized antibody of the present invention includes a humanized antibody which comprises VH of the antibody wherein CDR1, CDR2, and CDR3 comprise the amino acid sequences represented by SEQ ID NO:13, 14, and 15, respectively; and comprises VL of the antibody wherein CDR1, CDR2, and CDR3 comprise the amino acid sequences represented by SEQ ID NO:16, 17, and 18, respectively.

Specific examples of a humanized antibody of the present invention include a humanized antibody which comprises at least one of (a) VH and (b) VL, and the like, and there is no limit on the number of modifications to be introduced:

(a) VH of the antibody which comprises the amino acid sequence represented by SEQ ID NO:24 or an amino acid sequence in which at least one amino acid residue selected from Ala at position 9, Lys at position 12, Val at position 20, Arg at position 38, Met at position 48, Arg at position 67, Val at position 68, Ile at position 70, Tyr at position 95, and Val at position 112 in the amino acid sequence represented by SEQ ID NO:24 is substituted with other amino acid residue,

(b) VL of the antibody which comprises the amino acid sequence represented by SEQ ID NO:26 or an amino acid sequence in which at least one amino acid residue selected from Leu at position 15, Ala at position 19, Ile at position 21, and Leu at position 84 in the amino acid sequence represented by SEQ ID NO:26 is substituted with other amino acid residue.

Regarding VH which is included in a humanized antibody of the present invention, the following (1) to (11) are preferable:

(1) VH which comprises an amino acid sequence in which Ala at position 9, Lys at position 12, Val at position 20, Arg at position 38, Met at position 48, Arg at position 67, Val at position 68, Ile at position 70, Tyr at position 95, and Val at position 112 in the amino acid sequence represented by SEQ ID NO:24 are substituted with other amino acid residues,

(2) VH which comprises an amino acid sequence in which Ala at position 9, Lys at position 12, Val at position 20, Arg at position 38, Met at position 48, Arg at position 67, Val at position 68, Ile at position 70, and Tyr at position 95 in the amino acid sequence represented by SEQ ID NO:24 are substituted with other amino acid residues,

(3) VH which comprises an amino acid sequence in which Ala at position 9, Val at position 20, Arg at position 38, Met at position 48, Arg at position 67, Val at position 68, Ile at position 70, and Tyr at position 95 in the amino acid sequence represented by SEQ ID NO:24 are substituted with other amino acid residues,

(4) VH which comprises an amino acid sequence in which Ala at position 9, Arg at position 38, Met at position 48, Arg at position 67, Val at position 68, and Tyr at position 95 in the amino acid sequence represented by SEQ ID NO:24 are substituted with other amino acid residues,

(5) VH which comprises an amino acid sequence in which Ala at position 9, Arg at position 67, Val at position 68, Ile at position 70, and Tyr at position 95 in the amino acid sequence represented by SEQ ID NO:24 are substituted with other amino acid residues,

(6) VH which comprises an amino acid sequence in which Ala at position 9, Arg at position 38, Met at position 48, Arg at position 67, and Tyr at position 95 in the amino acid sequence represented by SEQ ID NO:24 are substituted with other amino acid residues,

(7) VH which comprises an amino acid sequence in which Arg at position 38, Met at position 48, Arg at position 67, Val at position 68, and Tyr at position 95 in the amino acid sequence represented by SEQ ID NO:24 are substituted with other amino acid residues,

(8) VH which comprises an amino acid sequence in which Ala at position 9, Arg at position 38, Met at position 48, and Tyr at position 95 in the amino acid sequence represented by SEQ ID NO:24 are substituted with other amino acid residues,

(9) VH which comprises an amino acid sequence in which Ala at position 9, Arg at position 67, and Tyr at position 95 in the amino acid sequence represented by SEQ ID NO:24 are substituted with other amino acid residues,

(10) VH which comprises an amino acid sequence in which Arg at position 38, Met at position 48, and Tyr at position 95 in the amino acid sequence represented by SEQ ID NO:24 are substituted with other amino acid residues,

(11) VH which comprises an amino acid sequence in which Arg at position 38 and Met at position 48 in the amino acid sequence represented by SEQ ID NO:24 are substituted with other amino acid residues.

Examples of the amino acid sequences of the VH includes an amino acid sequence in which at least one modification among amino acid modifications for substituting Ala at position 9 with Pro, Lys at position 12 with Val, Val at position 20 with Ile, Arg at position 38 with Lys, Met at position 48 with Ile, Arg at position 67 with Lys, Val at position 68 with Ala, Ile at position 70 with Leu, Tyr at position 95 with Phe, and Val at position 112 with Leu is introduced in the amino acid sequence represented by SEQ ID NO:24.

Specific examples of the amino acid sequence of VH in which ten modifications are introduced include an amino acid sequence in which substitutions of Ala at position 9 with Pro, Lys at position 12 with Val, Val at position 20 with Ile, Arg at position 38 with Lys, Met at position 48 with Ile, Arg at position 67 with Lys, Val at position 68 with Ala, Ile at position 70 with Leu, Tyr at position 95 with Phe, and Val at position 112 with Leu are introduced in the amino acid sequence represented by SEQ ID NO:24.

Specific examples of the amino acid sequence of VH in which nine modifications are introduced include the following amino acid sequences (1) to (10):

(1) an amino acid sequence in which substitutions of Lys at position 12 with Val, Val at position 20 with Ile, Arg at position 38 with Lys, Met at position 48 with Ile, Arg at position 67 with Lys, Val at position 68 with Ala, Ile at position 70 with Leu, Tyr at position 95 with Phe, and Val at position 112 with Leu are introduced in the amino acid sequence represented by SEQ ID NO:24,

(2) an amino acid sequence in which substitutions of Ala at position 9 with Pro, Val at position 20 with Ile, Arg at position 38 with Lys, Met at position 48 with Ile, Arg at position 67 with Lys, Val at position 68 with Ala, Ile at position 70 with Leu, Tyr at position 95 with Phe, and Val at position 112 with Leu are introduced in the amino acid sequence represented by SEQ ID NO:24,

(3) an amino acid sequence in which substitutions of Ala at position 9 with Pro, Lys at position 12 with Val, Arg at position 38 with Lys, Met at position 48 with Ile, Arg at position 67 with Lys, Val at position 68 with Ala, Ile at position 70 with Leu, Tyr at position 95 with Phe, and Val at position 112 with Leu are introduced in the amino acid sequence represented by SEQ ID NO:24,

(4) an amino acid sequence in which substitutions of Ala at position 9 with Pro, Lys at position 12 with Val, Val at position 20 with Ile, Met at position 48 with Ile, Arg at position 67 with Lys, Val at position 68 with Ala, Ile at position 70 with Leu, Tyr at position 95 with Phe, and Val at position 112 with Leu are introduced in the amino acid sequence represented by SEQ ID NO:24,

(5) an amino acid sequence in which substitutions of Ala at position 9 with Pro, Lys at position 12 with Val, Val at position 20 with Ile, Arg at position 38 with Lys, Arg at position 67 with Lys, Val at position 68 with Ala, Ile at position 70 with Leu, Tyr at position 95 with Phe, and Val at position 112 with Leu are introduced in the amino acid sequence represented by SEQ ID NO:24,

(6) an amino acid sequence in which substitutions of Ala at position 9 with Pro, Lys at position 12 with Val, Val at position 20 with Ile, Arg at position 38 with Lys, Met at position 48 with Ile, Val at position 68 with Ala, Ile at position 70 with Leu, Tyr at position 95 with Phe, and Val at position 112 with Leu are introduced in the amino acid sequence represented by SEQ ID NO:24,

(7) an amino acid sequence in which substitutions of Ala at position 9 with Pro, Lys at position 12 with Val, Val at position 20 with Ile, Arg at position 38 with Lys, Met at position 48 with Ile, Arg at position 67 with Lys, Ile at position 70 with Leu, Tyr at position 95 with Phe, and Val at position 112 with Leu are introduced in the amino acid sequence represented by SEQ ID NO:24,

(8) an amino acid sequence in which substitutions of Ala at position 9 with Pro, Lys at position 12 with Val, Val at position 20 with Ile, Arg at position 38 with Lys, Met at position 48 with Ile, Arg at position 67 with Lys, Val at position 68 with Ala, Tyr at position 95 with Phe, and Val at position 112 with Leu are introduced in the amino acid sequence represented by SEQ ID NO:24,

(9) an amino acid sequence in which substitutions of Ala at position 9 with Pro, Lys at position 12 with Val, Val at position 20 with Ile, Arg at position 38 with Lys, Met at position 48 with Ile, Arg at position 67 with Lys, Val at position 68 with Ala, Ile at position 70 with Leu, and Val at position 112 with Leu are introduced in the amino acid sequence represented by SEQ ID NO:24,

(10) an amino acid sequence in which substitutions of Ala at position 9 with Pro, Lys at position 12 with Val, Val at position 20 with Ile, Arg at position 38 with Lys, Met at position 48 with Ile, Arg at position 67 with Lys, Val at position 68 with Ala, Ile at position 70 with Leu, and Tyr at position 95 with Phe are introduced in the amino acid sequence represented by SEQ ID NO:24.

Specific examples of the amino acid sequence of VH in which eight modifications are introduced include the following amino acid sequences (1) to (17):

(1) an amino acid sequence in which substitutions of Ala at position 9 with Pro, Lys at position 12 with Val, Arg at position 38 with Lys, Met at position 48 with Ile, Arg at position 67 with Lys, Val at position 68 with Ala, Ile at position 70 with Leu, and Tyr at position 95 with Phe are introduced in the amino acid sequence represented by SEQ ID NO:24,

(2) an amino acid sequence in which substitutions of Lys at position 12 with Val, Val at position 20 with Ile, Arg at position 38 with Lys, Met at position 48 with Ile, Arg at position 67 with Lys, Val at position 68 with Ala, Ile at position 70 with Leu, and Tyr at position 95 with Phe are introduced in the amino acid sequence represented by SEQ ID NO:24,

(3) an amino acid sequence in which substitutions of Ala at position 9 with Pro, Val at position 20 with Ile, Arg at position 38 with Lys, Met at position 48 with Ile, Arg at position 67 with Lys, Val at position 68 with Ala, Ile at position 70 with Leu, and Tyr at position 95 with Phe are introduced in the amino acid sequence represented by SEQ ID NO:24,

(4) an amino acid sequence in which substitutions of Ala at position 9 with Pro, Lys at position 12 with Val, Val at position 20 with Ile, Met at position 48 with Ile, Arg at position 67 with Lys, Val at position 68 with Ala, Ile at position 70 with Leu, and Tyr at position 95 with Phe are introduced in the amino acid sequence represented by SEQ ID NO:24,

(5) an amino acid sequence in which substitutions of Ala at position 9 with Pro, Lys at position 12 with Val, Val at position 20 with Ile, Arg at position 38 with Lys, Arg at position 67 with Lys, Val at position 68 with Ala, Ile at position 70 with Leu, and Tyr at position 95 with Phe are introduced in the amino acid sequence represented by SEQ ID NO:24,

(6) an amino acid sequence in which substitutions of Ala at position 9 with Pro, Lys at position 12 with Val, Val at position 20 with Ile, Arg at position 38 with Lys, Met at position 48 with Ile, Val at position 68 with Ala, Ile at position 70 with Leu, and Tyr at position 95 with Phe are introduced in the amino acid sequence represented by SEQ ID NO:24,

(7) an amino acid sequence in which substitutions of Ala at position 9 with Pro, Lys at position 12 with Val, Val at position 20 with Ile, Arg at position 38 with Lys, Met at position 48 with Ile, Arg at position 67 with Lys, Ile at position 70 with Leu, and Tyr at position 95 with Phe are introduced in the amino acid sequence represented by SEQ ID NO:24,

(8) an amino acid sequence in which substitutions of Ala at position 9 with Pro, Lys at position 12 with Val, Val at position 20 with Ile, Arg at position 38 with Lys, Met at position 48 with Ile, Arg at position 67 with Lys, Val at position 68 with Ala, and Tyr at position 95 with Phe are introduced in the amino acid sequence represented by SEQ ID NO:24,

(9) an amino acid sequence in which substitutions of Ala at position 9 with Pro, Lys at position 12 with Val, Val at position 20 with Ile, Arg at position 38 with Lys, Met at position 48 with Ile, Arg at position 67 with Lys, Val at position 68 with Ala, and Ile at position 70 with Leu are introduced in the amino acid sequence represented by SEQ ID NO:24,

(10) an amino acid sequence in which substitutions of Lys at position 12 with Val, Arg at position 38 with Lys, Met at position 48 with Ile, Arg at position 67 with Lys, Val at position 68 with Ala, Ile at position 70 with Leu, Tyr at position 95 with Phe, and Val at position 112 with Leu are introduced in the amino acid sequence represented by SEQ ID NO:24,

(11) an amino acid sequence in which substitutions of Ala at position 9 with Pro, Arg at position 38 with Lys, Met at position 48 with Ile, Arg at position 67 with Lys, Val at position 68 with Ala, Ile at position 70 with Leu, Tyr at position 95 with Phe, and Val at position 112 with Leu are introduced in the amino acid sequence represented by SEQ ID NO:24,

(12) an amino acid sequence in which substitutions of Ala at position 9 with Pro, Lys at position 12 with Val, Met at position 48 with Ile, Arg at position 67 with Lys, Val at position 68 with Ala, Ile at position 70 with Leu, Tyr at position 95 with Phe, and Val at position 112 with Leu are introduced in the amino acid sequence represented by SEQ ID NO:24,

(13) an amino acid sequence in which substitutions of Ala at position 9 with Pro, Lys at position 12 with Val, Arg at position 38 with Lys, Arg at position 67 with Lys, Val at position 68 with Ala, Ile at position 70 with Leu, Tyr at position 95 with Phe, and Val at position 112 with Leu are introduced in the amino acid sequence represented by SEQ ID NO:24,

(14) an amino acid sequence in which substitutions of Ala at position 9 with Pro, Lys at position 12 with Val, Arg at position 38 with Lys, Met at position 48 with Ile, Val at position 68 with Ala, Ile at position 70 with Leu, Tyr at position 95 with Phe, and Val at position 112 with Leu are introduced in the amino acid sequence represented by SEQ ID NO:24,

(15) an amino acid sequence in which substitutions of Ala at position 9 with Pro, Lys at position 12 with Val, Arg at position 38 with Lys, Met at position 48 with Ile, Arg at position 67 with Lys, Ile at position 70 with Leu, Tyr at position 95 with Phe, and Val at position 112 with Leu are introduced in the amino acid sequence represented by SEQ ID NO:24,

(16) an amino acid sequence in which substitutions of Ala at position 9 with Pro, Lys at position 12 with Val, Arg at position 38 with Lys, Met at position 48 with Ile, Arg at position 67 with Lys, Val at position 68 with Ala, Tyr at position 95 with Phe, and Val at position 112 with Leu are introduced in the amino acid sequence represented by SEQ ID NO:24,

(17) an amino acid sequence in which substitutions of Ala at position 9 with Pro, Lys at position 12 with Val, Arg at position 38 with Lys, Met at position 48 with Ile, Arg at position 67 with Lys, Val at position 68 with Ala, Ile at position 70 with Leu, and Val at position 112 with Leu are introduced in the amino acid sequence represented by SEQ ID NO:24.

Specific examples of the amino acid sequence of VH in which seven modifications are introduced include the following amino acid sequences (1) to (8):

(1) an amino acid sequence in which substitutions of Lys at position 12 with Val, Arg at position 38 with Lys, Met at position 48 with Ile, Arg at position 67 with Lys, Val at position 68 with Ala, Ile at position 70 with Leu, and Tyr at position 95 with Phe are introduced in the amino acid sequence represented by SEQ ID NO:24,

(2) an amino acid sequence in which substitutions of Ala at position 9 with Pro, Arg at position 38 with Lys, Met at position 48 with Ile, Arg at position 67 with Lys, Val at position 68 with Ala, Ile at position 70 with Leu, and Tyr at position 95 with Phe are introduced in the amino acid sequence represented by SEQ ID NO:24,

(3) an amino acid sequence in which substitutions of Ala at position 9 with Pro, Lys at position 12 with Val, Met at position 48 with Ile, Arg at position 67 with Lys, Val at position 68 with Ala, Ile at position 70 with Leu, and Tyr at position 95 with Phe are introduced in the amino acid sequence represented by SEQ ID NO:24,

(4) an amino acid sequence in which substitutions of Ala at position 9 with Pro, Lys at position 12 with Val, Arg at position 38 with Lys, Arg at position 67 with Lys, Val at position 68 with Ala, Ile at position 70 with Leu, and Tyr at position 95 with Phe are introduced in the amino acid sequence represented by SEQ ID NO:24,

(5) an amino acid sequence in which substitutions of Ala at position 9 with Pro, Lys at position 12 with Val, Arg at position 38 with Lys, Met at position 48 with Ile, Val at position 68 with Ala, Ile at position 70 with Leu, and Tyr at position 95 with Phe are introduced in the amino acid sequence represented by SEQ ID NO:24,

(6) an amino acid sequence in which substitutions of Ala at position 9 with Pro, Lys at position 12 with Val, Arg at position 38 with Lys, Met at position 48 with Ile, Arg at position 67 with Lys, Ile at position 70 with Leu, and Tyr at position 95 with Phe are introduced in the amino acid sequence represented by SEQ ID NO:24,

(7) an amino acid sequence in which substitutions of Ala at position 9 with Pro, Lys at position 12 with Val, Arg at position 38 with Lys, Met at position 48 with Ile, Arg at position 67 with Lys, Val at position 68 with Ala, and Tyr at position 95 with Phe are introduced in the amino acid sequence represented by SEQ ID NO:24,

(8) an amino acid sequence in which substitutions of Ala at position 9 with Pro, Lys at position 12 with Val, Arg at position 38 with Lys, Met at position 48 with Ile, Arg at position 67 with Lys, Val at position 68 with Ala, and Ile at position 70 with Leu are introduced in the amino acid sequence represented by SEQ ID NO:24.

Specific examples of the amino acid sequence of VH in which six modifications are introduced include the following amino acid sequences (1) to (13):

(1) an amino acid sequence in which substitutions of Ala at position 9 with Pro, Arg at position 38 with Lys, Met at position 48 with Ile, Arg at position 67 with Lys, Val at position 68 with Ala, and Tyr at position 95 with Phe are introduced in the amino acid sequence represented by SEQ ID NO:24,

(2) an amino acid sequence in which substitutions of Arg at position 38 with Lys, Met at position 48 with Ile, Arg at position 67 with Lys, Val at position 68 with Ala, Ile at position 70 with Leu, and Tyr at position 95 with Phe are introduced in the amino acid sequence represented by SEQ ID NO:24,

(3) an amino acid sequence in which substitutions of Ala at position 9 with Pro, Met at position 48 with Ile, Arg at position 67 with Lys, Val at position 68 with Ala, Ile at position 70 with Leu, and Tyr at position 95 with Phe are introduced in the amino acid sequence represented by SEQ ID NO:24,

(4) an amino acid sequence in which substitutions of Ala at position 9 with Pro, Arg at position 38 with Lys, Arg at position 67 with Lys, Val at position 68 with Ala, Ile at position 70 with Leu, and Tyr at position 95 with Phe are introduced in the amino acid sequence represented by SEQ ID NO:24,

(5) an amino acid sequence in which substitutions of Ala at position 9 with Pro, Arg at position 38 with Lys, Met at position 48 with Ile, Val at position 68 with Ala, Ile at position 70 with Leu, and Tyr at position 95 with Phe are introduced in the amino acid sequence represented by SEQ ID NO:24,

(6) an amino acid sequence in which substitutions of Ala at position 9 with Pro, Arg at position 38 with Lys, Met at position 48 with Ile, Arg at position 67 with Lys, Ile at position 70 with Leu, and Tyr at position 95 with Phe are introduced in the amino acid sequence represented by SEQ ID NO:24,

(7) an amino acid sequence in which substitutions of Ala at position 9 with Pro, Arg at position 38 with Lys, Met at position 48 with Ile, Arg at position 67 with Lys, Val at position 68 with Ala, and Ile at position 70 with Leu are introduced in the amino acid sequence represented by SEQ ID NO:24,

(8) an amino acid sequence in which substitutions of Val at position 20 with Ile, Arg at position 38 with Lys, Met at position 48 with Ile, Arg at position 67 with Lys, Val at position 68 with Ala, and Tyr at position 95 with Phe are introduced in the amino acid sequence represented by SEQ ID NO:24,

(9) an amino acid sequence in which substitutions of Ala at position 9 with Pro, Val at position 20 with Ile, Met at position 48 with Ile, Arg at position 67 with Lys, Val at position 68 with Ala, and Tyr at position 95 with Phe are introduced in the amino acid sequence represented by SEQ ID NO:24,

(10) an amino acid sequence in which substitutions of Ala at position 9 with Pro, Val at position 20 with Ile, Arg at position 38 with Lys, Arg at position 67 with Lys, Val at position 68 with Ala, and Tyr at position 95 with Phe are introduced in the amino acid sequence represented by SEQ ID NO:24,

(11) an amino acid sequence in which substitutions of Ala at position 9 with Pro, Val at position 20 with Ile, Arg at position 38 with Lys, Met at position 48 with Ile, Val at position 68 with Ala, and Tyr at position 95 with Phe are introduced in the amino acid sequence represented by SEQ ID NO:24,

(12) an amino acid sequence in which substitutions of Ala at position 9 with Pro, Val at position 20 with Ile, Arg at position 38 with Lys, Met at position 48 with Ile, Arg at position 67 with Lys, and Tyr at position 95 with Phe are introduced in the amino acid sequence represented by SEQ ID NO:24,

(13) an amino acid sequence in which substitutions of Ala at position 9 with Pro, Val at position 20 with Ile, Arg at position 38 with Lys, Met at position 48 with Ile, Arg at position 67 with Lys, and Val at position 68 with Ala are introduced in the amino acid sequence represented by SEQ ID NO:24.

Specific examples of the amino acid sequence of VH in which five modifications are introduced include the following amino acid sequences (1) to (5):

(1) an amino acid sequence in which substitutions of Ala at position 9 with Pro, Arg at position 67 with Lys, Val at position 68 with Ala, Ile at position 70 with Leu, and Tyr at position 95 with Phe are introduced in the amino acid sequence represented by SEQ ID NO:24,

(2) an amino acid sequence in which substitutions of Ala at position 9 with Pro, Arg at position 38 with Lys, Met at position 48 with Ile, Arg at position 67 with Lys, and Tyr at position 95 with Phe are introduced in the amino acid sequence represented by SEQ ID NO:24,

(3) an amino acid sequence in which substitutions of Arg at position 38 with Lys, Met at position 48 with Ile, Arg at position 67 with Lys, Val at position 68 with Ala, and Tyr at position 95 with Phe are introduced in the amino acid sequence represented by SEQ ID NO:24,

(4) an amino acid sequence in which substitutions of Lys at position 12 with Val, Arg at position 38 with Lys, Met at position 48 with Ile, Arg at position 67 with Lys, and Tyr at position 95 with Phe are introduced in the amino acid sequence represented by SEQ ID NO:24,

(5) an amino acid sequence in which substitutions of Arg at position 38 with Lys, Met at position 48 with Ile, Arg at position 67 with Lys, Ile at position 70 with Leu, and Tyr at position 95 with Phe are introduced in the amino acid sequence represented by SEQ ID NO:24.

Specific examples of the amino acid sequence of VH in which four modifications are introduced include the following amino acid sequences (1) to (5):

(1) an amino acid sequence in which substitutions of Ala at position 9 with Pro, Arg at position 38 with Lys, Met at position 48 with Ile, and Tyr at position 95 with Phe are introduced in the amino acid sequence represented by SEQ ID NO:24,

(2) an amino acid sequence in which substitutions of Lys at position 12 with Val, Arg at position 38 with Lys, Met at position 48 with Ile, and Tyr at position 95 with Phe are introduced in the amino acid sequence represented by SEQ ID NO:24,

(3) an amino acid sequence in which substitutions of Arg at position 38 with Lys, Met at position 48 with Ile, Arg at position 67 with Lys, and Tyr at position 95 with Phe are introduced in the amino acid sequence represented by SEQ ID NO:24,

(4) an amino acid sequence in which substitutions of Arg at position 38 with Lys, Met at position 48 with Ile, Val at position 68 with Ala, and Tyr at position 95 with Phe are introduced in the amino acid sequence represented by SEQ ID NO:24,

(5) an amino acid sequence in which substitutions of Arg at position 38 with Lys, Met at position 48 with Ile, Ile at position 70 with Leu, and Tyr at position 95 with Phe are introduced in the amino acid sequence represented by SEQ ID NO:24.

Specific examples of the amino acid sequence of VH in which three modifications are introduced include the following amino acid sequences (1) to (12):

(1) an amino acid sequence in which substitutions of Ala at position 9 with Pro, Arg at position 67 with Lys, and Tyr at position 95 with Phe are introduced in the amino acid sequence represented by SEQ ID NO:24,

(2) an amino acid sequence in which substitutions of Arg at position 38 with Lys, Met at position 48 with Ile, and Tyr at position 95 with Phe are introduced in the amino acid sequence represented by SEQ ID NO:24,

(3) an amino acid sequence in which substitutions of Ala at position 9 with Pro, Lys at position 12 with Val, and Tyr at position 95 with Phe are introduced in the amino acid sequence represented by SEQ ID NO:24,

(4) an amino acid sequence in which substitutions of Ala at position 9 with Pro, Arg at position 38 with Lys, and Tyr at position 95 with Phe are introduced in the amino acid sequence represented by SEQ ID NO:24,

(5) an amino acid sequence in which substitutions of Ala at position 9 with Pro, Met at position 48 with Ile, and Tyr at position 95 with Phe are introduced in the amino acid sequence represented by SEQ ID NO:24,

(6) an amino acid sequence in which substitutions of Ala at position 9 with Pro, Val at position 68 with Ala, and Tyr at position 95 with Phe are introduced in the amino acid sequence represented by SEQ ID NO:24,

(7) an amino acid sequence in which substitutions of Ala at position 9 with Pro, Ile at position 70 with Leu, and Tyr at position 95 with Phe are introduced in the amino acid sequence represented by SEQ ID NO:24,

(8) an amino acid sequence in which substitutions of Ala at position 9 with Pro, Arg at position 38 with Lys, and Met at position 48 with Ile are introduced in the amino acid sequence represented by SEQ ID NO:24,

(9) an amino acid sequence in which substitutions of Lys at position 12 with Val, Arg at position 38 with Lys, and Met at position 48 with Ile are introduced in the amino acid sequence represented by SEQ ID NO:24,

(10) an amino acid sequence in which substitutions of Arg at position 38 with Lys, Met at position 48 with Ile, and Arg at position 67 with Lys are introduced in the amino acid sequence represented by SEQ ID NO:24,

(11) an amino acid sequence in which substitutions of Arg at position 38 with Lys, Met at position 48 with Ile, and Val at position 68 with Ala are introduced in the amino acid sequence represented by SEQ ID NO:24,

(12) an amino acid sequence in which substitutions of Arg at position 38 with Lys, Met at position 48 with Ile, and Ile at position 70 with Leu are introduced in the amino acid sequence represented by SEQ ID NO:24.

Specific examples of the amino acid sequence of VH in which two modifications are introduced include the following amino acid sequences (1) to (18):

(1) an amino acid sequence in which substitutions of Arg at position 38 with Lys and Met at position 48 with Ile are introduced in the amino acid sequence represented by SEQ ID NO:24,

(2) an amino acid sequence in which substitutions of Arg at position 38 with Lys and Tyr at position 95 with Phe are introduced in the amino acid sequence represented by SEQ ID NO:24,

(3) an amino acid sequence in which substitutions of Met at position 48 with Ile and Tyr at position 95 with Phe are introduced in the amino acid sequence represented by SEQ ID NO:24,

(4) an amino acid sequence in which substitutions of Ala at position 9 with Pro and Arg at position 38 with Lys are introduced in the amino acid sequence represented by SEQ ID NO:24,

(5) an amino acid sequence in which substitutions of Lys at position 12 with Val and Arg at position 38 with Lys are introduced in the amino acid sequence represented by SEQ ID NO:24,

(6) an amino acid sequence in which substitutions of Arg at position 38 with Lys and Arg at position 67 with Lys are introduced in the amino acid sequence represented by SEQ ID NO:24,

(7) an amino acid sequence in which substitutions of Arg at position 38 with Lys and Val at position 68 with Ala are introduced in the amino acid sequence represented by SEQ ID NO:24,

(8) an amino acid sequence in which substitutions of Arg at position 38 with Lys and Ile at position 70 with Leu are introduced in the amino acid sequence represented by SEQ ID NO:24,

(9) an amino acid sequence in which substitutions of Ala at position 9 with Pro and Met at position 48 with Ile are introduced in the amino acid sequence represented by SEQ ID NO:24,

(10) an amino acid sequence in which substitutions of Lys at position 12 with Val and Met at position 48 with Ile are introduced in the amino acid sequence represented by SEQ ID NO:24,

(11) an amino acid sequence in which substitutions of Met at position 48 with Ile and Arg at position 67 with Lys are introduced in the amino acid sequence represented by SEQ ID NO:24,

(12) an amino acid sequence in which substitutions of Met at position 48 with Ile and Val at position 68 with Ala are introduced in the amino acid sequence represented by SEQ ID NO:24,

(13) an amino acid sequence in which substitutions of Met at position 48 with Ile and Ile at position 70 with Leu are introduced in the amino acid sequence represented by SEQ ID NO:24,

(14) an amino acid sequence in which substitutions of Ala at position 9 with Pro and Tyr at position 95 with Phe are introduced in the amino acid sequence represented by SEQ ID NO:24,

(15) an amino acid sequence in which substitutions of Lys at position 12 with Val and Tyr at position 95 with Phe are introduced in the amino acid sequence represented by SEQ ID NO:24,

(16) an amino acid sequence in which substitutions of Arg at position 67 with Lys and Tyr at position 95 with Phe are introduced in the amino acid sequence represented by SEQ ID NO:24,

(17) an amino acid sequence in which substitutions of Val at position 68 with Ala and Tyr at position 95 with Phe are introduced in the amino acid sequence represented by SEQ ID NO:24,

(18) an amino acid sequence in which substitutions of Ile at position 70 with Leu and Tyr at position 95 with Phe are introduced in the amino acid sequence represented by SEQ ID NO:24.

Specific examples of the amino acid sequence of VH in which one modification is introduced include the following amino acid sequences (1) to (10):

(1) an amino acid sequence in which Ala at position 9 is substituted with Pro in the amino acid sequence represented by SEQ ID NO:24,
(2) an amino acid sequence in which Lys at position 12 is substituted with Val in the amino acid sequence represented by SEQ ID NO:24,
(3) an amino acid sequence in which Val at position 20 is substituted with Ile in the amino acid sequence represented by SEQ ID NO:24,
(4) an amino acid sequence in which Arg at position 38 is substituted with Lys in the amino acid sequence represented by SEQ ID NO:24,
(5) an amino acid sequence in which Met at position 48 is substituted with Ile in the amino acid sequence represented by SEQ ID NO:24,
(6) an amino acid sequence in which Arg at position 67 is substituted with Lys in the amino acid sequence represented by SEQ ID NO:24,
(7) an amino acid sequence in which Val at position 68 is substituted with Ala in the amino acid sequence represented by SEQ ID NO:24,
(8) an amino acid sequence in which Ile at position 70 is substituted with Leu in the amino acid sequence represented by SEQ ID NO:24,
(9) an amino acid sequence in which Tyr at position 95 is substituted with Phe in the amino acid sequence represented by SEQ ID NO:24,
(10) an amino acid sequence in which Val at position 112 is substituted with Leu in the amino acid sequence represented by SEQ ID NO:24.

Regarding VL which is included in a humanized antibody of the present invention, the following (1) to (4) are preferable:

(1) VL which comprises an amino acid sequence in which Leu at position 15, Ala at position 19, Ile at position 21, and Leu at position 84 in the amino acid sequence represented by SEQ ID NO:26 are substituted with other amino acid residues,
(2) VL which comprises an amino acid sequence in which Leu at position 15, Ala at position 19, and Leu at position 84 in the amino acid sequence represented by SEQ ID NO:26 are substituted with other amino acid residues,
(3) VL which comprises an amino acid sequence in which Ala at position 19, Ile at position 21, and Leu at position 84 in the amino acid sequence represented by SEQ ID NO:26 are substituted with other amino acid residues,
(4) VL which comprises an amino acid sequence in which Ala at position 19 and Leu at position 84 in the amino acid sequence represented by SEQ ID NO:26 are substituted with other amino acid residues.

Example of the amino acid sequences of the VL includes an amino acid sequence in which at least one modification among amino acid modifications for substituting Leu at position 15 with Ala, Ala at position 19 with Val, Ile at position 21 with Met, and Leu at position 84 with Val is introduced in the amino acid sequence represented by SEQ ID NO:26.

Specific examples of the amino acid sequences of VL in which four modifications are introduced include an amino acid sequence in which substitutions of Leu at position 15 with Ala, Ala at position 19 with Val, Ile at position 21 with Met, and Leu at position 84 with Val are introduced in the amino acid sequence represented by SEQ ID NO:26.

Specific examples of the amino acid sequence of VL in which three modifications are introduced include the following amino acid sequences (1) to (4):

(1) an amino acid sequence in which substitutions of Ala at position 19 with Val, Ile at position 21 with Met, and Leu at position 84 with Val are introduced in the amino acid sequence represented by SEQ ID NO:26,
(2) an amino acid sequence in which substitutions of Leu at position 15 with Ala, Ala Ile at position 21 with Met, and Leu at position 84 with Val are introduced in the amino acid sequence represented by SEQ ID NO:26,
(3) an amino acid sequence in which substitutions of Leu at position 15 with Ala, Ala at position 19 with Val, and Leu at position 84 with Val are introduced in the amino acid sequence represented by SEQ ID NO:26,
(4) an amino acid sequence in which substitutions of Leu at position 15 with Ala, Ala at position 19 with Val, and Ile at position 21 with Met are introduced in the amino acid sequence represented by SEQ ID NO:26.

Specific examples of the amino acid sequence of VL in which two modifications are introduced include the following amino acid sequences (1) to (6):

(1) an amino acid sequence in which substitutions of Leu at position 15 with Ala and Ala at position 19 with Val are introduced in the amino acid sequence represented by SEQ ID NO:26,
(2) an amino acid sequence in which substitutions of Leu at position 15 with Ala and Ile at position 21 with Met are introduced in the amino acid sequence represented by SEQ ID NO:26,
(3) an amino acid sequence in which substitutions of Leu at position 15 with Ala and Leu at position 84 with Val are introduced in the amino acid sequence represented by SEQ ID NO:26,
(4) an amino acid sequence in which substitutions of Ala at position 19 with Val and Ile at position 21 with Met are introduced in the amino acid sequence represented by SEQ ID NO:26,
(5) an amino acid sequence in which substitutions of Ala at position 19 with Val and Leu at position 84 with Val are introduced in the amino acid sequence represented by SEQ ID NO:26,
(6) an amino acid sequence in which substitutions of Ile at position 21 with Met and Leu at position 84 with Val are introduced in the amino acid sequence represented by SEQ ID NO:26.

Specific examples of the amino acid sequence of VL in which one modification is introduced include the following amino acid sequences (1) to (4):

(1) an amino acid sequence in which Leu at position 15 with Ala is substituted in the amino acid sequence represented by SEQ ID NO:26,
(2) an amino acid sequence in which Ala at position 19 with Val is substituted in the amino acid sequence represented by SEQ ID NO:26,
(3) an amino acid sequence in which Ile at position 21 with Met is substituted in the amino acid sequence represented by SEQ ID NO:26,
(4) an amino acid sequence in which Leu at position 84 with Val is substituted in the amino acid sequence represented by SEQ ID NO:26.

Specific examples of a humanized antibody of the present invention include the following humanized antibodies (1) to (3):

(1) a humanized antibody which comprises at least one of VH of the antibody comprising the amino acid sequence represented by SEQ ID NO:24, and VL of the antibody comprising the amino acid sequence represented by SEQ ID NO:26,
(2) a humanized antibody which comprises at least one of VH of the antibody comprising the amino acid sequence represented by SEQ ID NO:24, and VL of the antibody comprising any one amino acid sequence among amino acid sequences shown in Fig. 13,
(3) a humanized antibody which comprises at least one of VH of the antibody comprising any one amino acid sequence among amino acid sequences shown in Fig. 12, and VL of the antibody comprising the amino acid sequence represented by SEQ ID NO:26.

In addition, examples of the humanized antibody of the present invention include a humanized antibody which competes with the above humanized antibody and a humanized antibody which binds to the same epitope as that bound by the above humanized antibody.
A human antibody means an antibody naturally existing in the human body, and also includes an antibody obtained from a human antibody phage library or a human antibody-producing transgenic animal, which is prepared based on the recent advanced techniques in genetic engineering, cell engineering and developmental engineering.
The antibody naturally existing in the human body can be prepared, for example by isolating a human peripheral blood lymphocyte, immortalizing it by infecting with EB virus or the like, and then cloning it to thereby obtain lymphocytes capable of producing the antibody, culturing the lymphocytes thus obtained, and purifying the antibody from the supernatant of the culture.
The human antibody phage library is a library in which antibody fragments such as Fab and scFv are expressed on the phage surface by inserting a gene encoding an antibody prepared from a human B cell into a phage gene. A phage expressing an antibody fragment on the cell surface having the desired antigen binding activity can be recovered from the library, using its activity to bind to an antigen-immobilized substrate as the index. The antibody fragment can be converted further into a human antibody molecule consisting of two full H chains and two full L chains by genetic engineering techniques.
A human antibody-producing transgenic animal means an animal in which a human antibody gene is integrated into cells. Specifically, a human antibody-producing transgenic animal can be prepared by introducing a gene encoding a human antibody into a mouse ES cell, grafting the ES cell into an early stage embryo of other mouse and then developing it into a complete animal. A human antibody derived from the human antibody-producing transgenic non-human animal can be prepared by obtaining a human antibody-producing hybridoma by a hybridoma preparation method usually carried out in non-human animals, culturing the obtained hybridoma and producing and accumulating the human antibody in the supernatant of the culture.

An antibody or antibody fragment thereof in which one or more amino acids are deleted, substituted, inserted or added in the amino acid sequence constituting the above antibody or antibody fragment, having activity similar to the above antibody or antibody fragment is also included in the antibody or antibody fragment of the present invention.

The number of amino acid residues which are deleted, substituted, inserted and/or added is one or more, and is not specifically limited, but it is within the range where deletion, substitution or addition is possible by known methods such as the site-directed mutagenesis described in Molecular Cloning, Second Edition, Cold Spring Harbor Laboratory Press (1989); Current Protocols in Molecular Biology, John Wiley & Sons (1987-1997); Nucleic Acids Research, 10, 6487 (1982), Proc. Natl. Acad. Sci. USA, 79, 6409 (1982); Gene, 34, 315 (1985), Nucleic Acids Research, 13, 4431 (1985); Proc. Natl. Acad. Sci. USA, 82, 488 (1985) or the like. For example, the number is 1 to dozens, preferably 1 to 20, more preferably 1 to 10, and most preferably 1 to 5.

Deleting, substituting, inserting and/or adding one or more amino acid residue(s) in the amino acid sequence of the above antibody means the followings. That is, it means there is deletion, substitution, insertion or addition of one or plural amino acid residues at any positions in one or plural amino acid sequences in a single sequence. Also, the deletion, substitution, insertion or addition may exist at the same case and the amino acid which is substituted, inserted or added may be either a natural type or a non-natural type.

The natural type amino acid includes L-alanine, L-asparagine, L-aspartic acid, L-glutamine, L-glutamic acid, glycine, L-histidine, L-isoleucine, L-leucine, L-lysine, L-methionine, L-phenylalanine, L-proline, L-serine, L-threonine, L-tryptophan, L-tyrosine, L-valine, L-cysteine and the like.

Preferable examples of mutually substitutable amino acids are shown below. The amino acids in the same group are mutually substitutable.

Group A: leucine, isoleucine, norleucine, valine, norvaline, alanine, 2-aminobutanoic acid, methionine, O-methylserine, t-butylglycine, t-butylalanine, cyclohexylalanine

Group B: aspartic acid, glutamic acid, isoaspartic acid, isoglutamic acid, 2-aminoadipic acid, 2-aminosuberic acid

Group C: asparagine, glutamine

Group D: lysine, arginine, ornithine, 2,4-diaminobutanoic acid, 2,3-diaminopropionic acid

Group E: proline, 3-hydroxyproline, 4-hydroxyproline

Group F: serine, threonine, homoserine

Group G: phenylalanine, tyrosine

The antibody fragment of the present invention includes Fab, $F(ab')_2$, Fab', scFv, diabody, dsFv, a peptide comprising CDR and the like.

An Fab is an antibody fragment having a molecular weight of about 50,000 and antigen binding activity, in which about a half of the N-terminal side of H chain and the entire L chain, among fragments obtained by treating an IgG antibody molecule with a protease, papain (cleaved at an amino acid residue at position 224 of the H chain), are bound together through a disulfide bond.

The Fab of the present invention can be obtained by treating the monoclonal antibody of the present invention with papain. Also, the Fab can be produced by inserting DNA encoding Fab of the antibody into an expression vector for prokaryote or an expression vector for eukaryote, and introducing the vector into a prokaryote or an eukaryote to express the Fab.

An $F(ab')_2$ is an antibody fragment having a molecular weight of about 100,000 and having antigen binding activity and comprising two Fab regions which are bound in the hinge portion obtained by digesting the lower part of two disulfide bonds in the hinge region of IgG with an enzyme, pepsin.

The $F(ab')_2$ of the present invention can be obtained by treating the monoclonal antibody of the present invention with pepsin. Also, the $F(ab')_2$ can be produced by binding Fab' described below via a thioether bond or a disulfide bond.

An Fab' is an antibody fragment having a molecular weight of about 50,000 and antigen binding activity, which is obtained by cleaving a disulfide bond at the hinge region of the above $F(ab')_2$. The Fab' of the present invention can be obtained by treating $F(ab')_2$ of the present invention with a reducing agent, such as dithiothreitol. Also, the Fab' can be produced by inserting DNA encoding Fab' fragment of the antibody into an expression vector for prokaryote or an expression vector for eukaryote, and introducing the vector into a prokaryote or eukaryote to express the Fab'.

An scFv is a VH-P-VL or VL-P-VH polypeptide in which a VH and a VL are linked using an appropriate peptide linker (hereinafter referred to as "P") and is an antibody fragment having antigen binding activity.

The scFv of the present invention can be produced by obtaining cDNAs encoding VH and VL of the monoclonal antibody, constructing a DNA encoding the scFv, inserting the DNA into an expression vector for prokaryote or an expression vector for eukaryote, and then introducing the expression vector into a prokaryote or eukaryote to express the scFv.

A diabody is an antibody fragment wherein scFv is dimerized, is an antibody fragment having divalent antigen binding activity. In the divalent antigen binding activity, two antigens may be the same or different. The diabody of the present invention can be produced by obtaining cDNAs encoding VH and VL of the monoclonal antibody of the present invention, constructing DNA encoding scFv so that the length of the amino acid sequence of the peptide linker is 8 or less residues, inserting the DNA into an expression vector for prokaryote or an expression vector for eukaryote, and then introducing the expression vector into a prokaryote or eukaryote to express the diabody.

A dsFv is obtained by binding polypeptides in which one amino acid residue of each of VH and VL is substituted with a cysteine residue via a disulfide bond between the cysteine residues. The amino acid residue to be substituted with a cysteine residue can be selected based on a conformation prediction of the antibody in accordance with a known methods [Protein Engineering, 7, 697 (1994)].

The dsFv of the present invention can be produced by obtaining cDNAs encoding VH and VL of the monoclonal antibody of the present invention, constructing DNA encoding dsFv, inserting the DNA into an expression vector for prokaryote or an expression vector for eukaryote, and then introducing the expression vector into a prokaryote or eukaryote to express the dsFv.

A peptide comprising CDR is constituted by including one or more regions of CDRs of VH or VL. Peptides comprising plural CDRs can be connected directly or via an appropriate peptide linker.

The peptide comprising CDR of the present invention can be produced by constructing DNAs encoding CDRs of VH and VL of the monoclonal antibody of the present invention, inserting the DNAs into an expression vector for prokaryote or an expression vector for eukaryote, and then introducing the expression vector into a prokaryote or eukaryote to express the peptide. The peptide comprising CDRs can also be produced by a chemical synthesis method such as Fmoc method or tBoc method.

The present invention includes an antibody derivative in which the monoclonal antibody of the present invention is chemically or genetically bound to a radioisotope, a small-molecular weight agent, an high-molecular weight agent, a protein, a therapeutic antibody or the like.

The antibody derivative of the present invention can be produced by conjugating a radioisotope, a low-molecular-weight agent, a high-molecular-weight agent, an immunostimulator, a protein, a therapeutic antibody or the like to the N-terminal side or C-terminal side of an H chain or an L chain of the monoclonal antibody of the present invention and the antibody fragment thereof using chemical methods [Antibody Engineering Handbook, published by Chijin Shokan (1994)].

Also, the antibody derivative of the present invention can be genetically produced by linking a DNA encoding the monoclonal antibody of the present invention to other DNA encoding a protein or a therapeutic antibody to be conjugated, inserting the DNA into an expression vector, and introducing the expression vector into an appropriate host cell.

The radioisotopes include $^{131}I$, $^{125}I$, $^{90}Y$, $^{64}Cu$, $^{199}Tc$, $^{77}Lu$, $^{211}At$ and the like. The radioisotope can be directly conjugated with the antibody by Chloramine-T method or the like. Also, a substance chelating the radioisotope can be conjugated with the antibody. The chelating agent includes 1-isothiocyanatobenzyl-3-methyldiethylene-triaminepentaacetic acid (MX-DTPA) and the like.

The low-molecular-weight agents include an anti-tumor agent such as an alkylating agent, a nitrosourea agent, a metabolism antagonist, an antibiotic substance, a plant alkaloid, a topoisomerase inhibitor, a hormonotherapeutic agent, a hormone antagonist, an aromatase inhibitor, a P-glycoprotein inhibitor, a platinum complex derivative, an M-phase inhibitor and a kinase inhibitor [Rinsho Syuyo-gaku (Clinical Oncology), Gan to Kagaguryoho-Sha (1996)], a steroid agent such as hydrocortisone and prednisone, a nonsteroidal agent such as aspirin and indomethacin, immune-regulating agent such as aurothiomalate, penicillamine, immune-suppressing agent such as cyclophosphamide and azathioprine, anti-inflammatory agent such as anti-histamine agent, for example, chlorpheniramine maleate and clemastine [Ensho to Kouensho-Ryoho (Inflammation and Anti-inflammation Therapy), Ishiyaku Shuppann (1982)], and the like.

Examples of the antitumor agent include amifostine (Ethyol), cisplatin, dacarbazine (DTIC), dactinomycin, mecloretamin (nitrogen mustard), streptozocin, cyclophosphamide, iphosphamide, carmustine (BCNU), lomustine (CCNU), doxorubicin (adriamycin), epirubicin, gemcitabine (Gemsal), daunorubicin, procarbazine, mitomycin, cytarabine, etoposide, methotrexate, 5-fluorouracil, fluorouracil, vinblastine, vincristine, bleomycin, daunomycin, peplomycin, estramustine, paclitaxel (Taxol), docetaxel (Taxotea), aldesleukin, asparaginase, busulfan, carboplatin, oxaliplatin, nedaplatin, cladribine, camptothecin, 10-hydroxy-7-ethylcamptothecin (SN38), floxuridine, fludarabine, hydroxyurea, iphosphamide, idarubicin, mesna, irinotecan (CPT-11), nogitecan, mitoxantrone, topotecan, leuprolide, megestrol, melfalan, mercaptopurine, hydroxycarbamide, plicamycin, mitotane, pegasparagase, pentostatin, pipobroman, streptozocin, tamoxifen, goserelin, leuprorelin, flutamide, teniposide, testolactone, thioguanine, thiotepa, uracil mustard, vinorelbine, chlorambucil, hydrocortisone, prednisolone, methylprednisolone, vindesine, nimustine, semustine, capecitabine, Tomudex, azacytidine, UFT, oxaliplatin, gefitinib (Iressa), imatinib (STI 571), elrotinib, FMS-like tyrosine kinase 3 (Flt3) inhibitor, vascular endothelial growth facotr receptor (VEGFR) inhibitor, fibroblast growth factor receptor (FGFR) inhibitor, epidermal growth factor receptor (EGFR) inhibitor such as Iressa and Tarceva, radicicol, 17-allylamino-17-demethoxygeldanamycin, rapamycin, amsacrine, all-trans-retinoic acid, thalidomide, anastrozole, fadrozole, letrozole, exemestane, gold thiomalate, D-penicillamine, bucillamine, azathioprine, mizoribine, cyclosporine, rapamycin, hydrocortisone, bexarotene (Targretin), tamoxifen, dexamethasone, progestin substances, estrogen substances, anastrozole (Arimidex), Leuplin, aspirin, indomethacin, celecoxib, azathioprine, penicillamine, gold thiomalate, chlorpheniramine maleate, chlorpheniramine, clemastine, tretinoin, bexarotene, arsenic, voltezomib, allopurinol, calicheamicin, ibritumomab tiuxetan, Targretin, ozogamine, clarithromycin, leucovorin, ifosfamide, ketoconazole, aminoglutethimide, suramin, methotrexate, maytansinoid and derivatives thereof.

The method for conjugating the low-molecular-weight agent with the antibody include a method in which the agent and an amino group of the antibody are conjugated via glutaraldehyde, a method in which an amino group of the agent and a carboxyl group of the antibody are conjugated via water-soluble carbodiimide, and the like.

The high-molecular-weight agents include polyethylene glycol (hereinafter referred to as "PEG"), albumin, dextran, polyoxyethylene, styrene-maleic acid copolymer, polyvinylpyrrolidone, pyran copolymer, hydroxypropylmethacrylamide, and the like.

By binding these high-molecular-weight compaunds to the antibody or antibody fragment, the following effects are expected: (1) improvement of stability against various chemical, physical or biological factors, (2) remarkable prolongation of half life in blood, (3) depletion of immunogenicity or suppression of antibody production, and the like [Bioconjugate Drug, Hirokawa Shoten (1993)].

Examples of the methods for conjugating PEG to the antibody include a method for reacting an antibody with a PEG-modifying reagent [Bioconjugate Drug, Hirokawa Shoten (1993)]. Examples of the PEG-modifying reagents include a modifying agent for ε-amino group of lysine (Japanese Published Unexamined Patent Application No. 178926/86), a modifying agent for a carboxyl group of aspartic acid and glutamic acid (Japanese Published Unexamined Patent Application No. 23587/81), a modifying agent for a guanidino group of arginine (Japanese Published Unexamined Patent Application No. 117920/90) and the like.

The immunostimulator may be any natural products known as immunoadjuvants. Examples of the agents enhancing immunity include β(1→3)glucan (lentinan, schizophyllan), α-galactosylceramide and the like.

The proteins include a cytokine or a growth factor which activates a immunocompetent cell, such as NK cell, macrophage or neutrophil, a toxic protein, and the like.

Examples of the cytokines or the growth factors include interferon (hereinafter referred to as "INF")-α, INF-β, INF-γ, interleukin (hereinafter referred to as "IL")-2, IL-12, IL-15, IL-18, IL-21, IL-23, granulocyte-colony stimulating factor (G-CSF), granulocyte macrophage-colony stimulating factor (GM-CSF), macrophage-colony stimulating factor (M-CSF) and the like.

The toxic proteins include ricin, diphtheria toxin, ONTAK and the like, and also includes a toxic protein wherein mutation is introduced into a protein in order to control the toxicity.

The therapeutic antibody include an antibody against an antigen which induces apoptosis by binding of the antibody, an antibody against an antigen which participates in formation of tumor condition, an antibody against an antigen which regulates immunological function and an antibody against an antigen which relate to angiogenesis in the pathologic region.

The antigens which induces apoptosis by binding of the antibody includes cluster of differentiation (hereinafter "CD") 19, CD20, CD21, CD22, CD23, CD24, CD37, CD53, CD72, CD73, CD74, CDw75, CDw76, CD77, CDw78, CD79a, CD79b, CD80 (B7.1), CD81, CD82, CD83, CDw84, CD85, CD86 (B7.2), human leukocyte antigen (HLA)-Class II, epidermal growth factor receptor (EGFR) and the like.

The antigens participating in formation of tumor condition or the antigens for the antibody which regulates immunological function include CD4, CD40, CD40 ligand, B7 family molecule (CD80, CD86, CD274, B7-DC, B7-H2, B7-H3, B7-H4), ligand of B7 family molecule (CD28, CTLA-4, ICOS, PD-1, BTLA), OX-40, OX-40 ligand, CD137, tumor necrosis factor (TNF) receptor family molecule (DR4, DR5, TNFR1, TNFR2), TNF-related apoptosis-inducing ligand receptor (TRAIL) family molecule, receptor family of TRAIL family molecule (TRAIL-R1, TRAIL-R2, TRAIL-R3, TRAIL-R4), receptor activator of nuclear factor kappa B ligand (RANK), RANK ligand, CD25, folic acid receptor 4, cytokine [IL-1α, IL-1β, IL-4, IL-5, IL-6, IL-10, IL-13, transforming growth factor (TGF) β, TNFα, etc.], receptors of these cytokines, chemokine (SLC, ELC, I-309, TARC, MDC, CTACK, etc.) and receptors of these chemokines.

The antigens for the antibody which inhibits angiogenesis in the pathologic region include vascular endothelial growth factor (VEGF), angiopoietin, fibroblast growth factor (FGF), EGF, platelet-derived growth factor (PDGF), insulin-like growth factor (IGF), erythropoietin (EPO), TGFβ, IL-8, Ephilin, SDF-1, receptors thereof and the like.

A fusion antibody with a protein or therapeutic antibody can be produced by constructing a DNA encoding the fusion antibody in which a cDNA encoding the protein is linked to a cDNA encoding a monoclonal antibody or antibody fragment thereof, inserting the DNA into an expression vector for prokaryote or eukaryote, and then introducing the expression vector into a prokaryote or eukaryote to express.

In the case where the above antibody derivative is used for a detection method, a quantification method, a detection reagent, a quantification reagent or a diagnostic agent in the present invention, examples of the agents for conjugation to the monoclonal antibody of the present invention include a label used in general immunological detection or measuring method.

The labels include enzymes such as alkaline phosphatase, peroxidase and luciferase, luminescent materials such as acridinium ester and lophine, fluorescent materials such as fluorescein isothiocyanate (FITC) and tetramethyl rhodamine isothiocyanate (RITC), and the like.

Furthermore, the present invention includes an agent for treating a disease relating to a Trop-2 positive cell, comprising the monoclonal antibody of the present invention or the antibody fragment thereof as an active ingredient.

The disease relating to the Trop-2 positive cell is not limited, so long as it is a disease relating to a Trop-2-expressing cell, such as cancer.

The cancers include blood cancer, breast cancer, uterine cancer, colorectal cancer, esophageal cancer, gastric cancer, ovarian cancer, lung cancer, renal cancer, rectal cancer, thyroid cancer, uterine cervix cancer, small intestinal cancer, prostate cancer and pancreatic cancer. Preferable examples of the cancer include blood cancer, esophageal cancer, gastric cancer, colorectal cancer, liver cancer and prostate cancer.

The therapeutic agent comprising the monoclonal antibody of the present invention or the antibody fragment thereof, or the derivative thereof may comprise only the antibody or the antibody fragment thereof, or the derivative thereof as an active ingredient. It is generally preferred that the therapeutic agent is provided as a pharmaceutical formulation produced by mixing it with one or more pharmaceutically acceptable carriers and manufacturing by an appropriate method well known in the technical field of pharmaceutics.

It is preferred that the route of administrarion is most effective for the treatment. Examples include oral administration and parenteral administration, such as buccal, tracheal, rectal, subcutaneous, intramuscular or intravenous administration and intravenous administration is preferred.

The therapeutic agent may be in the form of spray, capsules, tablets, powder, granules, syrup, emulsion, suppository, injection, ointment, tape, and the like.

Although the dose or the frequency of administration varies depending on the objective therapeutic effect, administration method, treating period, age, body weight and the like, it is usually 10 $\mu$g/kg to 10 mg/kg per day and per adult.

Further, the present invention relates to a method for immunologically detecting or measuring Trop-2, a reagent for immunologically detecting or measuring Trop-2, a method for immunologically detecting or measuring a Trop-2-expressing cell, and a diagnostic agent for a disease relating to a Trop-2 positive cell, comprising the monoclonal antibody of the present invention or the antibody fragment thereof as an active ingredient.

In the present invention, the method for detecting or measuring the amount of Trop-2 may be any known method. For example, it includes an immunological detecting or measuring method.

The immunological detecting or measuring method means a method for detecting and measuring an antibody amount or an antigen amount using a labeled antigen or antibody. Examples of the immunological detecting or measuring method include radioimmunoassay (RIA), enzyme immunoassay (EIA or ELISA), fluorescent immunoassay (FIA), luminescent immunoassay, Western blotting method, physico-chemical method and the like.

The disease relating to Trop-2 can be diagnosed by detecting or measuring a Trop-2-expressing cell by using the monoclonal antibody or antibody fragment of the present invention.

For the detection of the Trop-2-expressing cell, known immunological detection methods can be used, and an immunoprecipitation method, a fluorescent cell staining method, an immune tissue staining method, immunofluorescence method and the like are preferably used. Also, a fluorescent antibody staining method using FMAT 8100 HTS system (Applied Biosystem) and the like can be used.

In the present invention, the living body sample to be used for detecting or measuring Trop-2 is not particularly limited, so long as it has a possibility of containing a Trop-2-expressing cell, such as tissue cells, blood, blood plasma, serum, pancreatic fluid, urine, fecal matter, tissue fluid or culture fluid.

The diagnostic agent containing the monoclonal antibody of the present invention or the antibody fragment thereof, or the derivative thereof may further contain a reagent for carrying out an antigen-antibody reaction or a reagent for detection of the reaction depending on the desired diagnostic method. The reagent for carrying out the antigen-antibody reaction includes a buffer, a salt, and the like.

The detection reagents include a reagent generally used for the immunological detecting or measuring method, such as labeled secondary antibody which recognizes the monoclonal antibody, antibody fragment thereof or derivative thereof and substrate corresponding to the labeling.

A process for producing the monoclonal antibody of the present invention, a method for treating the disease and a method for diagnosing the disease are specifically described below.

1. Preparation method of monoclonal antibodies

(1) Preparation of antigens

Trop-2 as an antigen or a Trop-2-expressing cell can be obtained by introducing an expression vector comprising cDNA encoding a full length of Trop-2 or a partial length thereof is introduced into *Escherichia coli,* yeast, an insect cell, an animal cell or the like. In addition, Trop-2 can be purified from various human tumor cell lines, human tissue and the like which express a large amount of Trop-2. The whole tumor cell line and the whole tissue can be used as antigens. Furthermore, a synthetic peptide having a partial sequence of the Trop-2 can be prepared by a chemical synthesis method such as Fmoc method or tBoc method and used as an antigen.

Trop-2 used in the present invention can be produced, for example, by expressing a DNA encoding Trop-2 in a host cell using a method described in Molecular Cloning, A Laboratory Manual, Second Edition, Cold Spring Harbor Laboratory Press (1989), Current Protocols in Molecular Biology, John Wiley & Sons (1987-1997) or the like according to the following method.

Firstly, a recombinant vector is prepared by inserting a full length cDNA comprising the region encoding Trop-2 into downstream of a promoter of an appropriate expression vector. At this time, if necessary, a DNA fragment having an appropriate length containing a region encoding the polypeptide, which is prepared based on the full length cDNA, may be used instead of the above full length cDNA. Next, a transformant producing polypeptides can be obtained by introducing the recombinant vector into a host cell suitable for the expression vector.

The expression vector may be any one, so long as it is autonomously replicable or abele to integrate into a chromosome of the host cell to be used, and comprises an appropriate promoter at a position that the DNA encoding the polypeptide can be transcribed.

The host cell may be any one, so long as it can express the objective gene. Examples include a microorganism which belongs to the genera *Escherichia,* such as *Escherichia coli,* a yeast, an insect cell, an animal cell and the like.

When a prokaryote such as *Escherichia coli* is used as the host cell, it is preferred that the recombinant vector used in the present invention is autonomously replicable in the prokaryote and comprising a promoter, a ribosome binding sequence, the DNA encoding Trop-2 and a transcription termination sequence. The recombinant vector is not necessary to have a transcription termination sequence, but a transcription termination sequence is preferably set just below the structural gene. The recombinant vector may further comprise a gene regulating the promoter.

Also, the above recombinant vector is preferably a plasmid in which the space between Shine-Dalgarno sequence, which is the ribosome binding sequence, and the initiation codon is adjusted to an appropriate distance (for example, 6 to 18 nucleotides).

Furthermore, the nucleotide sequence of the DNA encoding Trop-2 can be substituted with another base so as to be a suitable codon for expressing in a host cell, thereby improve the productivity of the objective Trop-2.

Any expression vector can be used, so long as it can function in the host cell to be used. Examples of the expression vectors include pBTrp2, pBTac1, pBTac2 (all manufactured by Roche Diagnostics), pKK233-2 (manufactured by Pharmacia), pSE280 (manufactured by Invitrogen), pGEMEX-1 (manufactured by Promega), pQE-8 (manufactured by QIAGEN), pKYP10 (Japanese Published Unexamined Patent Application No. 110600/83), pKYP200 [Agricultural Biological Chemistry, 48, 669 (1984)], pLSA1 [Agric. Biol. Chem., 53, 277 (1989)], pGEL1 [Proc. Natl. Acad. Sci. USA, 82, 4306 (1985)], pBluescript II SK(-) (manufactured by Stratagene), pTrs30 [prepared from *Escherichia coli* JM109/pTrS30 (FERM BP-5407)], pTrs32 [prepared from *Escherichia coli* JM109/pTrS32 (FERM BP-5408)], pGHA2 [prepared from *Escherichia coli* IGHA2 (FERM BP-400), Japanese Published Unexamined Patent Application No. 221091/85], pGKA2 [prepared from *Escherichia coli* IGKA2 (FERM BP-6798), Japanese Published Unexamined Patent Application No. 221091/85], pTerm2 (US4686191, US4939094, US5160735), pSupex, pUB110, pTP5, pC194, pEG400 [J. Bacteriol., 172, 2392 (1990)], pGEX (manufactured by Pharmacia), pET system (manufactured by Novagen), pME 18 SFL3 and the like.

Any promoter can be used, so long as it can function in the host cell to be used. Examples include promoters derived from *Escherichia coli,* phage and the like, such as trp promoter (Ptrp), lac promoter, PL promoter, PR promoter and T7 promoter. Also, artificially designed and modified promoters, such as a promoter in which two Ptrp are linked in tandem, tac promoter, lac T7 promoter and let I promoter, can be used.

Examples of host cell include *Escherichia coli* XL1-Blue, *Escherichia coli* XL2-Blue, *Escherichia coli* DH1, *Escherichia coli* MC1000, *Escherichia coli* KY3276, *Escherichia coli* W1485, *Escherichia coli* JM109, *Escherichia coli* HB101, *Escherichia coli* No. 49, *Escherichia coli* W3110, *Escherichia coli* NY49, *Escherichia coli* DH5α and the like.

Any introduction method of the recombinant vector can be used, so long as it is a method for introducing DNA into the host cell, and examples include a method using a calcium ion described in Proc. Natl. Acad. Sci. USA, 69, 2110 (1972), Gene, 17,107 (1982) and Molecular & General Genetics, 168, 111 (1979) and the like.

When an animal cell is used as the host cell, any expression vector can be used, so long as it can function in the animal cell. Examples include pcDNAI, pcDM8 (manufactured by Funakoshi), pAGE107 [Japanese Published Unexamined Patent Application No. 22979/91; Cytotechnology, 3, 133 (1990)], pAS3-3 (Japanese Published Unexamined Patent Application No. 227075/90), pCDM8 [Nature, 329, 840,(1987)], pcDNAI/Amp (manufactured by Invitrogen), pcDNA3.1 (manufactured by Invitrogen), pREP4 (manufactured by Invitrogen), pAGE103 [J. Biochemistry, 101, 1307 (1987)], pAGE210, pME18SFL3, pKANTEX93 (WO 97/10354) and the like.

Any promoter can be used, so long as it can function in an animal cell. Examples include a promoter of immediate early (IE) gene of cytomegalovirus (CMV), SV40 early promoter, a promoter of retrovirus, a metallothionein promoter, a heat shock promoter, SRα promoter, Molony murine leukemia virus promoter or enhancer, and the like. Also, the enhancer of the IE gene of human CMV can be used together with the promoter.

The host cell includes human Burkitt's lymphoma cell Namalwa, monkey kidney cell COS, Chinese hamster ovary cell CHO (Journal of Experimental Medicine, 108, 945 (1958); Proc .Natl. Acad. Sci. USA, 60 ,1275 (1968); Genetics, 55, 513 (1968); Chromosoma,41,129 (1973); Methods in Cell Science,18,115 (1996); Radiation Research, 148, 260 (1997); Proc. Natl. Acad. Sci. USA, 77, 4216 (1980); Proc. Natl. Acad. Sci.,60,1275 (1968); Cell, 6, 121 (1975); Molecular Cell Genetics, Appendix I, II (pp.883-900);), CHO/DG44, CHO-K1 (ATCC CCL-61), DUKXB11 (ATCC CCL-9096), Pro-5 (ATCC CCL-1781), CHO-S (Life Technologies, Cat # 11619), Pro-3, rat myeloma cell YB2/3HL.P2.G11.16Ag.20 (also referred to as YB2/0), mouse myeloma cell NS0, mouse myeloma cell SP2/0-Ag14, Syrian hamster kidney-derived BHK cell or human leukemic cell HBT5637 (Japanese Published Unexamined Patent Application No. 299/88) and the like.

Any introduction method of the recombinant vector can be used, so long as it is a method for introducing DNA into an animal cell, and examples include electroporation [Cytotechnology, 3, 133 (1990)], the calcium phosphate method (Japanese Published Unexamined Patent Application No. 227075/90), the lipofection method [Proc. Natl. Acad. Sci. USA, 84, 7413 (1987)], and the like.

Trop-2 can be produced by culturing the transformant derived from a microorganism, an animal cell or the like having a recombinant vector comprising the DNA encoding Trop-2 in a medium to form and accumulate Trop-2 in the culture, and recovering it from the culture. The method for culturing the transformant in the medium is carried out according to the usual method used in culturing of the hosts.

When Trop-2 is expressed in a cell derived from an eukaryote, Trop-2 with addition of sugars or sugar chains can be obtained.

When a microorganism transformed with a recombinant vector containing an inducible promoter is cultured, an inducer can be added to the medium, if necessary. For example, isopropyl-β-D-thiogalactopyranoside or the like can be added to the medium when a microorganism transformed with a recombinant vector using *lac* promoter is cultured; or indoleacrylic acid or the like can be added thereto when a microorganism transformed with a recombinant vector using trp promoter is cultured.

The media for culturing a transformant obtained using an animal cell as the host cell, the medium include generally used RPMI 1640 medium [The Journal of the American Medical Association, 199, 519 (1967)], Eagle's MEM medium [Science, 122, 501 (1952)], Dulbecco's modified MEM medium [Virology, 8, 396 (1959)] and 199 medium [Proceeding of the Society for the Biological Medicine, 73, 1 (1950)], Iscoove's modified Dulbecco's medium (IMDM), the media to which fetal calf serum, *etc.* is added, and the like. The culturing is carried out generally at a pH of 6 to 8 and 30 to 40°C for 1 to 7 days in the presence of 5% $CO_2$. If necessary, an antibiotic such as kanamycin or penicillin can be added to the medium during the culturing.

As the expression method for the gene encoding Trop-2, in addition to direct expression, secretory production, fusion protein expression and the like can be carried out according to the method described in Molecular Cloning, A Laboratory Manual, Second Edition, Cold Spring Harbor Laboratory Press (1989).

The process for producing Trop-2 includes a method of intracellular expression in a host cell, a method of extracellular secretion from a host cell, a method of producing on a host cell membrane outer envelope, and the like. The appropriate method can be selected by changing the host cell used and the structure of the Trop-2 produced.

When the Trop-2 is produced in a host cell or on a host cell membrane outer envelope, Trop-2 can be positively secreted extracellularly in accordance with the method of Paulson et al. [J. Biol. Chem., 264, 17619 (1989)], the method of Lowe et al. [Proc. Natl. Acad. Sci. USA, 86, 8227 (1989), Genes Develop., 4, 1288 (1990)], the methods described in Japanese Published Unexamined Patent Application No. 336963/93 and WO 94/23021, and the like.

Also, the production amount of Trop-2 can be increased in accordance with the method described in Japanese Published Unexamined Patent Application No. 227075/90 utilizing a gene amplification system using a dihydrofolate reductase gene.

The resulting Trop-2 can be isolated and purified, for example, as follows.

When Trop-2 is intracellularly expressed as a soluble form, the post-cultured cells are recovered by centrifugation, suspended in an aqueous buffer and then homogenated using ultrasonicator, French press, Manton Gaulin homogenizer, dynomill or the like to obtain a cell-free extract.

Using a supernatant derived by centrifugation of the cell-free extract, a purified preparation can be obtained by a general protein isolation and purification techniques such as solvent extraction; salting out with ammonium sulfate *etc.;* desalting; organic solvent precipitation; anion exchange chromatography using a resin such as diethylaminoethyl (DEAE)-sepharose, DIAION HPA-75 (manufactured by Mitsubishi Chemical); cation exchange chromatography using a resin such as S-Sepharose FF (manufactured by Pharmacia); hydrophobic chromatography using a resin such as butyl-Sepharose or phenyl-Sepharose; gel filtration using a molecular sieve; affinity chromatography; chromatofocusing; electrophoresis such as isoelectric focusing; and the like which may be used alone or in combination.

When Trop-2 is expressed intracellularly by forming an inclusion body, the cells are recovered, homogenated and centrifuged in the same manner as the described above, and the inclusion body of Trop-2 are recovered as a precipitation fraction. The recovered inclusion body of Trop-2 is solubilized with a protein denaturing agent. The Trop-2 is turned back into a normal conformation by diluting or dialyzing the solubilized solution, and then a purified preparation of Trop-2 is obtained by the same isolation purification method as above.

When Trop-2 or the derivative thereof such as a glycosylated product is secreted extracellularly, Trop-2 or the derivative such as a glycosylated product can be recovered from the culture supernatant. A purified preparation of Trop-2 can be obtained from the culture supernatant by preparing a soluble fraction using a method such as centrifugation in the same manner as described above and applying the same isolation purification method as described above.

Also, Trop-2 used in the present invention can be produced by a chemical synthesis method, such as Fmoc method or tBoc method. Also, it can be chemically synthesized using a peptide synthesizer manufactured by Advanced ChemTech, Perkin-Elmer, Pharmacia, Protein Technology Instrument, Synthecell-Vega, PerSeptive, Shimadzu Corporation, or the like.

(2) Immunization of animal and preparation of antibody-producing cells for fusion

A mouse, rat or hamster 3 to 20 weeks old is immunized with the antigen prepared in the above (1), and antibody-producing cells are collected from the spleen, lymph node or peripheral blood of the animal. Also, when the increase of a sufficient titer in the above animal is not found due to low immunogenecity, a Trop-2 knockout mouse may be used as an animal to be immunized.

The immunization is carried out by administering the antigen to the animal through subcutaneous, intravenous or intraperitoneal injection together with an appropriate adjuvant (such as complete Freund's adjuvant, combination of aluminum hydroxide gel with pertussis vaccine). When a partial peptide is used as the antigen, a conjugate with a carrier protein such as BSA (bovine serum albumin), KLH (keyhole limpet hemocyanin) or the like is produced to use as an immunogen.

The administration of the antigen is carried out 5 to 10 times every one week or every two weeks after the first administration. On the 3rd to 7th day after each administration, a blood sample is collected from the eyeground venous plexus to determine antibody titer of the serum by enzyme immunoassay [Antibodies-A Laboratory Manual, Cold Spring Harbor Laboratory (1988)] or the like. An animal showing a sufficient antibody titer in their sera against the antigen used for the immunization is used as a donor of antibody-producing cells for fusion.

Three to seven days after final administration of the antigen, tissue containing the antibody-producing cells such as the spleen from the immunized animal is excised to collect the antibody-producing cells. When spleen cells are used, the spleen is cut out and loosened, followed by centrifuged, and then erythrocytes are removed to obtain antibody-producing cells for fusion.

(3) Preparation of myeloma cells

Cell lines established from a mouse are used as myeloma cells. Examples include 8-azaguanine-resistant mouse (derived from BALB/c) myeloma cell line P3-X63Ag8-U1 (P3-U1) [Current Topics in Microbiology and Immunology, 18, 1 (1978)], P3-NS1/1-Ag41 (NS-1) [European J. Immunology, 6, 511 (1976)], SP2/0-Ag14 (SP-2) [Nature, 276, 269 (1978)], P3-X63-Ag8653 (653) [J. Immunology, 123, 1548 (1979)], P3-X63-Ag8 (X63) [Nature, 256,495 (1975)] and the like.

The myeloma cells are subcultured in a normal medium [RPMI1640 medium containing glutamine, 2-mercaptoethanol, gentamicin, FBS and 8-azaguanine] and then cultured in the normal medium 3 or 4 days before cell fusion to ensure the cell number of $2 \times 10^7$ or more on the day for fusion.

(4) Cell fusion and preparation of hybridomas for producing monoclonal antibodies

The antibody-producing cells for fusion obtained by the above (2) and the myeloma cells obtained by the above (3) were thoroughly washed with a minimum essential medium (MEM) or PBS (1.83 g of disodium hydrogen phosphate, 0.21 g of potassium dihydrogen phosphate, 7.65 g of sodium chloride, 1 liter of distilled water, pH 7.2) and mixed to give a ratio of the antibody-producing cells: the myeloma cells = 5 to 10: 1, followed by centrifugation, and then, the supernatant is discarded.

After thoroughly loosening the precipitated cell clumps, the mixture of polyethylene glycol-1000 (PEG-1000), MEM and dimethylsulfoxide is added to the cell under stirring at 37°C. In addition, 1 to 2 mL of MEM medium is added several times every one or two minutes, and MEM is added to give a total amount of 50 mL. After centrifugation, the supernatant is discarded. After the cell clumps are gently loosen, the cells are gently suspended in HAT medium [the normal medium containing hypoxanthine, thymidine and aminopterin]. The suspension is cultured in a 5% $CO_2$ incubator for 7 to 14 days at 37°C.

After the culturing, a portion of the culture supernatant is sampled and cell lines which is reactive to an antigen with Trop-2 and is not reactive to an antigen without Trop-2 is selected by binding assay as described below or the like. Then, cloning is carried out twice by a limiting dilution method [Firstly, HT medium (in the first round, HAT medium without aminopterin) is used, and sin the second round, the normal medium is used], and a hybridoma which shows a stably high antibody titer is selected as the monoclonal antibody-producing hybridoma.

(5) Preparation of purified monoclonal antibodies

The hybridoma cells producing a monoclonal antibody obtained by the above (4) are administered by intraperitoneal injection into 8- to 10-week-old mice or nude mice pre-treated with 0.5 mL of pristane (2,6,10,14-tetramethylpentadecane (Pristane) is intraperitoneally administered, followed by feeding for 2 weeks). The hybridoma forms ascites tumor in 10 to 21 days. The ascitic fluid is collected from the mice, centrifuged to remove solids, subjected to salting out with 40 to 50% ammonium sulfate and then precipitated by caprylic acid, passed through a DEAE-Sepharose column, a protein A column or a gel filtration column to collect IgG or IgM fractions as purified monoclonal antibodies.

Furthermore, after a monoclonal antibody-producing hybridoma obtained by the above (4) is cultured in RPMI1640 medium containing 10% FBS or the like, the supernatant is removed by centrifugation. The precipitated cells are suspended in Hybridoma SFM medium and cultured for 3 to 7 days. The purified monoclonal antibody can be obtained by centrifusing the obtained cell suspension, followed by purifying the resulting supernatant with Protein A colomun or Protein G colomn to collect the IgG fractions. Hybridoma SFM medium may contain 5% DIGO GF21.

The subclass of the antibody can be determined using a subclass typing kit by enzyme immunoassay. The amount of the protein can be calculated by the Lowry method or from the absorbance at 280 nm.

(6) Selection of monoclonal antibodies

Selection of monoclonal antibody is carried out by the following binding assay using an enzyme immunoassay method, and a kinetic analysis using Biacore. Other than these methods, a target antigen can be identified by a publically-known method (The Prostane, 67, 1163 (2007)).

(6-a) Binding assay

As the antigen, a gene-introduced cell or a recombinant protein obtained by introducing an expression vector containing a cDNA encoding Trop-2 obtained in (1) into *Escherichia coli,* yeast, an insect cell, an animal cell or the like, or a purified polypeptide or a partial peptide obtained from a human tissue is used. When a partial peptide is used as the antigen, a conjugate with a carrier protein such as BSA or KLH is prepared to be used.

After immobilizing these antigens as a solid layer by dispensing in a 96-well plate, a test substance such as serum, a culture supernatant of a hybridoma or a purified monoclonal antibody is dispensed therein as the primary antibody and allowed to react. After thoroughly washing with PBS or PBS-Tween, an anti-immunoglobulin antibody labeled with biotin, an enzyme, a chemiluminescent material, a radiation compound or the like is dispensed therein as the secondary antibody and allowed to react. After thoroughly washing with PBS-Tween, the reaction depending on the label of the secondary antibody is carried out to select a monoclonal antibody which specifically reacts with the antigen.

In addition, the antibody which competes with the monoclonal antibody of the present invention can be obtained by adding a test antibody to the above-mentioned binding assay system and carrying out reaction. That is, a monoclonal antibody which competes for binding to an extracellular region of Trop-2 with the obtained monoclonal antibody can be prepared by screening an antibody which inhibits the binding of the monoclonal antibody when the test antibody is added.

Furthermore, an antibody which binds to an epitope which is the same as the epitope recognized by the monoclonal antibody of the present invention can be obtained by identifying the epitope of the antibody obtained in the above binding assay, and preparing a partial synthetic peptide, a synthetic peptide mimicking the conformation of the epitope or the like, followed by immunization.

(6-b) Kinetic analysis with Biacore

The kinetics between an antigen and a test substance is measured using Biacore T100 and then the obtained results are analyzed using analysis software accompanied with the apparatus. After anti-IgG mouse antibody is immobilized onto a CM5 sensor chip by an amine coupling method, a test substance such as culture supernatant of a hybridoma, a purified antibody is allowed to flow, bind at an appropriate amount, and further flow an antigen at plural known concentrations, followed by measuring the binding and dissociation.

Using the obtained data and the software accompanied with the apparatus, the kinetics analysis is carried out using the 1:1 binding model to obtain various parameters. Otherwise, after human Trop-2 is immobilized onto the sensor chip by an amine coupling method or the like, a purified monoclonal antibody is allowed to flow at plural known concentrations followed by measuring the binding and dissociation. Using the obtained data and the software accompanied with the apparatus, the kinetics analysis is carried out using bivalent binding model to obtain various parameters.

2. Preparation of recombinant antibodies

As preparation examples of recombinant antibodies, the methods for producing a human chimeric antibody and a humanized antibody are shown below.

(1) Construction of an expression vector of a recombinant antibody

An expression vector of a recombinant antibody is an expression vector for animal cell into which DNAs encoding CH and CL of a human antibody have been inserted, and can be constructed by cloning each of DNAs encoding CH and CL of a human antibody into an expression vector for animal cell.

The C region of a human antibody may be CH and CL of any human antibody. Examples include CH belonging to $\gamma$1 subclass, CL belonging to $\kappa$ class, and the like. As the DNAs encoding CH and CL of a human antibody, the cDNA may be generally used and a chromosomal DNA comprising an exon and an intron can be also used.

As the expression vector for animal cell, any expression vector can be used, so long as a gene encoding the C region of a human antibody can be inserted thereinto and expressed therein. Examples include pAGE107 [Cytotechnol., 3, 133 (1990)], pAGE103 [J. Biochem., 101, 1307 (1987)], pHSG274 [Gene, 27, 223 (1984)], pKCR [Proc. Natl. Acad. Sci. USA, 78, 1527 (1981)], pSG1bd2-4 [Cytotechnol., 4, 173 (1990)], pSE1UK1Sed1-3 [Cytotechnol., 13, 79 (1993)] and the like.

Examples of promoters and enhancers used for expression vectors for animal cells include an SV40 early promoter [J. Biochem., 101, 1307 (1987)], a Moloney mouse leukemia virus LTR [Biochem. Biophys. Res. Commun., 149, 960 (1987)], an immunoglobulin H chain promoter [Cell, 41, 479 (1985)] and enhancer [Cell, 33, 717 (1983)] and the like.

The exoression vector of a recombinant antibody may be either of a type in which a gene encoding an antibody H chain and a gene encoding an antibody L chain exist on separate vectors or of a type in which both genes exist on the same vector (tandem type). In respect of easiness of construction of an expression vector for of recombinant antibody, easiness of introduction into animal cells, and balance between the expression amounts of antibody H and L chains in animal cells, a tandem type of the expression vector of a recombinant antibody is more preferred [J. Immunol. Methods, 167, 271 (1994)]. Examples of the tandem type of the expression vector of a recombinant antibody include pKANTEX93 (WO 97/10354), pEE18 [Hybridoma, 17, 559 (1998)], and the like.

(2) Obtaining of cDNAs encoding V regions of an antibody derived from a non-human animal and analysis of amino acid sequences

Obtaining of cDNAs encoding VH and VL of a non-human animal antibody and analysis of amino acid sequence are carried out as follows. mRNA is extracted from hybridoma cells producing a non-human animal antibody to synthesize cDNA. The synthesized cDNA is cloned into a vector such as a phage or a plasmid, to prepare a cDNA library.

Each of a recombinant phage or recombinant plasmid containing cDNA encoding VH or VL is isolated from the library using DNA encoding a part of the C region or V region of a mouse antibody as a probe. The full length of the objected nucleotide sequences of VH and VL of a mouse antibody on the recombinant phage or recombinant plasmid are determined, and the full length of the amino acid sequences of VH and VL are deduced from the nucleotide sequences, respectively.

As the non-human animal for preparing a hybridoma cell which produces a non-human antibody, mouse, rat, hamster, rabbit or the like can be used. Any animals can be used so long as a hybridoma cell can be produced therefrom.

For preparing total RNA from a hybridoma cell, a guanidine thiocyanate-cesium trifluoroacetate method [Methods in Enzymol., 154, 3 (1987)], the use of a kit such as RNA easy kit (manufactured by Qiagen) or the like may be used.

For preparing mRNA from total RNA, an oligo (dT) immobilized cellulose column method [Molecular Cloning, A Laboratory Manual, Second Edition, Cold Spring Harbor Laboratory Press (1989)], a kit such as Oligo-dT30 <Super> mRNA Purification Kit (manufactured by Takara Bio) or the like may be used.

Also, mRNA can be prepared from a hybridoma cell using Fast Track mRNA Isolation Kit (manufactured by Invitrogen), Quick Prep mRNA Purification Kit (manufactured by Pharmacia) and the like.

For synthesizing cDNA and preparing a cDNA library, publically-known methods [Molecular Cloning, A Laboratory Manual, Cold Spring Harbor Lab. Press (1989); Current Protocols in Molecular Biology, Supplement 1, John Wiley & Sons (1987-1997)]; a kit such as Super Script Plasmid System for cDNA Synthesis and Plasmid Cloning (manufactured by GIBCO BRL), ZAP-cDNA Kit (manufactured by Stratagene), *etc.;* or the like may be used.

As the vector for insertion of the synthesized cDNA templated by mRNA extracted from a hybridoma cell, any vector can be used for preparing a cDNA library may be any vector, so long as the cDNA can be inserted. Examples include ZAP Express [Strategies, 5, 58 (1992)], pBluescript II SK(+) [Nucleic Acids Research, 17, 9494 (1989)], λzapII (manufactured by Stratagene), λgt10 and λgt11 [DNA Cloning: A Practical Approach, I, 49 (1985)], Lambda BlueMid (manufactured by Clontech), λExCell and pT7T3 18U (manufactured by Pharmacia), pcD2 [Mol. Cell. Biol., 3, 280 (1983)], pUC18 [Gene, 33, 103 (1985)], and the like.

Any *Escherichia coli* for introducing the cDNA library constructed by a phage or plasmid vector may be used, so long as the cDNA library can be introduced, expressed and maintained. Examples include XL1-Blue MRF[t] [Strategies, 5, 81 (1992)], C600 [Genetics, 39, 440 (1954)], Y1088 and Y1090 [Science, 222: 778 (1983)], NM522 [J. Mol. Biol., 166, 1 (1983)], K802 [J. Mol. Biol., 16, 118 (1966)], JM105 [Gene, 38, 275 (1985)], and the like.

A colony hybridization or plaque hybridization method using an isotope- or fluorescence-labeled probe may be used for selecting cDNA clones encoding VH or VL of a non-human antibody or the like from the cDNA library [Molecular Cloning, A Laboratory Manual, Second Edition, Cold Spring Harbor Laboratory Press (1989)].

Also, the cDNAs encoding VH or VL can be prepared by synthesizing primers and performing polymerase chain reaction (hereinafter referred to as "PCR"; Molecular Cloning, A Laboratory Manual, Second Edition, Cold Spring Harbor Laboratory Press (1989); Current Protocols in Molecular Biology, Supplement 1, John Wiley & Sons (1987-1997)) using cDNAs synthesized from mRNAs or a cDNA library as a template.

The nucleotide sequence of the cDNA can be determined by digesting the selected cDNA with appropriate restriction enzymes or the like, cloning the fragments into a plasmid such as pBluescript SK(-) (manufactured by Stratagene), carrying out a genarally-used method of nucleotide sequence analysis. As a method for nucleotide sequence analysis, an automatic nucleotide sequence analyzer such as ABI PRISM3700 (manufactured by PE Biosystems), A.L.F. DNA sequencer (manufactured by Pharmacia) or the like may be used after a reaction such as the dideoxy method [Proc. Natl. Acad. Sci. USA, 74, 5463 (1977)].

Whether the obtained cDNAs encode the complete amino acid sequences of VH and VL of the antibody containing a secretory signal sequence can be confirmed by estimating the whole amino acid sequences of VH and VL from the determined nucleotide sequence and comparing them with the whole amino acid sequences of VH and VL of known antibodies [Sequences of Proteins of Immunological Interest, US Dept. Health and Human Services (1991)].

Regarding the complete amino acid sequences of VL and VH of the antibody comprising a secretory signal sequence, the length of the secretory signal sequence and N-terminal amino acid sequence can be deduced by comparing the whole amino acid sequences of VH and VL of the antibody with the whole amino acid sequences of VH and VL of known antibodies [Sequences of Proteins of Immunological Interest, US Dept. Health and Human Services (1991)], and further the subgroup to which they belong can also be known. Furthermore, the amino acid sequence of each of CDRs of VH and VL can be identified by comparing with amino acid sequences of VH and VL of known antibodies [Sequences of Proteins of Immunological Interest, US Dept. Health and Human Services (1991)].

Moreover, the novelty of the complete amino acid sequence of VH and VL can be confirmed by carrying out a homology search such as BLAST method [J. Mol. Biol., 215, 403 (1990)] with the obtained complete amino acid sequences of VH and VL in arbitrary database such as SWISS-PROT and PIR-Protein.

(3) Construction of an expression vector of a human chimeric antibody

cDNAs encoding each of VH and VL of a non-human animal antibody are cloned in the upstream of genes encoding CH and CL of human antibody of an expression vector of the recombinant antibody mentioned in the above (1) to thereby construct an expression vector of a human chimeric antibody.

In order to ligate a 3'-terminal of cDNA encoding VH or VL of a non-human animal antibody and a 5'-terminal of CH or CL of a human antibody, each cDNA encoding VH and VL of of a non-human animal antibody is constructed so that a nucleotide sequence of a linkage portion would encode appropriate amino acids and have an appropriate recognition sequence of a restriction enzyme.

An expression vector for human chimeric antibody is constructed by cloning the obtained cDNAs encoding VH and VL respectively in the upstream of gene encoding CH or CL of a human antibody of the expression vector for human CDR-grafted antibody mentioned in the above (1) so that each of them is expressed in an appropriate form.

In addition, cDNA encoding VH and VL of a non-human antibody is amplified respectively by PCR using a synthetic DNA having a recognition sequence of an appropriate restriction enzyme at both ends and each of them is cloned to the expression vector of the recombinant antibody obtained in the above (1).

(4) Construction of cDNAs encoding V regions of a humanized antibody

A cDNA encoding VH or VL of a humanized antibody can be obtained as follows.

Amino acid sequences of framework regions (hereinafter referred to as "FR") in VH or VL of a human antibody, to which amino acid sequences of CDRs in VH or VL of an antibody derived from a non-human antibody are grafted are respectively selected. Any amino acid sequences of FRs of a human antibody can be used, so long as they are derived from human.

Examples include amino acid sequences of FRs of human antibodies registered in database such as Protein Data Bank or the like, and amino acid sequences common to subgroups of FRs of human antibodies [Sequences of Proteins of Immunological Interest, US Dept. Health and Human Services (1991)], and the like.

In order to reduce the loss in the binding activity of the antibody, amino acid sequences having high homology (at least 60% or more) with the amino acid sequence of FR in VH or VL of the original antibody is selected.

Then, amino acid sequences of CDRs of the original antibody are grafted to the selected amino acid sequence of FRs in VH and VL of the human antibody, respectively, to design each amino acid sequence of VH and VL of a human CDR-grafted antibody. The designed amino acid sequences are converted to DNA sequences by considering the frequency of codon usage found in nucleotide sequences of genes of antibodies [Sequence of Proteins of Immunological Interest, US Dept. Health and Human Services (1991)], and the DNA sequences encoding the amino acid sequences of VH and VL of a human CDR-grafted antibody are designed, respectively.

Based on the designed nucleotide sequences, several synthetic DNAs consisting of a length of about 100 nucleotides are synthesized, and PCR is carried out using them. In this case, it is preferred that 6 synthetic DNAs per each of the VH and the VL are designed in view of the reaction efficiency of PCR and the DNA lengths possibly synthesized. Furthermore, the cDNA encoding VH or VL of a human CDR-grafted antibody can be easily cloned into the expression vector of human CDR-grafted antibody constructed in (1) by introducing the recognition sequence of an appropriate restriction enzyme to each 5' terminal of the synthetic DNAs existing on the both ends.

After the PCR, each amplified product is cloned into a plasmid such as pBluescript SK (-) (manufactured by Stratagene) or the like, and the nucleotide sequence is determined according to a method similar to the method described in (2) to obtain a plasmid comprising a DNA sequence encoding the amino acid sequence of VH or VL of a desired human CDR-grafted antibody.

Otherwise, based on the designed DNA sequence, a synthetic DNA as one DNA encoding each of the full-length VH and the full-length VL can be used instead of the above PCR amplification products. Furthermore, the cDNA encoding VH or VL of a human CDR-grafted antibody can be easily cloned into the expression vector of human CDR-grafted antibody constructed in (1) by introducing the recognition sequence of an appropriate restriction enzyme to the both ends of the synthetic DNAs.

(5) Modification of amino acid sequences of V regions of a human CDR-grafted antibody

It is known that by just grafting only CDRs in VH and VL of a non-human antibody into FRs of VH and VL of a human antibody, the antigen binding activity decreases compared to the original non-human antibody [BIO/TECHNOLOGY, 9, 266 (1991)].

In human CDR-grafted antibodies, among the amino acid sequences of FRs in VH and VL of a human antibody, amino acid residues which directly relate to binding to an antigen, amino acid residues which interact with amino acid residues in CDRs, and amino acid residues which maintain the conformation of an antibody and indirectly relate to binding to an antigen are identified and modified to an amino acid residues which are found in the original non-human antibody to thereby increase the antigen binding activity which has been decreased.

In order to identify the amino acid residues relating to the antigen binding activity in FRs, the three-dimensional structure of an antibody can be constructed and analyzed by X-ray crystallography [J. Mol. Biol., 112, 535 (1977)], computer-modeling [Protein Engineering, 7, 1501 (1994)] or the like.

In addition, a modified human CDR-grafted antibody having a sufficient antibody-binding activity can be obtained by repetition of various attempts for producing several modified antibodies of each antibody and examining the correlation between each of the modified antibodies and its antibody binding activity.

The amino acid sequences of FRs in VH and VL of a human antibody can be modified using various synthetic DNA for modification by PCR as described in (4). The nucleotide sequence of the amplified product obtained by the PCR is determined according to the method as described in (2) so as to confirm that the designed modification has been generated.

(6) Construction of an expression vector of a human CDR-grafted antibody

An expression vector for of a human CDR-grafted antibody can be constructed by cloning each cDNA encoding VH or VL of a constructed recombinant antibody into upstream of each gene encoding CH or CL of the human antibody in the expression vector of the recombinant antibody as described in (1).

For example, by introducing a recognition sequences of appropriate restriction enzymes on the 5'-terminals of synthetic DNAs, which are selected to be positioned at both ends among synthetic DNAs used for the construction of VH or VL of the human CDR-grafted antibody in (4) and (5), VH and VL can be cloned into the upstream of each gene encoding CH or CL of the human antibody in the expression vector of the human CDR-grafted antibody as described in (1), so that they are expressed in appropriate forms.

(7) Transient expression of a recombinant antibody

The antigen binding activity of various human CDR-grafted antibodies produced can be efficiently evaluated by transiently expressing the recombinant antibodies using the expression vector of the recombinant antibody as described in (3) and (6) or the modified expression vector thereof.

Any cell can be used as a host cell, so long as the host cell can express a recombinant antibody. Examples include COS-7 cell (ATCC CRL1651) [Methods in Nucleic Acids Res., CRC Press, 283 (1991)].

Examples of the method for introducing the expression vector into COS-7 cell include a DEAE-dextran method [Methods in Nucleic Acids Res., CRC Press, 283 (1991)], a lipofection method [Proc. Natl. Acad. Sci. USA, 84, 7413 (1987)], and the like.

After introduction of the expression vector, the expression amount and antigen binding activity of the recombinant antibody in the culture supernatant can be determined by the enzyme immunoassay [Monoclonal Antibodies-Principles and practice, Third edition, Academic Press (1996), Antibodies-A Laboratory Manual, Cold Spring Harbor Laboratory (1988), Monoclonal Antibody Experiment Manual, Kodansha Scientific (1987)] and the like.

(8) Obtaining transformants which stably expresses a recombinant antibody and preparation of a recombinant antibody

A transformant which stably expresses a recombinant antibody can be obtained by introducing the expression vector of a recombinant antibody described in (3) and (6) into an appropriate host cell.

Examples of the methods for introducing the expression vector into a host cell include electroporation [Japanese Published Unexamined Patent Application No. 257891/90, Cytotechnology, 3, 133 (1990)] and the like.

As the host cell for introduction of an expression vector of a recombinant antbody, any cell can be used, so long as it is a host cell which can produce the recombinant antibody. Examples include Chinese hamster ovary cell CHO-K1 (ATCC CCL-61), DUKXB11 (ATCC CCL-9096), Pro-5 (ATCC CCL-1781), CHO-S (Life Technologies, Cat # 11619), rat myeloma cell YB2/3HL.P2.G11.16Ag.20 (also referred to as YB2/0), mouse myeloma cell NSO, mouse myeloma cell SP2/0-Ag14 (ATCC No. CRL1581), mouse myeloma P3X63-Ag8.653 cell (ATCC No. CRC1580), CHO cell deficient in dihydrofolate reductase gene (hereinafter referred to as "dhfr") [Proc. Natl. Acad. Sci. U.S.A., 77, 4216 (1980)], and the like.

In addition, host cells in which activity of a protein such as an enzyme relating to synthesis of GDP-fucose, which is an intracellular sugar nucleotide, a protein such as an enzyme relating to the modification of a sugar chain in which 1-position of fucose is bound to 6-position of N-acetylglucosamine in the reducing end through $\alpha$-bond in a complex type N-glycoside-linked sugar chain, or a protein relating to transport of GDP-fucose, which is an intracellular sugar nucleotide, to the Golgi body are introduced is decreased or deleted, such as CHO cell deficient in $\alpha$1,6-fucosyltransferase gene (WO05/35586, WO02/31140), Lec13 acquiring a lectin resistance [Somatic Cell and Molecular genetics, 12, 55 (1986)] or the like, can also be used.

After introduction of the expression vector, transformants which stably express a recombinant antibody are selected by culturing in an animal cell culture medium containing an agent such as G418 sulfate (hereinafter referred to as "G418") or the like (Japanese Published Unexamined Patent Application No. 257891/90).

Examples of the animal cell culture media include RPMI1640 medium (manufactured by Invitrogen), GIT medium (manufactured by Nihon Pharmaceutical), EX-CELL301 medium (manufactured by JRH), IMDM medium (manufactured by Invitrogen), Hybridoma-SFM medium (manufactured by Invitrogen), media thereby obtaining various additives such as fetal calf serum (hereinafter referred to as "FCS"), and the like.

The recombinant antibody can be produced and accumulated in a culture supernatant by culturing the obtained transformants in a medium. The expression amount and antigen binding activity of the recombinant antibody in the culture supernatant can be measured by ELISA or the like. Also, in the transformant, the expression amount of the recombinant antibody can be increased by DHFR amplification system (Japanese Published Unexamined Patent Application No. 257891/90) or the like.

The recombinant antibody can be purified from the culture supernatant of the transformant by a protein A column [Monoclonal Antibodies-Principles and practice, Third edition, Academic Press (1996), Antibodies-A Laboratory Manual, Cold Spring Harbor Laboratory (1988)]. In addition, a method for use in protein purification such as gel filtration, ion-exchange chromatography, ultrafiltration can be combimed.

The molecular weight of the H chain or the L chain of the purified recombinant antibody or of the whole antibody molecule is determined by polyacrylamide gel electrophoresis [Nature, 227, 680 (1970)], Western blotting [Monoclonal Antibodies-Principles and practice, Third edition, Academic Press (1996), Antibodies-A Laboratory Manual, Cold Spring Harbor Laboratory (1988)], and the like.

3. Activity evaluation of the monoclonal antibodies or antibody fragments

The activity of the purified monoclonal antibody or antibody fragment of the present invention can be evaluated in the following manner. The binding activity to Trop-2-expressing cell is evaluated by the binding assay described in the above 1-(6-a) and a surface plasmon resonance method using such as Biacore system described in the above (6-b).

Furthermore, the binding activity can be measured by fluorescent antibody technique [Cancer Immunol. Immunother., 36, 373 (1993)] or the like. In addition, CDC activity or ADCC activity against an antigen positive cultured cell line is evaluated by a known method [Cancer Immunol. Immunother., 36, 373 (1993)].

4. Method of controlling effector activity of antibodies

As a method for controlling an effector activity of the monoclonal antibody of the present invention, a method for controlling an amount of fucose (hereinafter, referred to also as "core fucose") which is bound in $\alpha$-1,6 linkage to N-acetylglucosamine (GlcNAc) present in a reducing end of a complex type N-linked sugar chain which is bound to asparagine (Asn) at position 297 of an Fragment, crystalizable region (hereinafter reffered to as Fc region) of an antibody (WO2005/035586, WO2002/31140, and WO00/61739), a method for controlling an effector activity of a monoclonal antibody by modifying amino acid group(s) of an Fc region of the antibody, and the like are known. The effector activity of the monoclonal antibody of the present invention can be controlled by using any of the methods.

The effector activity means an antibody-dependent activity which is induced via an Fc region of an antibody. As the effector activity, an antibody-dependent cellular cytotoxicity (ADCC activity), a complement-dependent cytotoxicity (CDC activity), an antibody-dependent phagocytosis (ADP activity) by phagocytic cells such as macrophages or dendritic cells, and the like are known.

By controlling a content of core fucose of a complex type N-linked sugar chain of Fc, an effector activity of the antibody can be increased or decreased. As a method for reducing a content of fucose which is bound to a complex type N-linked sugar chain bound to Fc of the antibody, an antibody to which fucose is not bound can be obtained by the expression of an antibody using a CHO cell which is deficient in $\alpha$1,6-fucosyltransferase gene. The antibody to which fucose is not bound has a high ADCC activity.

On the other hand, as a method for increasing a content of fucose which is bound to a complex type N-linked sugar chain bound to Fc of an antibody, an antibody to which fucose is bound can be obtained by the expression of an antibody using a host cell into with introduction of $\alpha$1,6-fucosyltransferase gene is introduced. The antibody to which fucose is bound has a lower ADCC activity than the antibody to which fucose is not bound.

Further, by modifying amino acid residue(s) in an Fc region of an antibody, the ADCC activity or CDC activity can be increased or decreased. For example, the CDC activity to an antibody can be increased by using the amino acid sequence of the Fc region described in US2007/0148165. Further, the ADCC activity or CDC activity can be increased or decreased by modifying the amino acid as described in US Patent Nos. 6,737,056, 7,297,775 or 7,317,091.

Furthermore, an antibody in which the effector actibity is controlled can be obtained by combining the above method.

5. Method for treating disease using the monoclonal antibody or antibody fragment of the present invention

The monoclonal antibody or an antibody fragment thereof of the present invention can be used for treating a disease relating to a Trop-2 positive cell.

The therapeutic agent comprising the antibody or antibody fragment of the present invention or derivatives thereof may contain only the antibody or antibody fragment or derivatives thereof as an active ingredient, and is typically supplied as a pharmaceutical preparation produced by an publically-known method in the technical field of pharmaceutics, by mixing it with one or more pharmaceutically acceptable carriers.

Examples of a route of administration include oral administration and parenteral administration, such as buccal, tracheal, rectal, subcutaneous, intramuscular or intravenous administration. Examples of the dosage form includes sprays, capsules, tablets, powder, granules, syrups, emulsions, suppositories, injections, ointments, tapes and the like.

The pharmaceutical preparation suitable for oral administration includes emulsions, syrups, capsules, tablets, powders, granules and the like.

Liquid preparations such as emulsions and syrups can be produced using, as additives, water; sugars such as sucrose, sorbitol and fructose; glycols such as polyethylene glycol and propylene glycol; oils such as sesame oil, olive oil and soybean oil; antiseptics such as p-hydroxybenzoic acid esters; flavors such as strawberry flavor and peppermint; and the like.

Capsules, tablets, powders, granules and the like can be produced using, as additives, excipients such as lactose, glucose, sucrose and mannitol; disintegrating agents such as starch and sodium alginate; lubricants such as magnesium stearate and talc; binders such as polyvinyl alcohol, hydroxypropylcellulose and gelatin; surfactants such as fatty acid ester; plasticizers such as glycerin; and the like.

The pharmaceutical preparation suitable for parenteral administration includes injections, suppositories, sprays and the like.

Injections can be prepared using a carrier such as a salt solution, a glucose solution or a mixture of both thereof.

Suppositories can be prepared using a carrier such as cacao butter, hydrogenated fat or carboxylic acid.

Sprays can be prepared using the antibody or antibody fragment as such or using it together with a carrier which does not stimulate the buccal or airway mucous membrane of the patient and can facilitate absorption of the compound by dispersing it as fine particles. The carrier includes lactose, glycerol and the like. It is possible to produce pharmaceutical preparations such as aerosols and dry powders

In addition, the components exemplified as additives for oral preparations can also be added to the parenteral preparations.

6. Method for diagnosing diseases using the monoclonal antibody or antibody fragment of the present invention

The disease relating to Trop-2 can be diagnosed by detecting or determining Trop-2 or a Trop-2-expressing cell using the monoclonal antibody or antibody fragment of the present invention.

A diagnosis of cancer which is one of the diseases relating to Trop-2 can be carried out by, such as the following detection or measurement of Trop-2. The diagnosis can be carried out by detecting Trop-2 expressing on the cancer cell in a patient's body by an immunological method such as a flow cytometry.

An immunological method means a method in which an antibody amount or an antigen amount is detected or determined using a labeled antigen or antibody. Examples of the immunological method include radioimmunoassays, enzyme immunoassay, fluorescent immunoassay, luminescent immunoassay, Western blotting method, physico-chemical meathod, and the like.

Examples of the radioimmunoassays include a method, in which the antibody or the antibody fragment of the present invention is allowed to react with an antigen, an antigen-expressing cell or the like, then a radiolabeling anti-immunoglobulin antibody or a binding fragment thereof is allowed to react therewith, followed by determination using a scintillation counter or the like.

Examples of the enzyme immunoassays include a method, in which the antibody or antibody fragment of the present invention is allowed to react with an antigen, an antigen-expressing cell or the like, then a labeling anti-immunoglobulin antibody or an binding fragment thereof is allowed to react therewith and the chromogenic pigment is measured by a spectrophotometer, and, for example, sandwich ELISA may be used. As a label used in the enzyme immunoassay, any known enzyme label [Enzyme Immunoassay, published by Igaku Shoin, (1987)] can be used as described already. Examples include alkaline phosphatase labeling, peroxidase labeling, luciferase labeling, biotin labeling and the like.

Sandwich ELISA is a method in which an antibody is bound to a solid phase, a target antigen for detection or measurement is trapped and another antibody is allowed to react with the trapped antigen. In the ELISA, after two kinds of antibodies, which recognize the target antigen for detection or measurement and has difference in a recognizing site, or the antibody fragment thereof are prepared and the first antibody or antibody fragment is previously fixed on a plate (such as a 96-well plate) and the second antibody or antibody fragment is labeled with a fluorescent substance such as FITC, an enzyme such as peroxidase, a biotin or the like.

The plate to which the above antibody is fixed is allowed to react with the cell separated from living body or homogenate solution thereof, tissue or homogenate solution thereof, cultured cell supernatant, serum, pleural effusion, ascites, eye solution or the like, then allowed to react with a labeled monoclonal antibody or an antibody fragment and a detection reaction corresponding to the labeled substance is carried out. The antigen concentration in the sample to be tested can be calculated from a calibration curve prepared by a stepwise dilution of antigen of known concentration.

As antibodies used for sandwich ELISA, either polyclonal antibodies or monoclonal antibodies may be used. Also, antibody fragments such as Fab, Fab' and $F(ab)_2$ may be used. As a combination of two kinds of antibodies used in sandwich ELISA, a combination of monoclonal antibodies or antibody fragments recognizing different epitopes or a combination of a polyclonal antibody with a monoclonal antibody or a antibody fragment may be used.

Examples of fluorescent immunoassays include a method described in the literatures [Monoclonal Antibodies - Principles and practice, Third Edition, Academic Press (1996); Manual for Monoclonal Antibody Experiments, Kodansha Scientific (1987)] and the like. As a label for the fluorescent immunoassay, any of known fluorescent labels [Fluorescent Immunoassay, by Akira Kawao, Soft Science, (1983)] may be used as described already. Examples of the labels include FITC, RITC and the like.

The luminescent immunoassay can be carried out using the methods described in the literature [Bioluminescence and Chemical Luminescence, Rinsho Kensa, 42, Hirokawa Shoten (1998)] and the like. As a label for luminescent immunoassay, any of known luminescent labels can be included. Examples include acridinium ester, lophine or the like may be used.

Western blotting can be carried out using a method in which an antigen or an antigen-expressing cell is fractionated by SDS-polyacrylamide gel electrophoresis [Antibodies-A Laboratory Manual (Cold Spring Harbor Laboratory, 1988)], the gel is blotted onto PVDF membrane or nitrocellulose membrane, the membrane is allowed to react with an antibody or antibody fragment which can recognize the antigen, further allowed to react with an anti-mouse IgG antibody or antibody fragment which is labeled with a fluorescent substance such as FITC, an enzyme label such as peroxidase, a biotin labeling, and the label is visualized to confirm the reaction. An example thereof is described below.

Cells or tissues expressing a polypeptide having the amino acid sequence represented by SEQ ID NO: is expressed are dissolved in a solution and, 0.1 to 30 μg of protein per lane is electrophoresed under reducing conditions by an SDS-PAGE method. The electrophoresed protein is transferred to a PVDF membrane and allowed to react with PBS containing 1 to 10% of BSA (hereinafter referred to as "BSA-PBS") at room temperature for 30 minutes for blocking.

Here, the monoclonal antibody of the present invention is allowed to react therewith, washed with PBS containing 0.05 to 0.1% Tween 20 (hereinafter referred to as "Tween-PBS") and allowed to react with a peroxydase-labeled goat anti-mouse IgG at room temperature for 2 hours. After washing with Tween-PBS, a band bound by the monoclonal antibody is detected using ECL Western Blotting Detection Reagents (manufactured by Amersham) or the like to thereby detect a polypeptide having the amino acid sequence represented by SEQ ID NO:1. As an antibody used for the detection in Western blotting, an antibody which can be bound to a polypeptide having a native conformation is used.

The physicochemical method can be carried out, for example, by reacting Trop-2 as the antigen with the antibody or antibody fragment of the present invention to form an aggregate, and detecting this aggregate. Other examples of the physicochemical methods include a capillary method, a one-dimensional immunodiffusion method, an immunoturbidimetry, a latex immunoturbidimetry [Handbook of Clinical Test Methods, Kanehara Shuppan, (1998)] and the like.

For example, in a latex immunodiffusion method, a carrier such as polystyrene latex having a particle size of about of 0.1 to 1 μm sensitized with antibody or antigen may be used and when an antigen-antibody reaction is carried out using the corresponding antigen or antibody, scattered light in the reaction solution increases while transmitted light decreases. When such a change is detected as absorbance or integral sphere turbidity, it is now possible to measure antigen concentration, etc. in the sample to be tested.

In addition, for the detection of the Trop-2-expressing cell, known immunological detection methods can be used, and an immunoprecipitation method, an immuno cell staining method, an immune tissue staining method, a fluorescent antibody staining method and the like are preferably used.

An immunoprecipitation method can be carried out using a method in which a cell expressing Trop-2 is allowed to react with the monoclonal antibody or antibody fragment thereof of the present invention and then a carrier having specific binding ability to immunoglobulin such as protein G-Sepharose is added so that an antigen-antibody complex is precipitated. Also, the following method can be carried out. The above-described antibody or antibody fragment of the present invention is immobilized on a 96-well plate for ELISA and then blocked with BSA-PBS.

When the antibody is in a non-purified state such as a culture supernatant of hybridoma, anti-mouse immunoglobulin or anti-rat immunoglobulin or protein A or Protein G or the like is previously immobilized to a 96-well plate for ELISA and blocked with BSA-PBS and a culture supernatant of hybridoma cell is dispensed thereto for binding. After BSA-PBS is discarded and the residue is sufficiently washed with PBS, reaction is carried out with a dissolved solution of cells or tissues expressing Trop-2. An immune precipitate is extracted from the well-washed plate with a sample buffer for SDS-PAGE and detected by the above-described Western blotting.

An immune cell staining method or an immune tissue staining method are a method where antigen-expressing cells or tissues are treated, if necessary, with a surfactant, methanol or the like to increase permeability of an antibody to the cells or tissues, then the monoclonal antibody of the present invention is allowed to react therewith, then further allowed to react with an anti-immunoglobulin antibody or binding fragment thereof labeled by a fluorescent substrate such as FITC, an enzyme label such as peroxidase, a biotin or the like, and the label is visualized and observed under a microscope.

In addition, detection can be carried out by an immunofluorescent staining method [Monoclonal Antibodies - Principles and practice, Third Edition, Academic Press (1996), Manual for Experiments of Monoclonal Antibodies, Kodansha Scientific (1987)] in which cells are allowed to react with a fluorescence-labeled antibody and analyzed by a flow cytometer. Particularly, the monoclonal antibody or antibody fragment of the present invention which binds to an extracellular region of the Trop-2 can detect a cell expressing the polypeptide maintaining a native conformation.

In addition, in the case of using FMAT8100HTS system (manufactured by Applied Biosystems) and the like among fluorescent antibody staining methods, the antigen amount or antibody amount can be measured without separating the formed antibody-antigen complex and the free antibody or antigen which does not participate in the formation of the antibody-antigen complex.

The present invention can provide a monoclonal antibody or an antibody fragment thereof, which binds to the extracellular region of human Trop-2 with high affinity, and exhibits high antibody-dependent cellular cytotoxicity (hereinafter referred to as "ADCC activity") and high antitumor activity; a hybridoma which produces the antibody; a DNA which encodes the antibody; a vector which comprises the DNA; a transformant obtainable by introducing the vector; a process for producing an antibody or an antibody fragment thereof using the hybridoma or the transformant; and a therapeutic agent or a diagnostic agent using the antibody or the antibody fragment thereof.

EXAMPLE

Hereinafter, exemplary embodiments of the present invention will be described specifically. However, the present invention is not limited to the following examples

[Example 1]

Obtaining of an anti-Trop-2 mouse monoclonal antibody

(1) Preparation of antigen

The cancer cell line Pc1 was established from a human prostate cancer and used as an antigen.

(2) Immunization of animal and preparation of antibody-producing cells

Balb/c mice were immunized by administering with $1\times10^6$ Pc1 cells 5 times every two weeks and spleens were isolated on three days after the last administration. Spleens were cut out in an RPMI1640 (hereinafter referred as "RPMI medium") and crushed out and centrifuged (1,200 rpm, 5 minutes). Then, a Tris-ammonium chloride buffer (pH 7.6) was added to the obtained precipitate fraction and treated for 1 minute at 37°C to remove erythrocytes. After washing the precipitate fraction (the cell fraction) 3 times with the RPMI medium, the obtained cells was used for cell fusion.

(3) Preparation of mouse myeloma cells

An 8-azaguanine-resistant mouse myeloma cell line P3X63Ag8U.1(P3-U1) was cultured in RPMI1640 (manufactured by Invitrogen) supplemented with 10% fetal calf serum and used as a parental cell line for cell fusion.

(4) Preparation of adenovirus vector

Ax3CAZ3-FZ33 in which β-galactosidase gene was introduced in a modified adenovirus type 5 containing a Z33 motif of protein A which has a binding ability IgG in the HI loop was used for the introduction into hybridomas.

(5) Preparation of hybridoma

The mouse spleen cells obtained in Example 1(2) and the myeloma cells obtained in Example 1(3) were mixed at a ratio of 5:1 and centrifuged (1,200 rpm, 5 minutes). After thoroughly loosening a group of cells in the thus obtained precipitate fraction, 1 ml of polyethyleneglycol-4000 (PEG-4000) was added thereto over 1 minute under stirring, 1 ml of the RPMI medium was added to the cell solution several times every one minute, and then the total volume was adjusted to 50 ml by adding the RPMI medium. The cell suspension was centrifuged (900 rpm, 5 minutes), cells of the obtained precipitation fraction were mildly loosened, and then the cells were gently suspended in 100 ml of HAT medium [MI1640 medium supplemented with 10% fetal bovine serum and HAT Media Supplement]. The suspension was dispensed at 200μ l/well into a 96-well culture plate and cultured in a 5% $CO_2$ incubator at 37°C until the cells reached 50% confluence.

Meanwhile, the culture medium was removed from the Pc1 cells cultured in a 96-well culture plate, and the culture supernatant of the hybridoma was added thereto. After the cells were incubated at 4°C for 1 hour, the culture supernatant was removed and the cells were washed twice with PBS. Then, Ax3CAZ3-FZ33 prepared in Example 1(4) was added into the cells so as to give a multiplicity of infection (MOI) of 1,000. After the cells were incubated again at 4°C for 1 hour, the virus solution was removed and the cells were washed twice with PBS.

β-Galactosidase reporter assay was carried out using β-Gal Reporter Gene Assay, chemiluminescent kit (manufactured by Roche Diagnostics), and wells of which activity was high were selected. Cloning the cells of the selected wells by the limiting dilution method was repeated twice, and an antibody which reacts with Pc1 cell-producing a hybridoma which produced an antibody which reacts with Pc1 cell was isolated [The Prostate, 67, 1163 (2007)].

(6) Determination of target antigen

A target antigen of the hybridoma established in Example 1(5) was determined by a known method [The Prostate, 67, 1163 (2007)] and antibody KM4097 which recognized Trop-2 was found among the hybridomas. The isotype of the antibody was determined as IgG1 and κ by using Iso Strip mouse monoclonal antibody isotyping kit (manufactured by Roche Applied Science).

[Example 2]

Preparation of IgG1 type chimeric antibody and defucose type antibody of anti-Trop-2 mouse antibody AR47A6.4.2

(1) Construction of vectors for expression of AR47A6.4.2-derived human chimeric antibody

For the purpose of the comparison with the activity of an anti-Trop-2 monoclonal antibody of the present invention, a chimeric antibody (hereinafter referred as "cAR47A6.4.2") comprising the variable regions of known anti-Trop-2 mouse monoclonal antibody AR47A6.4.2 and the Fc region of human IgG1 was prepared.

Based on the sequence information disclosed in the specification of U.S. Patent No 7,420,040, plasmids in which the DNA sequence represented by SEQ ID NO:3 and the DNA sequence represented by SEQ ID NO:4 were inserted into a pZErO-2 vector as a gene encoding VH and a gene encoding VL of AR47A6.4.2, respectively, were made in custom synthesis (offered by IDT).

The plasmid comprising VH of AR47A6.4.2 was treated with restriction enzymes *Apa*I (manufactured by New England Biolabs) and *Not*I (manufactured by New England Biolabs) to obtain, the *Not*I-*Apa*I fragment comprising VH was obtained. In addition, the plasmid comprising VL of AR47A6.4.2 was obtained with restriction enzymes *BsiW*I (manufactured by New England Biolabs) and *EcoR*I (manufactured by New England Biolabs) to obtain, the *EcoR*I-*BsiW*I fragment.

Meanwhile, an antibody expression vector pKANTEX93 was treated with two kinds of combinations of restriction enzymes *Not*I and *Apa*I, or *EcoR*I and *BsiW*I. Two kinds of combinations of the *Not*I-*Apa*I fragment comprising VH and the *Not*I-*Apa*I fragment derived from pKANTEX93, and the *EcoR*I-*BsiW*I fragment comprising VL and the *EcoR*I-*BsiW*I fragment derived from pKANTEX93 were ligated using Ligation high (manufactured by Toyobo Co., Ltd.).

*Escherichia coli* DH5α (manufactured by Toyobo Co., Ltd.) was transformed using the obtained ligation reaction solution. Plasmid DNAs were prepared from the transformed clone, and pKANTEX93 vectors in which a cDNA encoding VH or VL of AR47A6.4.2 was cloned were obtained.

After treating each pKANTEX93 vector comprising VH or VL of AR47A6.4.2 with restriction enzymes *EcoR*I and *Not*I, the *EcoR*I-*Not*I fragment comprising VL and the *Not*I- *EcoR*I fragment comprising VH were obtained. The two kinds of obtained fragments were ligated using Ligation high (manufactured by Toyobo Co., Ltd.).

*Escherichia coli* DH5α (manufactured by Toyobo Co., Ltd.) was transformed using the thus obtained ligation reaction solution. A plasmid DNA was prepared from the transformed clone, and an anti-Trop-2 human chimeric antibody expression vector comprising cDNA encoding VH and VL of AR47A6.4.2 was obtained.

(2) Expression of AR47A6.4.2-derived human chimeric antibody using animal cells

The antibody expression vector obtained in Example 2(1) was introduced into an animal cell line by a conventional method [Antibody Engineering, A Practical Guide, W. H. Freeman and Company (1992)] to obtain a transformant which produces anti-Trop-2 human chimeric antibody. For a host animal cell line, a CHO/DG44 cell line and a CHO/DG44 cell in which α1,6 fucosyltransferase (FUT8) gene is knocked out (hereinafter referred as "FUT8 knockout CHO/DG44 cell) were used.

(3) Obtaining of AR47A6.4.2 purified chimeric antibody

The cell suspensions of each transformant obtained in Example 2(2) were centrifuged for 20 minutes under conditions of 3,000 rpm at 4°C to recover the culture supernatants, and then the culture supernatants were filtration-sterilized using Millex GV filter having a pore size of 0.22 μm (manufactured by Millipore). Each anti-Trop-2 human chimeric antibody was purified from the thus obtained culture supernatant using Protein A High-capacity Resin (manufactured by Millipore). The antibody obtained from the transformant derived from a CHO/DG44 cell and the antibody obtained from the transformant derived from FUT8 knockout CHO/DG44 cell were called as cAR47A6.4.2 and cAR47A6.4.2-P, respectively.

(4) Determination of fucose content in AR47A6.4.2 chimeric antibody

In accordance with the method described in WO2002/31140, a ratio of a sugar chain in which fucose was not bound to the complex-type N-linked sugar chains present in cAR47A6.4.2 and cAR47A6.4.2-P was examined. The results are shown in Table 1.

[Table 1]
Fucose content of anti-Trop-2 antibody

| | |
|---|---|
| cAR47A6.4.2 | 100 % |
| cAR47A6.4.2-P | 0 % |

As the results shown in Table 1, it was confirmed that fucose was not added to cAR47A6.4.2-P.

[Example 3]

Evaluation of antigen binding activity of anti-Trop-2 mouse monoclonal antibody KM4097

(1) Reactivity of KM4097 to human Trop-2-positive cell line by flow cytometry (hereinafter referred as "FCM")

In PBS containing 1% of BSA (hereinafter referred as "BSA-PBS"), each of human pancreatic carcinoma cell line BxPC-3, human breast cancer cell line MCF-7, ovarian cancer cell line CaOV-3 and colon cancer cell line Colo205 were suspended at a density of 1 to $2 \times 10^5$ cells for blocking.

Then, anti-Trop-2 monoclonal antibody KM4097 was diluted appropriately with BSA-PBS and added to the cells to give a total volume of 100 $\mu$l. After the cell suspension was allowed to incubate on ice for 60 minutes, the cells were washed with BSA-PBS. Then, 100 $\mu$l of a FITC-labeled goat anti-mouse IgG antibody (manufactured by Dako Japan Co., Ltd.) which was diluted with BSA-PBS was added to the cells to react on ice for 30 minutes. After washing the cells with BSA-PBS, the cells were suspended in PBS. The fluorescence intensity was measured by FCM (manufactured by Beckman Coulter Inc.).

Histograms and the mean fluorescence intensity (MFI value) in cases where 10 $\mu$g/ml of KM4097 was allowed to react are shown in Fig. 1(a) to Fig. 1(d) and Table 2.

[Table 2]
MFI value of anti-Trop-2 monoclonal antibody KM4097

| | |
|---|---|
| BxPC-3 | 124 |
| CaOV-3 | 146 |
| MCF-7 | 92.8 |
| Colo205 | 54.8 |

Based on the results shown in Fig. 1(a) to Fig. 1(d) and Table 2, it was found that KM4097 could bind to any of the Trop-2-positive cell lines in an antibody concentration-dependent manner.

(2) Binding activity of KM4097 to recombinant human Trop-2 using Biacore

In order to analyze the binding activity of each anti-Trop-2 mouse monoclonal antibody to recombinant human Trop-2 in view of the reaction kinetics, the binding activity was measured by a surface plasmon resonance method (SPR method). In addition, the binding activity of 77220 (manufactured by R&D) and MOv16 (manufactured by Alexis Biomedical) which was known anti-Trop-2 monoclonal antibodies were also analyzed as a control.

All the operations described below were carried out using Biacore T100 (manufactured by GE Healthcare Bio-sciences). Tetra-His antibody (manufactured by Qiagen) was immobilized so as to be 12,000 RU (resonance unit) on the CM5 sensor 77 chip (manufactured by GE Healthcare Bio-sciences) using Amine Coupling Kit (manufactured by GE Healthcare Bio-sciences). After recombinant Trop-2/Fc/His fusion proteins (manufactured by R&D) were allowed to be captured on the sensor chip, anti-Trop-2 antibodies which were diluted in five-fold steps, starting from a concentration of 5,000ng/ml were flowed at 30µL/min, followed by obtaining sensorgrams at each concentration.

Using the software for analysis accompanied with the equipment, the results were analyzed based on Bivalent Analyte model to obtain the binding rate constant and the dissociation rate constant for binding of each antibody to a recombinant human Trop-2/Fc/His fusion protein.

The binding rate constants (hereinafter referred as "ka"), the dissociation rate constants (hereinafter referred as "kd"), and the dissociation constants $K_D$ (kd/ka) of each antibody were shown in Table 3 (the mean value of duplicate experiments).

[Table 3]

|  | kd (1/Ms) | ka (1/s) | $K_D$ (nM) |
|---|---|---|---|
| KM4097 | $3.89 \times 10^5$ | $1.01 \times 10^{-4}$ | 0.261 |
| 77220 | $5.55 \times 10^5$ | $6.10 \times 10^{-4}$ | 1.10 |
| MOv16 | $1.83 \times 10^5$ | $1.84 \times 10^{-4}$ | 0.929 |

As shown in Table 3, KM4097 exhibited higher affinity than conventional antibodies.

(3) Evaluation of competition binding activities between anti-Trop-2 monoclonal antibodies using FCM

In BSA-PBS, human pancreatic carcinoma cell line BxPC-3 was suspended at a density of 1 to $2 \times 10^5$ cells for blocking. Meanwhile, the concentration of cAR47A6.4.2 was made to be 0.3 µg/ml with BSA-PBS and KM4097 or 77220 was serially diluted with BSA-PBS. Each prepared antibody solution was added into the cell suspension to give a total volume of 100 µl.

After the cell suspension was allowed to react on ice for 60 minutes, the cells were washed with BSA-PBS. Then, 100 µl of a FITC-labeled goat anti-mouse IgG antibody (manufactured by Jackson Immuno research Laboratories) which was diluted with BSA-PBS was added to the cells to allow to react on ice for 30 minutes. After washing the cells with BSA-PBS, the cells were suspended in PBS. The fluorescence intensity was measured by FCM (manufactured by Beckman Coulter Inc.).

Fig.2 shows the mean fluorescence intensity (MFI value) of the binding activity of cAR47A6.4.2 to BxPC-3 cells when the concentration of KM4097 or 77220 was changed. It was found that, while 77220 inhibited the binding of cAR47A6.4.2 to cells in a concentration-dependent manner, KM4097 did not inhibit the binding of cAR47A6.4.2 to BxPC-3 cells.

Subsequently, BxPC-3 cells were blocked with BSA-PBS in the same manner as described the above. Meanwhile, the concentration of KM4097 or 77220 was made to be 1 µg/ml with BSA-PBS and cAR47A6.4.2 was serially diluted with BSA-PBS. The prepared antibody solution was added into the cell suspension and then the total volume was adjusted to 100 µl.

After the cell suspension was allowed to react on ice for 60 minutes and the cells were washed with BSA-PBS. Then, 100 µl of a FITC-labeled goat anti-mouse IgG antibody (manufactured by Dako Japan Co., Ltd.) which was diluted with BSA-PBS was added to the cells to carry out the reaction on ice for 30 minutes. After washing the cells with BSA-PBS, the cells were suspended in PBS and then the fluorescence intensity was measured by FCM (manufactured by Beckman Coulter Inc.).

Fig. 3 shows that the mean fluorescence intensity (MFI values) of the binding ability of KM4097 or 77220 to BxPC-3 cells when the concentration of cAR47A6.4.2 was changed. As shown in Fig. 3, cAR47A6.4.2 was found not to inhibit the binding of KM4097 to BxPC-3 cells.

Based on the above results, it was shown that KM4097 recognizes an epitope different from cAR47A6.4.2.

(4) Evaluation of competitive activities between anti-Trop-2 monoclonal antibodies using binding ELISA of Trop-2

Into a 96-well ELISA plate (manufactured by Greiner), 2 µg/ml of a recombinant human Trop-2/Fc/His fusion protein (manufactured by R&D) was dispensed at 50 µl/well, and allowed to stand at 4°C overnight for immobilization. After washing the plate with PBS, BSA-PBS was added thereto at 100 µl/well and allowed to react at room temperature for 1 hour for blocking.

After removing BSA-PBS from the plate, BSA-PBS dilution solution of 0.3 µg/ml of KM4097 (mouse IgG1 subclass) as the primary antibody and serial dilution solutions of a competing antibody (subclass of the antibody is other than mouse IgG1) with BSA-PBS were dispensed to give a total volume of 50 µl/well and allowed to stand for 2 hours.

After washing the plate with PBS containing 0.05% polyoxyethylene (20) sorbitan monolaurate manufactured by Wako Pure Chemical Industries, Ltd.; equivalent to ICI Inc.'s trademark Tween 20)(hereinafter referred as "Tween-PBS"), a peroxidase-labeled rabbit anti-mouse IgG1 antibody (manufactured by Zymed) as the second antibody was added at 50 μl/well and allowed to stand at room temperature for 1 hour.

After washing the plate with Tween-PBS, the ABTS [2,2-Azino-bis(3-ethyl benzothiazol-6-sulfonic acid)ammonium)] substrate solution [1 mmol/l ABTS, 0.1 mol/l citric acid buffer (pH 4.2), 0.1% $H_2O_2$] was added at 50 μl/well for color development, and the chromogenic reaction was stopped by adding a 5% SDS (sodium lauryl sulfate) solution at a volume of 50 μl/well. Thereafter, the value of the absorbance of OD415 nm was measured by a plate reader Emax (manufactured by Molecular Devices, LLC.).

Fig. 4 shows the binding of KM4097 to a Trop-2/Fc/His fusion protein when the concentration of 77220 was changed. As shown in Fig. 4, it was found that 77220 did not inhibit the binding of KM4097 to a Trop-2/Fc/His fusion protein.

Next, in the same manner as described in the experiment before, a recombinant human Trop-2/Fc/His fusion protein was immobilized onto a 96-well ELISA plate, washed with PBS, and blocked with BSA-PBS. In the plate, BSA-PBS dilution solution of 0.3 μg/ml of 77220 (mouse IgG2a subclass) as the primary antibody and BSA-PBS serial dilution solutions of a competing antibody (subclass of the antibody is other than mouse IgG2a) were dispensed to give a total volume of 50 μl/well and allowed to stand for 2 hours.

After washing the plate with Tween-PBS, a peroxidase-labeled rabbit anti-mouse IgG2a antibody (manufactured by Zymed) as the second antibody was added at 50 μl/well and allowed to stand at room temperature for 1 hour. After washing the plate with Tween-PBS, the ABTS substrate solution was added at 50 μl/well for color development, and the chromogenic reaction was stopped by adding a 5% SDS solution at 50 μl/well. Then, the value of the absorbance of OD415 nm was measured using a plate reader Emax (manufactured by Molecular Devices, LLC.).

Fig. 5 shows the binding of 77220 to a Trop-2/Fc/His chimeric protein when the concentrations of KM4097 and MOv16 were changed. As shown in Fig. 5, it was found that MOv16 inhibited the binding of 77220 to a Trop-2/Fc/His chimeric protein, but KM4097 did not inhibit the binding of 77220 to a Trop-2/Fc/His chimeric protein.

Based on the above results, it was found that KM4097 recognized the epitope which was different from those of 77220 and MOv16.

[Example 4]

Isolation of cDNA encoding the variable regions of anti-Trop-2 mouse monoclonal antibody KM4097

(1) Preparation of mRNA from KM4097 producing hybridoma cells

From $5\times10^7$ to $1\times10^8$ cells of the hybridoma cells obtained in Example 1, mRNA was prepared using RNAeasy Maxi Kit (manufactured by Qiagen) and Oligotex™-dT30<Super>mRNA Purification Kit (manufactured by Takara Bio Inc.).

(2) Gene cloning of H chain variable regions and L chain variable regions of KM4097

Using SMART RACE cDNA Amplification Kit (manufactured by Clontech), a cDNA was obtained from the mRNA of KM4097 obtained in Example 4(1). A cDNA fragment of VH of each antibody was amplified by carrying out PCR using the thus obtained cDNA as the template and using a universal primer Amix (attached to the kit) and the mouse IgG1-specific primers (SEQ ID NOs:5 and 6). AcDNA fragment of VL of each antibody was amplified by carrying out PCR using the mouse Ig(κ)-specific primers (SEQ ID NOs:7 and 8) instead of primers specific to subclass of each antibody. In the every PCRs, using PTC-200 DNA Engine (manufactured by Bio-Rad Laboratories), after heating at 94°C for 2 minutes, reaction was carried out by 30 cycles, each cycle consisting of reaction at 94°C for 15 seconds and reaction at 68°C for 1 minutes.

The thus obtained PCR products were separated by agarose gel electrophoresis, and a DNA fragment comprising VH and a DNA fragment comprising VL were obtained. Subsequently, after deoxyadenines were attached to both ends of the obtained each DNA fragment using Target Clone Plus (manufactured by Toyobo Co., Ltd.), each of the DNA fragments was ligated to a pTA2 vector. Each of the obtained vectors was introduced into *Escherichia coli* DH5α, and each of the plasmids was extracted from the obtained transformed clone. The nucleotide sequence of each PCR product obtained by cloning was analyzed by BigDye Terminator Cycle sequencing FS Ready Reaction Kit (manufactured by PE Biosystems) and a sequencer manufactured by the same company, ABI PRISM 3700. As a result, it was determined that a plasmid comprising complete length cDNA of VH and a plasmid comprising complete length cDNA of VL in which an ATG sequence considered to be the initiation codon was present in the 5' terminal of the cDNA, were obtained.

(3) Analysis of V region amino acid sequences of KM4097

Complete nucleotide sequence of VH of KM4097 contained in the plasmid obtained in Example 4(2) was shown as SEQ ID NO:9, complete amino acid sequence of VH including a signal sequence deduced from the nucleotide sequence was shown as SEQ ID NO:10, the complete nucleotide sequence of VL contained in the plasmid is represented by SEQ ID NO:11, and the complete amino acid sequence of VL including a signal sequence deduced from the nucleotide sequence was shown as SEQ ID NO:12.

Based on the comparison with the known sequence data of mouse antibodies [SEQUENCES of Proteins of Immunological Interest, US Dept. Health and Human Services (1991)], it was found that the isolated each cDNA was a complete length cDNA encoding the variable region (hereinafter referred as "V region") including a secretion signal sequence of KM4097, and the sequence at positions 1 to 19 of the amino acid sequence represented by SEQ ID NO: 10 is the secretion signal sequence of the H chain of KM4097, and the sequence at positions 1 to 20 of the amino acid sequence represented by SEQ ID NO:12 is the secretion signal sequence of the L chain of KM4097.

Next, the novelty of the amino acid sequences of the VH and the VL of KM4097 was examined. Using GCG Package (version 9.1, Genetics Computer Group) as the sequence analyzing system, amino acid sequence data base of the known proteins was retrieved by the BLASTP method [Nucleic Acids Res., 25, 3389 (1997)]. As a result, since completely identical amino acid sequences were not found for both of the VH and VL in the data base, it was found that the VH and the VL of KM4097 have novel amino acid sequences.

CDRs of the VH and VL of KM4097 were identified by comparing with known amino acid sequences of antibodies. The amino acid sequences of CDR1, CDR2 and CDR3 of the VH of KM4097 were represented by SEQ ID NOs:13, 14 and 15, respectively, and the amino acid sequences of the CDR1, CDR2 and CDR3 of the VL were represented by SEQ ID NOs:16, 17 and 18, respectively.

[Example 5]

Preparation of human chimeric antibody KM4097

(1) Construction of vector for expression of human chimeric antibody KM4097

A chimeric antibody prepared in the present invention is a chimeric antibody in which the H chain constant region of Fc region of human IgG1 and human κ type L chain constant region were ligated to the variable region of the H chain and the variable region of the L chain of KM4097 obtained in Example 4(2), respectively. A vector for expression of a human chimeric antibody KM4097 was constructed in the following manner using the vector for expression of antibody pKANTEX93 and each pTA2 vector comprising VH or VL obtained in Example 4(2).

PCR was carried out by preparing 15 μl of a reaction solution containing 100 ng of the pTA2 vector having the VH or VL of KM4097 as the template, 1.5 μl of 10×ExTaq buffer, 1.2 μl of 2.5 mmol/l dNTP, 1 μl of ExTaq DNA polymerase (manufactured by Takara Bio Inc.), and 0.75 μl of 10 μmol/l of each of the primers which are specific for the VH or VL. The primers of the VH of KM4097 are represented by SEQ ID NOs:19 and 20 and the primers of the VL of KM4097 are represented by SEQ ID NOs:21 and 22. The PCR was carried out by, after heat treatment at 94°C for 2 minutes, repeating 30 cycles, each cycle consisting of reaction at 94°C for 15 seconds, reaction at 55°C for 30 seconds, and reaction 72°C for 1 minute, followed by reaction at 72°C for 10 minutes.

Respective PCR reaction products were separated by agarose gel electrophoresis, and the DNA fragment comprising VH and the DNA fragment comprising VL were obtained. Next, using TOPO TA cloning kit for sequencing (manufactured by Invitrogen), the each DNA fragment was inserted into a pCR4-TOPO vector.

After treating the plasmid comprising the VH of KM4097 with restriction enzymes *Apa*I (manufactured by New England Biolabs) and *Not*I (manufactured by New England Biolabs), the *Not*I-*Apa*I fragment comprising the VH was obtained. In addition, after treating the plasmid comprising the VL of KM4097 with restriction enzymes *Bsi*WI (manufactured by New England Biolabs) and *Eco*RI (manufactured by New England Biolabs), the *Eco*RI-*Bsi*WI fragment was obtained.

Meanwhile, the antibody expression vector pKANTEX93 was treated with two kinds of combinations of restriction enzymes *Not*I and *Apa*I, or *Eco*RI and *Bsi*WI. Two kinds of combinations of the *Not*I-*Apa*I fragment comprising the VH and the *Not*I-*Apa*I fragment derived from pKANTEX93, and the *Eco*RI-*Bsi*WI fragment comprising the VL and the *Eco*RI-*Bsi*WI fragment derived from pKANTEX93 were ligated using Ligation high (manufactured by Toyobo Co., Ltd.).

*Escherichia coli* DH5α (manufactured by Toyobo Co., Ltd.) was transformed using the thus obtained ligation reaction solution. Plasmid DNAs were prepared from the transformed clone, and pKANTEX93 vectors were obtained in which a cDNA encoding the VH or a cDNA encoding the VL of KM4097 were cloned, respectively.

After treating each pKANTEX93 vector comprising the VH or VL of KM4097 with restriction enzymes *Eco*RI and *Not*I, the *Eco*RI-*Not*I fragment comprising the VL and the *Not*I- *Eco*RI fragment comprising the VH were obtained.

The two kinds of fragments obtained were ligated using Ligation high (manufactured by Toyobo Co., Ltd.). *Escherichia coli* DH5α (manufactured by Toyobo Co., Ltd.) was transformed using the thus obtained ligation reaction solution. A plasmid DNA was prepared from the transformed clone, and anti-Trop-2 human chimeric antibody expression vectors which had a cDNA encoding the VH and a cDNA encoding the VL of KM4097 were obtained.

(2) Obtaining KM4097 human chimeric antibody using animal cells

In the same manner described in Example 2, the vector for expression of anti-Trop-2 chimeric antibody obtained in Example 5(1) was introduced into a CHO/DG44 cell line and FUT8 knockout CHO/DG44 cell line, and from the obtained each transformant, anti-Trop-2 human chimeric antibodies KM4590 and KM4591 were obtained.

(3) Determination of fucose content in KM4097 chimeric antibodies

In accordance with the method described in WO2002/31140, a ratio of a sugar chain in which fucose was not bound to the complex-type N-linked sugar chains present in KM4590 and KM4591 was examined. The results are shown in Table 4.

[Table 4]
Fucose content of anti-Trop-2 antibody

| | |
|---|---|
| KM4590 | 100 % |
| KM4591 | 0 % |

As the results shown in Table 4, it was demonstrated that fucose was not added to human chimeric antibody KM4591 prepared in Example 5(2).

[Example 6]

Activity evaluation of anti-Trop-2 human chimeric antibodies KM4590 and KM4591

Activity of KM4590 and KM4591 obtained in Example 5 and cAR47A6.4.2 and cAR47A6.4.2-P obtained in Example 2 were evaluated.

(1) Binding activity of anti-Trop-2 human chimeric antibody to recombinant human Trop-2 using Biacore

In the same manner described in Example 3, the affinity of KM4591 and cAR47A6.4.2-P to human Trop-2/Fc/His fusion proteins (manufactured by R&D) were measured. ka, kd, and $K_D$ (kd/ka) of each antibody are shown in Table 5 (the mean values of duplicate experiments).

[Table 5]

| | kd (1/Ms) | ka (1/s) | $K_D$ (nM) |
|---|---|---|---|
| KM4591 | $4.16 \times 10^5$ | $5.80 \times 10^{-5}$ | 0.143 |
| cAR47A6.4.2-P | $2.21 \times 10^5$ | $2.18 \times 10^{-4}$ | 1.01 |

As shown in Table 5, KM4591 exhibited higher affinity than cAR47A6.4.2-P.

(2) Evaluation of ADCC activity of anti-Trop-2 human chimeric antibodies toward human cancer cell lines

ADCC activity of KM4590 and cAR47A6.4.2-1 on human cancer cell lines was measured in the following manner.

(2)-1 Preparation of target cell suspension

After washing with PBS, human cancer cell lines MCF-7 and Colo205 were washed using phenol red-free RPMI1640 medium (manufactured by Invitrogen) containing 5% fetal bovine serum (FBS, manufactured by Invitrogen) (hereinafter referred as "ADCC activity measuring medium"), and then the cell density was adjusted appropriately and used as the target cell suspension.

(2)-2 Preparation of effector cell solution

According to the following manner, peripheral blood mononuclear cells (PMBC) were separated from peripheral blood of healthy subjects. Using a syringe containing 0.5 ml of heparin sodium (manufactured by Ajinomoto), 50 ml of healthy normal peripheral blood was collected from a healthy normal person. The equal volume of saline (manufactured by Otsuka Pharmaceutical Co., Ltd.) was added to the collected peripheral blood and the blood was diluted and thoroughly mixed.

In 15 ml tubes, 10 ml of diluted peripheral blood was carefully overlayered onto 4.5 ml of Lymphoprep (manufactured by Axis-Shield) dispensed and centrifuged at 2,000 rpm for 20 minutes at room temperature with no brake. Then, a PBMC fraction was separated. The thus separated PBMC fraction was washed twice with the ADCC activity measuring medium and then the cell density was adjusted appropriately with the same medium and used as the effector cell suspension.

(2)-3 Measurement of ADCC activity

Into each well of a 96-well U bottom plate (manufactured by Falcon), the antibody solution in which each antibody was serially ten-fold diluted starting from 3 $\mu$g/ml, was dispensed at 50 $\mu$l. Next, the target cell solution prepared in the above-mentioned (2)-1 was dispensed at $1 \times 10^4$ cells/50 $\mu$l/well and finally, the effector cell solution prepared in the above-mentioned (2)-2 was dispensed at $2.5 \times 10^5$ cells/50 $\mu$l/well into the 96-well U bottom plate. After adjusting the total volume to 150 $\mu$l, allowed to react at 37°C for 4 hours. The ratio of the effector cell (E) and target cell (T) was set to be 25:1 in the present system. After the reaction, each color development in the supernatant was measured using LDH Cytotoxic Test (manufactured by Wako Pure Chemical Industries, Ltd.).

The ADCC activity was calculated by the following formula. Those results are shown in Fig. 6A and Fig. 6B, Fig. 7A and Fig. 7B, Fig. 8A and Fig. 8B, and Fig. 9A and Fig. 9B.

(Formula)

$$\text{ADCC activity (\%)} = \{([\text{absorbance of sample}]-[\text{absorbance of target cell spontaneous release}]-[\text{absorbance of effector cell spontaneous release}])/([\text{absorbance of target cell total release}]-[\text{absorbance of target cell spontaneous release}])\} \times 100$$

As shown in Fig. 6A and Fig. 6B and Fig. 7A and Fig. 7B, KM4590 shows the ADCC activity for human cancer cell lines MCF-7 and Colo205, and the activity was higher than that of a known anti-Trop-2 human chimeric antibody cAR47A6.4.2. As shown in Fig. 8A and Fig. 8B, and Fig. 9A and Fig. 9B, KM4591 had higher ADCC activity on human cancer cell lines MCF-7 and Colo205 than KM4590.

(3) Evaluation of antitumor activity of human chimeric antibody anti-Trop-2 on xenograft mouse of BxPC-3 cell line

BxPC-3 cells were prepared with PBS to give a density of $5 \times 10^7$ cells/ml and injected under the right armpit of a 6 week-old male SCID mouse (manufactured by CLEA Japan) at a dose of 100 $\mu$l Six days after injection, tumor diameter was measured and animals were divided into groups so as to be the average tumor volume [tumor volume was calculated by length $\times$(breadth)$^2$ $\times$0.5] of each group of 100 mm$^3$ (six animals per group). PBS or anti-Trop-2 chimeric antibodies (KM4591 or cAR47A6.4.2-P) was administered twice a week for 4 weeks, and the antitumor activity was evaluated.

The results are shown in Fig. 10. As shown in Fig. 10, KM4591 showed significant antitumor activity and the activity was higher than that of cAR47A6.4.2-P.

(4) Evaluation of antitumor activity on anti-Trop-2 chimeric antibody on xenograft mouse of Colo205 cell line

Colo205 cells were prepared with PBS to give a density of $5 \times 10^7$ cells/ml and injected under the right armpit of a 5 week-old male SCID mouse (manufactured by CLEA Japan) at a dose of 100 $\mu$l. Five days after injection, tumor diameter was measured and animals were divided into groups with average tumor volume [tumor volume was calculated by length $\times$(breadth)$^2$ $\times$0.5] of each group of 100 mm$^3$ (five animals per group). PBS or anti-Trop-2 chimeric antibodies (KM4591 or cAR47A6.4.2-P) was administered twice a week for 4 weeks, and the antitumor activity was evaluated.

The results are shown in Fig. 11. As shown in Fig. 11, KM4591 showed significant antitumor activity and the activity was higher than that of cAR47A6.4.2-P.

[Example 7]

Epitope analysis of anti-Trop-2 human chimeric antibody KM4590

(1) Evaluation of competitive activities for various anti-Trop-2 monoclonal antibodies using binding ELISA of Trop-2

Into a 96-well ELISA plate (manufactured by Greiner), 2 $\mu$g/ml of a recombinant human Trop-2/Fc/His chimeric protein (manufactured by R&D) was dispensed at 50 $\mu$l/well, and allowed to stand at 4°C overnight for immobilization. After washing the plate with PBS, BSA-PBS was added thereto at 100 $\mu$l/well and allowed to stand at room temperature for 1 hour for blocking the active groups.

Then, BSA-PBS was removed from the plate, BSA-PBS dilution solution of 0.3 $\mu$g/ml of various mouse anti-Trop-2 antibodies [77220 (manufactured by R&D), MOv16 (manufactured by ALEXIS BIOCHEMICALS), MM0588-49D6 (manufactured by Angio-Proteomie), YY-01 (manufactured by Santa Cruz) or 162-46 (manufactured by BD Pharmigen)] as the primary antibody and serial dilution solutions of a competing chimeric antibody (KM4590 or cAR47A6.4.2) were dispensed at 50 $\mu$l/well and allowed to stand for 2 hours.

After washing the plate with Tween-PBS, a peroxidase-labeled rabbit anti-human IgG antibody (manufactured by American Qualex) was added at 50 $\mu$l/well as the second antibody and allowed to stand at room temperature for 1 hour. After washing the plate with Tween-PBS, the ABTS [2,2-Azino-bis(3-Ethyl benzothiazol-6-Sulfonic Acid)ammonium)] substrate solution [1 mmol/l ABTS, 0.1mol/l citric acid buffer (pH 4.2), 0.1% $H_2O_2$] was added at 50 $\mu$l/well for color development, and the chromogenic reaction was stopped by adding a 5% SDS (Sodium lauryl sulfate) solution at 50 $\mu$l/well. Thereafter, the value of the absorbance of OD415 nm was measured by a plate reader Emax (manufactured by Molecular Devices, LLC.).

The bindings of various anti-Trop-2 mouse monoclonal antibodies to Trop-2/Fc/His chimeric proteins when the concentrations of KM4590 and cAR47A6.4.2 were changed were shown in Fig. 14(a) to Fig. 14(f).

As shown in Fig. 14(a) to Fig. 14(f), it was found that the binding of anti-Trop-2 mouse monoclonal antibodies other than KM4097 to Trop-2/Fc/His chimeric proteins were not inhibited by KM4590. Meanwhile, it was found that the bindings of anti-Trop-2 mouse monoclonal antibodies other than KM4097 to Trop-2/Fc/His chimeric proteins were inhibited by cAR47A6.4.2.

Based on the above results, it was found that KM4097 and its chimeric antibody, KM4590, were recognized epitopes which were different from those of other anti-Trop-2 antibodies.

(2) Production of Trop-2 extracellular region protein and the domain deletion mutant protein

An extracellular region of Trop-2 and a domain deletion mutant were produced in order to determine an epitope of an anti-Trop-2 monoclonal antibody of the present invention [Fig. 15]. First, a vector (INPEP4-FLAG-Fc) wherein the DNA (SEQ ID NO::39) in which FLAG tag-encoding DNA and Fc region-encoding DNA were arranged in order from 5' terminal to 3' terminal was inserted into a INPEP4 vector (manufactured by Biogen-IDEC) was produced.

Next, by inserting the DNA (SEQ ID NO:40) encoding a protein in which the secretion signal sequence was added to the Trop-2 extracellular region to the 5' terminal of FLAG tag sequence of INPEP4-FLAG-Fc vector, a vector for expressing a fusion protein of Trop-2 extracellular region, FLAG tag and Fc (referred as "Trop-2/FLAG/Fc") was constructed.

In the same manner, using DNAs (SEQ ID NOs:41, 42, and 43) encoding three types of proteins (referred to as Mutant A, Mutant B, and Mutant C, respectively) which lacked only domain I; domain I and domain II; and full length of domain I and domain II and part of domain III, respectively in the extracellular region of Trop-2, respectively, expression vectors of fusion proteins of the extracellular region of Trop-2, FLAG tag and Fc were constructed. The amino acid sequences of Trop-2/FLAG/FC, Mutant A, Mutant B or Mutant C are represented by SEQ ID NOs:44 to 47, respectively.

Gene introduction and protein expression were carried out using HEK293F as a host cell and using FreeStyle™ 293 Expression system (manufactured by Invitrogen). First, 30 $\mu$g of the expression vector plasmid was mixed with OPTI-MEM I (manufactured by Invitrogen) to give a total volume of 900 $\mu$l. Next, 30 $\mu$l of 293 fectin™ (manufactured by Invitrogen) was mixed with OPTI-MEM I to give a total volume of 900 $\mu$l and then allow to stand at room temperature for 5 minutes.

The 293 fectin™ solution was mixed with the expression vector plasmid solution and allowed to stand at room temperature for 20 to 30 minutes. After all the mixed plasmid solution was added to 30 ml of HEK293F at a cell density of $1.1 \times 10^6$cells/ml which was cultured with FreeStyle™ 293 SFM (manufactured by Invitrogen), the cells were cultured in suspension at 37°C, 5% $CO_2$, on an orbital shaker at 125 rpm for four days.

After culture, the cell suspension was centrifuged for 20 minutes under conditions of 3,000 rpm at 4°C to recover the culture supernatant, and then the culture supernatant was filtration-sterilized using Millex GV filter having a pore size of 0.22 μm.

Econo-Pac column was packed with 1 ml of MabSelect (manufactured by GE Healthcare) and the thus recovered culture supernatant was loaded onto the column, and the column was washed with 10 ml of PBS. After washing, the antibody adsorbed to a carrier was eluted using 0.1mol/l citric acid buffer (pH 3.4).

The eluted fraction was neutralized with 1mol/l Tris buffer (pH 8.5). Next, the fractions in which the elution of target proteins was confirmed by SDS-PAGE analysis were gathered, and the thus gathered fraction was concentrated with Amicon Ultra-10K (manufactured by Millipore) and filtration-sterilized using Millex GV filter having a pore size of 0.22 μm. The absorbance at 280 nm (OD 280 nm) of the concentrated solution was measured using an absorption spectrophotometer (Nanodrop 2000 manufactured by ThermoScientific) and the concentration of each purified protein was calculated.

(3) Epitope analysis by Western blotting

Each protein of Trop-2/FLAG/Fc, Mutant A, Mutant B, or Mutant C prepared in this Example 2 was electrophoresed under reducing condition (treatment of samples in the presence of 10 mmol/l DTT at 95°C for 5 minutes) or non-reducing condition (treatment of samples in the absence of DTT at room temperature for 10 minutes) by an SDS-PAGE method. The proteins in the gel were transferred to polyvinylidene difluoride (PVDF) membrane at 200 mA for 1 hour using a semi-dry method.

After the blocking of the PVDF membrane was carried out using PBS containing 5% BSA at room temperature for 1 hour, the PVDF membrane was soaked in the primary antibody solution which was prepared with TBST [an aqueous solution which was prepared by diluting Tris buffered saline (10× concentration) (pH 7.4) manufactured by Nakarai Tesque Inc.) ten-fold and further adding 0.1% concentration of 0.05% polyoxyethylene (20) sorbitan monolaurate (manufactured by Wako Pure Chemical Industries, Ltd., equivalent to ICI Inc.'s trademark Tween 20)] comprising 0.1% BSA to react for 1 hour at room temperature or overnight at 4°C.

After washing the PVDF membrane 3 to 4 times with TBST, a peroxidase-labeled goat anti-mouse IgG antibody (manufactured by GE Healthcare) prepared with TBST containing 0.1% BSA or a peroxidase-labeled goat anti-human κ chain antibody (manufactured by Southern Biotech) as a secondary antibody was allowed to react therewith at room temperature for 1 hour. After washing the PVDF membrane 3 to 4 times with TBST, the chemical luminescent reaction was carried out by adding SuperSignal West Pico Chemiluminescent Substrate (manufactured by ThermoScientific) to the PVDF membrane.

Using the imaging system LAS4000 mini (manufactured by Fujifilm), the luminescence images were obtained. Meanwhile, for the purpose of analyzing the amount of each FLAG/Fc fusion protein on the PVDF membrane, the experiment was also performed in which a peroxidase-labeled rabbit anti-human IgG antibody (manufactured by American Qualex) which recognized human Fc was allowed to react with the PVDF membrane without the primary antibody reaction for one hour, followed by the chemical luminescent reaction.

Fig. 16(a) shows the result of the experiment in which KM4590 was reacted as a primary antibody in Western blotting. In addition, Fig. 16(b) shows the result of the experiment in which cAR47A6.4.2 was reacted as a primary antibody in Western blotting.

As shown in Fig. 16(a), in both cases of non-reducing condition and reducing condition, the reactivity of KM4590 for Mutant A and Mutant B in which domain I of Trop-2 was deleted lowered. On the other hand, as shown in Fig. 16(b), the reactivity of cAR47A6.4.2 lowered only to Mutant C in which a region to a part of domain III was deleted.

Based on the above results, it was found out that epitopes of chimeric antibody KM4590 and its mouse monoclonal antibody KM4097 were present in domain I and an epitope of cAR47A6.4.2 was present in domain III.

Next, the reactivity of KM4590 and cAR47A6.4.2 in non-reducing condition and reducing condition of Trop-2/FLAG/Fc proteins was compared. The results of the experiments are shown in Fig. 17(a), Fig. 17(b) and Fig. 18.

From the PVDF membrane to which each of KM4590 and cAR47A6.4.2 was allowed to react by Western blot, the antibodies were detached using Restore™ PLUS Western Blot Stripping Buffer (manufactured by Thermo Scientific) and the blocking was carried out in the same manner as before. By allowing a peroxidase-labeled rabbit anti-human IgG antibody to react with the PVDF membrane, the amount of Trop-2/FLAG/Fc protein was evaluated.

As a result, there were no significant differences in the contained amount of Trop-2/FLAG/Fc protein between two PVDF membranes as shown in Fig. 17(b). In addition, it was found that KM4590 showed very weak reactivity to Trop-2/FLAG/Fc protein under reducing condition while cAR47A6.4.2 showed strong reactivity to Trop-2/FLAG/Fc protein under reducing condition.

Fig. 18 showed results obtained by analyzing and then quantifying the signal intensity of the band, using the image interpretation software, Multi Gauge Ver. 3.0 (manufactured by Fujifilm). Based on the above results, it was shown that KM4590 recognized the structure comprising a disulfide bond in domain I of a Trop-2 protein.

Based on the above results, it was obvious that KM4590 and its mouse antibody KM4097 recognized domain I of the extracellular region of Trop-2 which was different from epitopes of known antibodies.

[Example 8]

Preparation of anti-Trop-2 humanized antibody

(1) Design of amino acid sequences of VH and VL of KM4097 humanized antibody

The amino acid sequence of VH of KM4097 humanized antibody was designed in the following manner. The amino acid sequence of FR of VH of a human antibody for grafting the amino acid sequences of CDR1 to CDR3 represented by SEQ ID NOs:13 to 15, respectively, of KM4097VH was selected in the following manner.
Kabat *et al.* classified the VH of known various human antibodies into three subgroups (HSG I to III) based on the homology of their amino acid sequences and reported on the consensus sequences of each of the subgroups [Sequences of Proteins of Immunological Interest, US Dept. Health and Human Services (1991)]. Accordingly, homology search between the amino acid sequence of FR in the consensus sequences of the subgroups I to III of the VH of human antibodies and the amino acid sequence of FR of KM4097VH was carried out.
As a result of the homology search, the homologies with HSG I, HSG II and HSG III were 74.7%, 55.2% and 59.8%, respectively. Accordingly, the amino acid sequence of FR of KM4097VH had the highest homology with the subgroup I.
Based on the above result, the amino acid sequences (SEQ ID NOs:13 to 15, respectively) of CDRs of the KM4097VH were grafted to an appropriate position of the amino acid sequence of FR in the consensus sequence of subgroup I of VH of a human antibody. In this manner, the amino acid sequence KM4097HV0 of VH of anti-Trop-2 KM4097 humanized antibody represented by SEQ ID NO:24 was designed.
Next, the amino acid sequence of VL of KM4097 humanized antibody was designed in the following manner. The amino acid sequences of FR of VL of a human antibody which were suitable for grafting the amino acid sequences of CDR1 to CDR3 (SEQ ID NOs:16 to 18, respectively) of KM4097VL were selected in the following manner.
Kabat *et al.* classified the VL of known various human antibodies into four subgroups (HSG I to IV) based on the homology of their amino acid sequences and further reported on the consensus sequences for each of the subgroups [Sequences of Proteins of Immunological Interest, US Dept. Health and Human Services (1991)]. Accordingly, homology search among the amino acid sequences of FR in the consensus sequences of the subgroups I to IV of the VL of human antibodies and the amino acid sequence of FR of KM4097VL was performed.
As a result of the homology search, the homologies with HSG I, HSG II, HSG III and HSG IV were 78.8%, 73.8%, 72.5% and 83.8%, respectively. Accordingly, the amino acid sequence of FR of KM4097VL had the highest homology with the subgroup IV.
Based on the above result, the amino acid sequences (SEQ ID NOs:16 to 18) of CDR of the KM4097VL were grafted to an appropriate position of the amino acid sequence of FR of the consensus sequence of subgroup IV of human antibody VL. However, since Leu at position 110 in the amino acid sequence of KM4097VL (SEQ ID NO:12) was not the amino acid residue having highest usage frequency cited by Kabat *et al.,* but was an amino acid residue which is used at a relatively high frequency, the above-mentioned amino acid residue which is recognized in the amino acid sequence of KM4097VL was used.
In this manner, the amino acid sequence KM4097LV0 of VL of anti-Trop-2 KM4097 humanized antibody (SEQ ID NO:26) was designed.
The amino acid sequence KM4097HV0 of VH and the amino acid sequence KM4097LV0 of VL of KM4097 humanized antibody designed in the above are sequences in which merely the amino acid sequences of CDRs of mouse monoclonal antibody KM4097 were grafted to the amino acid sequence of FR of the selected human antibody.
However, in general, when a humanized antibody is prepared, its binding activity is frequently lowered in the case where merely the amino acid sequences of CDRs of a mouse antibody are grafted to the amino acid sequence of FR of a human antibody. In order to avoid lowering of the binding activity, modification of the amino acid residues considered to have influence upon the binding activity, among the amino acid residues in FR which are different between human antibodies and mouse antibodies, is carried out together with the grafting of the amino acid sequences of CDRs.
Thus, the amino acid residues of FR which were considered to have influence upon the binding activity were identified and modified in this Example as follows.
Firstly, three-dimensional structure of an antibody V region (hereinafter referred to as HVOLV0) consisting of the amino acid sequence KM4097HV0 ofVH and the amino acid sequence KM4097LV0 of VL of KM4097 humanized antibody designed in the above was constructed using a computer modeling technique. For the preparation of the three dimensional structure coordinates and display of the three-dimensional structure, Discovery Studio (manufactured by Accelrys) was used. In addition, a computer model of the three-dimensional structure of V region of KM4097 was also constructed in the same manner.

By selecting amino acid residues which were different from those of KM4097 in the amino acid sequences of FR of VH and VL of HV0LV0 and then preparing the amino acid sequence in which the selected amino acid residues were substituted with the amino acid residues of KM4097, a three-dimensional structure model was constructed in the same manner. By comparing three-dimensional structures of the V regions of KM4097, HV0LV0 and modified product, the amino acid residues which were considered to have influence upon the binding activity were identified.

As a result, as the amino acid residues among amino acid residues of FR of HV0LV0, which are considered to change three-dimensional structure of the antigen binding region and have influence upon the binding activity, Ala at position 9, Lys at position 12, Val at position 20, Arg at position 38, Met at position 48, Arg at position 67, Val at position 68, Ile at position 70, Tyr at position 95 and Val at position 112 in the KM4097HV0 and Leu at position 15, Ala at position 19, Ile at position 21 and Leu at position 84 in the KM4097LV0, respectively, were selected.

By modifying at least one or more of these selected amino acid residues into the amino acid residues which were present at the same positions of the amino acid sequence of KM4097, VH and VL of humanized antibody having various modifications were designed.

Specifically, regarding VH, at least one modification was introduced among amino acid modifications for substituting Ala at position 9 with Pro, Lys at position 12 with Val, Val at position 20 with Ile, Arg at position 38 with Lys, Met at position 48 with Ile, Arg at position 67 with Lys, Val at position 68 with Ala, Ile at position 70 with Leu, Tyr at position 95 with Phe and Val at position 112 with Leu in the amino acid sequence represented by SEQ ID NO:24.

In addition, regarding VL, at least one modification was introduced among amino acid modifications for substituting Leu at position 15 with Ala, Ala at position 19 with Val, Ile at position 21 with Met and Leu at position 84 with Val in the amino acid sequence represented by SEQ ID NO:26.

As an antibody V region of KM4097 humanized antibody in which at least one amino acid residue in HV0LV0 in FR was substituted, HV0LV0, HV0LV2, HV0LV3a, HV0LV3b, HV0LV4, HV2LV0, HV2LV2, HV2LV3a, HV2LV3b, HV2LV4, HV3aLV0, HV3aLV2, HV3aLV3a, HV3aLV3b, HV3aLV4, HV3bLV0, HV3bLV2, HV3bLV3a, HV3bLV3b, HV3bLV4, HV4LV0, HV4LV2, HV4LV3a, HV4LV3b, HV4LV4, HV5aLV0, HV5aLV2, HV5aLV3a, HV5aLV3b, HV5aLV4, HV5bLV0, HV5bLV2, HV5bLV3a, HV5bLV3b, HV5bLV4, HV5cLV0, HV5cLV2, HV5cLV3a, HV5cLV3b, HV5cLV4, HV6LV0, HV6LV2, HV6LV3a, HV6LV3b, HV6LV4, HV8LV0, HV8LV2, HV8LV3a, HV8LV3b, HV8LV4, HV9LV0, HV9LV2, HV9LV3a, HV9LV3b, HV9LV4, HV10LV0, HV10LV2, HV10LV3a, HV10LV3b and HV10LV4 were designed.

The amino acid sequences of H chain variable regions HV2, HV3a, HV3b, HV4, HV5a, HV5b, HV5c, HV6, HV8, HV9, HV10 and the amino acid sequences of L chain variable regions LV2, LV3a, LV3b and LV4, are shown in Fig. 12 and Fig. 13, respectively.

(2) Design of cDNA sequences of VH and VL of KM4097 humanized antibody

A DNA encoding the amino acid sequence of a variable region of KM4097 humanized antibody was designed using codons which were used in DNAs (SEQ ID NOs:23 and 25) encoding amino acid sequences of KM4097VH and KM4097VL, respectively. When the amino acid modification was carried out, the DNA was designed and prepared using codons which was used in high frequency in mammalian cells. Using these DNA sequences, antibody expression vectors were constructed and humanized antibodies were expressed.

(3) Construction of cDNA encoding VH of KM4097 humanized antibody

cDNAs (SEQ ID NOs:23, 27, 29, 31 and 33) encoding the amino acid sequence KM4097HV0 (SEQ ID NO:24) of VH of KM4097 humanized antibody designed in Example 1; and HV3a (SEQ ID NO:28), HV4 (SEQ ID NO:30), HV5 (SEQ ID NO:32) and HV10 (SEQ ID NO:34) which were designed in Example 1 and were shown in Fig. 12, respectively, were prepared by full-length synthesis.

(4) Construction of cDNA encoding VL of KM4097 humanized antibody

cDNAs (SEQ ID NOs:25, 35 and 37) encoding the amino acid sequence LV0 (SEQ ID NO:26) of VL of KM4097 humanized antibody which were designed in Example 1; and LV2 (SEQ ID NO:36) and LV4 (SEQ ID NO:38) which were designed in Example 1 and shown in Fig. 13, respectively, were prepared in full-length synthesis.

(5) Construction of vector for expression of KM4097 humanized antibody

Various vectors for expression of KM4097 humanized antibody were constructed by inserting cDNA encoding any one of HV0, HV3a, HV4, HV5 and HV10 and cDNA encoding any one of LV0, LV2 and LV4 obtained in (3) and (4) of this Example into appropriate sites of the vector for expression of humanized antibody pKANTEX93 described in WO97/10354.

(6) Expression of KM4097 humanized antibody using animal cells

A cell line (FUT8 knockout CHO cell) in which $\alpha$1,6-fucosyltransferase (FUT8) gene is knocked out in a CHO-K1 cell (The Institute of Physical and Chemical Research Cell Bank, Accession No. RCB0403) was used for a gene introduction of the antibody expression vector for an animal cell which was constructed in Example 5.

Using a FUT8 knockout CHO cell as a host cell, a series of step of a gene introduction to the expression of antibody was carried out based on the instructions of FreeStyle™ MAX CHO Expression System (manufactured by Invitrogen). With OptiPro™ SFM (manufactured by Invitrogen), 312.5 $\mu$g of the expression vector plasmid was mixed and the total volume was adjusted to 5 ml.

With OptiPro™ SFM, 312.5 $\mu$l of FreeStyle™ MAX Transfection Reagent (manufactured by Invitrogen) was mixed and the total volume was adjusted to 5 ml. The expression vector plasmid solution and the FreeStyle™ MAX Transfection Reagent were mixed and allowed to stand at room temperature for 10 minutes. Then, all the mixed vector solution was added to 250 ml of FUT8 knockout CHO cells with a cell density of $1.0 \times 10^6$ cells/ml cultured with FreeStyle™ CHO Expression Medium (manufactured by Invitrogen) and cultured in suspension at 37°C, 8% $CO_2$, on an orbital shaker at 135 rpm for 5 to 7 days.

After culture, the cell suspension was centrifuged for 20 minutes under conditions of 3,000 rpm at 4°C to recover the culture supernatant and then the culture supernatant was filtration-sterilized using Millex GV filter having a pore size of 0.22 $\mu$m.

(7) Purification of KM4097 humanized antibody

A column having a diameter of 0.8 cm was filled with 0.5 ml of MabSelect SuRe (manufactured by GE Healthcare) and the carrier was equilibrated by loading 3.0 ml of purified water, 2.0 ml of 0.1 M citric acid buffer (pH 3.5) and 1.5 ml of 150 mM NaCl, 0.2 M sodium borate buffer (pH 7.5) in order. Next, the supernatant recovered in this Example 6 was loaded onto the column and the column was washed with 5 ml of 150 mM NaCl, 0.2 M sodium borate buffer (pH 7.5). After washing, the antibody adsorbed to the carrier was eluted using 2 ml of 0.1 M citric acid buffer (pH 3.5).

The elution was carried out to obtain four fractions of 500 $\mu$l per fraction. Next, through SDS-PAGE analysis of the thus purified fraction, fractions in which the elution of the antibody was confirmed were gathered. Then, the gathered fractions were dialyzed with 150 mM NaCl, 10 mM sodium citrate solution (pH 6.0) overnight at 4°C. After dialysis, a solution of anti-Trop-2 KM4097 humanized antibody was recovered. After filtration sterilization using Millex GV filter having a pore size of 0.22 $\mu$m (manufactured by Millipore), the absorbance at 280 nm (OD 280 nm) was measured by an absorptiometer (SHIMADZU UV-1700) and the concentration of each purified KM4097 humanized antibody was calculated.

Based on the above, six kinds of KM4097 humanized antibodies, HV0LV0 in which VH and VL of the antibody were HV0 and LV0, respectively; HV3aLV0 in which VH and VL of the antibody were HV3a and LV0, respectively; HV4LV0 in which VH and VL of the antibody were HV4 and LV0, respectively; HV5LV0 in which VH and VL of the antibody were HV5 and LV0, respectively; HV10LV2 in which VH and VL of the antibody were HV10 and LV2, respectively; and HV10LV4 in which VH and VL of the antibody were HV10 and LV4, respectively, were prepared.

(8) Determination of fucose content in KM4097 humanized antibody

In accordance with the method described in WO2002/31140, a ratio of a sugar chain in which fucose was not bound to the complex-type N-linked sugar chains present in six kinds of KM4097 humanized antibodies prepared in this Example 7 was examined. As a result, it was found that fucose was not attached to the six kinds of KM4097 humanized antibodies prepared in Example 7.

[Example 9]

Activity evaluation of KM4097 humanized antibodies

The activities of KM4591 obtained in Example 5 and six kinds of KM4097 humanized antibodies (HV0LV0, HV3aLV0, HV4LV0, HV5LV0, HV10LV2 and HV10LV4) obtained in Example 8 were evaluated.

(1) Evaluation of Binding activity on KM4097 humanized antibody to recombinant human Trop-2 using Biacore

In the same manner described in Example 3(2), the affinity of KM4591 and six kinds of KM4097 humanized antibodies (HV0LV0, HV3aLV0, HV4LV0, HV5LV0, HV10LV2 and HV10LV4) obtained in Example 8 to human Trop-2/Fc/His fusion proteins (manufactured by R&D) were evaluated.

ka, kd and K$_D$ (kd/ka) of each antibody are shown in Table 6 (the mean value of duplicate experiments).

|         | kd (1/Ms)            | ka (1/s)             | K$_D$ (nM) |
|---------|----------------------|----------------------|-----------|
| KM4591  | $3.23\times10^5$     | $1.13\times10^{-4}$  | 0.36      |
| HV0LV0  | $3.30\times10^5$     | $1.60\times10^{-4}$  | 0.53      |
| HV3aLV0 | $5.06\times10^5$     | $1.60\times10^{-4}$  | 0.30      |
| HV4LV0  | $3.35\times10^5$     | $1.26\times10^{-4}$  | 0.42      |
| HV5LV0  | $3.83\times10^5$     | $1.90\times10^{-4}$  | 0.49      |
| HV10LV2 | $3.75\times10^5$     | $1.48\times10^{-4}$  | 0.40      |
| HV10LV4 | $3.42\times10^5$     | $1.16\times10^{-4}$  | 0.37      |

As shown in Table 6, all the anti-Trop-2 KM4097 humanized antibodies (HV0LV0, HV3aLV0, HV4LV0, HV5LV0, HV10LV2 and HV10LV4) exhibited high affinity value (K$_D$) of $1\times10^{-9}$ M or less. Among them, five kinds of KM4097 humanized antibodies (HV3aLV0, HV4LV0, HV5LV0, HV10LV2 and HV10LV4) exhibited high K$_D$ value of $5\times10^{-10}$ M or less.

(2) Evaluation of the ADCC activity on anti-Trop-2 humanized antibody on human Trop-2 expression cell line

(2)-1 Preparation of target cell suspension

A tag-added human Trop-2 expression vector was constructed by inserting the DNA (SEQ ID NO:49) encoding the sequence (SEQ ID NO:48) in which myc tag and His tag were added to the C-terminus of human Trop-2 into the appropriate site of pKANTEX93. The transformant which was obtained by introducing the expression vector into the CHO/DG44 cells was used for the target cell of the present evaluation. After washing with PBS, the cells were washed with phenol red-free RPMI1640 medium comprising 5% fetal bovine serum (FBS, manufactured by Invitrogen) (hereinafter referred as "the ADCC activity measuring medium", manufactured by Invitrogen) and the cells were adjusted to be optimum with the same medium and used as the target cell suspension.

(2)-2 Preparation of effector cell suspension

Peripheral blood mononuclear cells (PBMC, manufactured by AllCells) stored in frozen state were thawed and suspended in RPMI medium comprising DNase I (manufactured by Roche Applied Science) and 10% FBS and then centrifuged at 1,500 rpm for 10 minutes. After removing the supernatant, the cells were suspended in RPMI medium comprising DNase I and 10% FBS and incubated at 37(C for 20 minutes. After the suspension was centrifuged at 1,500 rpm for 10 minutes, the supernatant was removed and the thus suspension was washed twice with the ADCC activity measuring medium. Then the cell density was adjusted to be optimum with the same medium and used as the effector cell suspension.

(2)-3 Measurement of ADCC activity

Into each of wells of a 96-well U bottom plate (manufactured by Falcon), the antibody solution in which each antibody was serially ten-fold diluted, starting from 3 μg/ml was dispensed at 50 μl. Next, the target cell solution prepared in the above-mentioned (2)-1 was dispensed at $1\times104$ cells/50 μl/well. After the effector cell solution prepared in the above-mentioned (2)-2 was further dispensed at $2.5\times105$ cells/50 μl/well into the 96-well U bottom plate to give a total volume of 150 μl, the mixed solution was allowed to react at 37°C for 24 hours. The ratio of the effector cell (E) and target cell (T) was set to be 25:1 in the present system. After the reaction, color development in each supernatant was measured using LDH Cytotoxic Test (manufactured by Wako Pure Chemical Industries, Ltd.).

The ADCC activity was calculated by the following formula. Those results are shown in Fig. 19.

(Formula)

$$\text{ADCC activity (\%)} = \{([\text{absorbance of sample}]-[\text{absorbance of target cell spontaneous release}]-[\text{absorbance of effector cell spontaneous release}])/([\text{absorbance of target cell total release}]-[\text{absorbance of target cell spontaneous release}])\}\times100$$

As shown in Fig. 19, the prepared humanized antibodies HV3aLV0 exhibited the same ADCC activity as KM4591.

While the invention has been described in detail and with reference to specific embodiments thereof, it will be apparent to one skill in the art that various changes and modifications can be made therein without departing from the spirit and scope thereof. This application is based on U.S. provisional application No. 61/353,430, filed on June 10, 2010, the entire contents of which are incorporated hereinto by reference. All references cited herein are incorporated in their entirety.

Industrial Applicability

According to the present invention, a monoclonal antibody or an antibody fragment thereof, which binds to the extracellular region of human Trop-2 with high affinity and exhibits high antibody-dependent cellular cytotoxicity (hereinafter referred to as "ADCC activity") and high antitumor activity; a hybridoma which produces the antibody; a DNA which encodes the antibody; a vector which comprises the DNA; a transformant obtainable by introducing the vector; a process for producing an antibody or an antibody fragment thereof using the hybridoma or the transformant; and a therapeutic agent or a diagnostic agent using the antibody or the antibody fragment thereof can be provided.

Free Text of Sequence Listing

SEQ ID NO:1 : Human Trop-2 amino acid sequence
SEQ ID NO:2 : Human Trop-2 nucleotide sequence
SEQ ID NO:3 : AR47A6.4.2 VH nucleotide sequence
SEQ ID NO:4 : AR47A6.4.2 VL nucleotide sequence
SEQ ID NO:5 : Mouse IgG1 primer mG1a1 nucleotide sequence
SEQ ID NO:6 : Mouse IgG1 primer mG1a2 nucleotide sequence
SEQ ID NO:7 : Mouse κ chain primer Ka1 nucleotide sequence
SEQ ID NO:8 : Mouse κ chain primer Ka2 nucleotide sequence
SEQ ID NO:9 : KM4097 VH nucleotide sequence
SEQ ID NO:10 : KM4097 VH amino acid sequence
SEQ ID NO:11 : KM4097 VL nucleotide sequence
SEQ ID NO:12 : KM4097 VL amino acid sequence
SEQ ID NO:13 : KM4097 VHCDR1 amino acid sequence
SEQ ID NO:14 : KM4097 VHCDR2 amino acid sequence
SEQ ID NO:15 : KM4097 VHCDR3 amino acid sequence
SEQ ID NO:16 : KM4097 VLCDR1 amino acid sequence
SEQ ID NO:17 : KM4097 VLCDR2 amino acid sequence
SEQ ID NO:18 : KM4097 VLCDR3 amino acid sequence
SEQ ID NO:19 : Nucleotide sequence of a forward primer for amplification of KM4097 VH chain to construct KM4097 chimeric antibody
SEQ ID NO:20 : Nucleotide sequence of a reverse primer for amplification of KM4097 VH chain to construct KM4097 chimeric antibody
SEQ ID NO:21 : Nucleotide sequence of a forward primer for amplification of KM4097 VL chain to construct KM4097 chimeric antibody
SEQ ID NO:22 : Nucleotide sequence of a reverse primer for amplification of KM4097 VL chain to construct KM4097 chimeric antibody
SEQ ID NO:23 : KM4097 HV0 variable region nucleotide sequence
SEQ ID NO:24 : KM4097 HV0 variable region amino acid sequence
SEQ ID NO:25 : KM4097 LV0 variable region nucleotide sequence
SEQ ID NO:26 : KM4097 LV0 variable region amino acid sequence
SEQ ID NO:27 : KM4097 HV3a variable region nucleotide sequence
SEQ ID NO:28 : KM4097 HV3a variable region amino acid sequence
SEQ ID NO:29 : KM4097 HV4 variable region nucleotide sequence
SEQ ID NO:30 : KM4097 HV4 variable region amino acid sequence
SEQ ID NO:31 : KM4097 HV5 variable region nucleotide sequence
SEQ ID NO:32 : KM4097 HV5 variable region amino acid sequence
SEQ ID NO:33 : KM4097 HV10 variable region nucleotide sequence
SEQ ID NO:34 : KM4097 HV10 variable region amino acid sequence

SEQ ID NO:35 : KM4097 LV2 variable region nucleotide sequence
SEQ ID NO:36 : KM4097 LV2 variable region amino acid sequence
SEQ ID NO:37 : KM4097 LV4 variable region nucleotide sequence
SEQ ID NO:38 : KM4097 LV4 variable region amino acid sequence
SEQ ID NO:39 : Nucleotide sequence of FLAG tag and Fc
SEQ ID NO:40 : Nucleotide sequence in which signal sequence was added to the extracellular region of Trop-2
SEQ ID NO:41 : Nucleotide sequence in which FLAG tag and Fc region were removed from Mutant A
SEQ ID NO:42 : Nucleotide sequence in which FLAG tag and Fc region were removed from Mutant B
SEQ ID NO:43 : Nucleotide sequence in which FLAG tag and Fc region were removed from Mutant C
SEQ ID NO:44 : Trop-2/FLAG/Fc amino acid sequence
SEQ ID NO:45 : Mutant A amino acid sequence
SEQ ID NO:46 : Mutant B amino acid sequence
SEQ ID NO:47 : Mutant C amino acid sequence
SEQ ID NO:48 : C-terminal tagged human Trop-2 amino acid sequence
SEQ ID NO:49 : C-terminal tagged human Trop-2 nucleotide sequence

**Claims**

1.  I A monoclonal antibody or an antibody fragment thereof against human Trop-2, which binds to at least domain I in an extracellular region of human Trop-2.

2.  The monoclonal antibody or the antibody fragment thereof according to claim 1, which binds to at least one amino acid in amino acids at positions 34 to 72 in the amino acid sequence represented by SEQ ID NO:1.

3.  The monoclonal antibody or the antibody fragment thereof according to claim 1 or 2, which has a dissociation constant ($K_D$) of $5 \times 10^{-10}$M or less against an antigen of an antibody.

4.  The monoclonal antibody or the antibody fragment thereof according to any one of claims 1 to 3, which has high ADCC activity and antitumor activity.

5.  The monoclonal antibody or the antibody fragment thereof according to any one of claims 1 to 4, wherein CDR1 to 3 of an H chain of the antibody comprise the amino acid sequences represented by SEQ ID NOs:13 to 15, respectively; and CDR1 to 3 of an L chain of the antibody comprise the amino acid sequences represented by SEQ ID NOs:16 to 18, respectively.

6.  A monoclonal antibody or an antibody fragment thereof, which binds to an epitope which is the same as an epitope in an extracellular region of Trop-2 to which a monoclonal antibody comprising VH comprising the amino acid sequence represented by SEQ ID NO:10 and VL comprising the amino acid sequence represented by SEQ ID NO: 12 binds.

7.  A monoclonal antibody or an antibody fragment thereof, which binds to an extracellular region of Trop-2 while competing with the antibody according to claim 5 or 6.

8.  The monoclonal antibody or the antibody fragment thereof according to any one of claims 1 to 7, which is a recombinant antibody.

9.  The monoclonal antibody or the antibody fragment thereof according to claim 8, which is a recombinant antibody selected from a human chimeric antibody, a humanized antibody and a human antibody.

10. The monoclonal antibody or the antibody fragment thereof according to claim 9, which is a humanized antibody and comprises at least one of the following (a) VH and (b) VL:

    (a) VH which comprises the amino acid sequence represented by SEQ ID NO:24 or an amino acid sequence in which at least one modification in amino acid modifications for substituting Ala at position 9 with Pro, Lys at position 12 with Val, Val at position 20 with Ile, Arg at position 38 with Lys, Met at position 48 with Ile, Arg at position 67 with Lys, Val at position 68 with Ala, Ile at position 70 with Leu, Tyr at position 95 with Phe and Val at position 112 with Leu is introduced in the amino acid sequence represented by SEQ ID NO:24,
    (b) VL which comprises the amino acid sequence represented by SEQ ID NO:26 or an amino acid sequence in which at least one modification in amino acid modifications for substituting Leu at position 15 with Ala, Ala at position 19 with Val, Ile at position 21 with Met and Leu at position 84 with Val is introduced in the amino acid sequence represented by SEQ ID NO:26.

11. The monoclonal antibody or the antibody fragment thereof according to claim 10, which is selected from the following (1) to (5):

    (1) a humanized antibody which comprises at least one of VH of the antibody comprising the amino acid sequence represented by SEQ ID NO:28 and VL of the antibody comprising the amino acid sequence represented by SEQ ID NO:26,
    (2) a humanized antibody which comprises at least one of VH of the antibody comprising the amino acid sequence represented by SEQ ID NO:30 and VL of the antibody comprising the amino acid sequence represented by SEQ ID NO:26,

(3) a humanized antibody which comprises at least one of VH of the antibody comprising the amino acid sequence represented by SEQ ID NO:32 and VL of the antibody comprising the amino acid sequence represented by SEQ ID NO:26,

(4) a humanized antibody which comprises at least one of VH of the antibody comprising the amino acid sequence represented by SEQ ID NO:34 and VL of the antibody comprising the amino acid sequence represented by SEQ ID NO:36,

(5) a humanized antibody which comprises at least one of VH of the antibody comprising the amino acid sequence represented by SEQ ID NO:34 and VL of the antibody comprising the amino acid sequence represented by SEQ ID NO:38.

12. The antibody fragment according to any one of claims 1 to 11, which is an antibody fragment selected from Fab, Fab', F(ab')$_2$, a single chain antibody (scFv), a dimerized V region (diabody), a disulfide stabilized V region (dsFv) and a peptide comprising CDR.

13. A DNA which encodes the monoclonal antibody or the antibody fragment according to any one of claims 1 to 12.

14. A recombinant vector comprising the DNA according to claim 13.

15. A transformant obtainable by introducing the recombinant vector according to claim 14 into a host cell.

16. A process for producing the monoclonal antibody or the antibody fragment thereof according to any one of claims 1 to 12, which comprises culturing the transformant according to claim 14 in a medium to thereby form and accumulate the antibody or the antibody fragment thereof according to any one of claims 1 to 12 in culture, and recovering the antibody or the antibody fragment thereof from the culture.

17. A reagent for detecting or measuring human Trop-2, which comprises the monoclonal antibody or the antibody fragment thereof according to any one of claims 1 to 12.

18. A diagnostic agent for a disease relating to human Trop-2-positive cells, which comprises the monoclonal antibody or the antibody fragment thereof according to any one of claims 1 to 12 as an active ingredient.

19. The diagnostic agent according to claim 18, wherein the disease relating to human Trop-2-positive cells is a cancer.

20. A therapeutic agent for a disease relating to human Trop-2-positive cells, which comprises the monoclonal antibody or the antibody fragment thereof according to any one of claims 1 to 12 as an active ingredient.

21. The therapeutic agent according to claim 20, wherein the disease relating to human Trop-2-positive cells is a cancer.

22. A method for diagnosing a disease relating to human Trop-2-positive cells, which comprises using the monoclonal antibody or the antibody fragment thereof according to any one of claims 1 to 12 to detect or measure human Trop-2-positive cells.

23. A method for diagnosing a disease relating to human Trop-2-positive cells, which comprises using the monoclonal antibody or the antibody fragment thereof according to any one of claims 1 to 12 to detect or measure human Trop-2.

24. The diagnosing method according to claim 22 or 23, wherein the disease relating to human Trop-2-positive cells is a cancer.

25. Use of the monoclonal antibody or the antibody fragment thereof according to any one of claims 1 to 12 for manufacture of a diagnostic agent for a disease relating to human Trop-2-positive cells.

26. The use of the monoclonal antibody or the antibody fragment thereof according to claim 25, wherein the disease relating to human Trop-2-positive cells is a cancer.

27. Use of the monoclonal antibody or the antibody fragment thereof according to any one of claims 1 to 12 for manufacture of a therapeutic agent for a disease relating to human Trop-2-positive cells.

**28.** The use of the monoclonal antibody or the antibody fragment thereof according to claim 27, wherein the disease relating to human Trop-2-positive cells is a cancer.

Fig. 1

(a)

(b)

**BxPC-3**

**CaOV-3**

(c)

(d)

**MCF-7**

**Colo 205**

Fig. 2

Fig. 3

# Fig. 4

Fig. 5

Fig. 6

(a)

(b)

## Fig. 7

(a)

(b)

Fig. 8

(a)

(b)

Fig. 9

(a)

(b)

Fig. 10

Fig. 11

# Fig. 12

```
                      1           2           3           4           5           6
            1234567890  1234567890  1234567890  1234567890  1234567890  1234567890
KM4097HV0   QVQLVQSGAE  VKKPGASVKV  SCKASGYTFT  IYWLGWVRQA  PGQGLEWMGN  IFPGSAYINY
KM4097HV2                                            K           I
KM4097HV3a        P
KM4097HV3b                                           K           I
KM4097HV4         P                                  K           I
KM4097HV5a        P
KM4097HV5b        P                                  K           I
KM4097HV5c                                           K           I
KM4097HV6         P                                  K           I
KM4097HV8         P             I                    K           I
KM4097HV9         P      V       I                   K           I
KM4097HV10        P      V       I                   K           I


                      7           8           9          10          11
            1234567890  1234567890  1234567890  1234567890  1234567890  123456
KM4097HV0   NEKFKGRVTI  TADTSTSTAY  MELSSLRSED  TAVYYCAREG  SNSGYWGQGT  LVTVSS
KM4097HV2
KM4097HV3a       K                                   F
KM4097HV3b                                           F
KM4097HV4                                            F
KM4097HV5a       KA L                                F
KM4097HV5b       K                                   F
KM4097HV5c       KA                                  F
KM4097HV6        KA                                  F
KM4097HV8        KA L                                F
KM4097HV9        KA L                                F
KM4097HV10       KA L                                F                           L
```

EP 2 594 589 A1

# Fig. 13

<table>
<thead>
<tr><th></th><th>1<br>1234567890</th><th>2<br>1234567890</th><th>3<br>1234567890</th><th>4<br>1234567890</th><th>5<br>1234567890</th><th>6<br>1234567890</th></tr>
</thead>
<tbody>
<tr><td>KM4097LV0</td><td>DIVMTQSPDS</td><td>LAVSLGERAT</td><td>INCKSSQSLL</td><td>NSGNQQNYLA</td><td>WYQQKPGQPP</td><td>KLLIYGASTR</td></tr>
<tr><td>KM4097LV2</td><td></td><td>V</td><td></td><td></td><td></td><td></td></tr>
<tr><td>KM4097LV3a</td><td>A</td><td>V</td><td></td><td></td><td></td><td></td></tr>
<tr><td>KM4097LV3b</td><td></td><td>V M</td><td></td><td></td><td></td><td></td></tr>
<tr><td>KM4097LV4</td><td>A</td><td>V M</td><td></td><td></td><td></td><td></td></tr>
</tbody>
</table>

<table>
<thead>
<tr><th></th><th>7<br>1234567890</th><th>8<br>1234567890</th><th>9<br>1234567890</th><th>10<br>1234567890</th><th>11<br>1234567890</th><th>123</th></tr>
</thead>
<tbody>
<tr><td>KM4097LV0</td><td>ESGVPDRFSG</td><td>SGSGTDFTLT</td><td>ISSLQAEDVA</td><td>VYYCQSDHIY</td><td>PYTFGQGTKL</td><td>EIK</td></tr>
<tr><td>KM4097LV2</td><td></td><td></td><td>V</td><td></td><td></td><td></td></tr>
<tr><td>KM4097LV3a</td><td></td><td></td><td>V</td><td></td><td></td><td></td></tr>
<tr><td>KM4097LV3b</td><td></td><td></td><td>V</td><td></td><td></td><td></td></tr>
<tr><td>KM4097LV4</td><td></td><td></td><td>V</td><td></td><td></td><td></td></tr>
</tbody>
</table>

## Fig. 14

(a)

(b)

(c)

## Fig. 14

(d)

(e)

(f)

Fig. 15

Fig. 16

(a)

KM4590

Non-reducing  Reducing

1 2 3 4    1 2 3 4

(b)

cAR47A6.4.2

Non-reducing  Reducing

1 2 3 4    1 2 3 4

(c)

Anti-Fc

Non-reducing  Reducing

1 2 3 4    1 2 3 4

Fig. 17

Fig. 18

EP 2 594 589 A1

Fig. 19

## INTERNATIONAL SEARCH REPORT

| International application No. |
| --- |
| PCT/JP2011/063294 |

A. CLASSIFICATION OF SUBJECT MATTER
*C07K16/28*(2006.01)i, *A61K39/395*(2006.01)i, *A61K45/00*(2006.01)i, *C12N5/10*
(2006.01)i, *C12N15/09*(2006.01)i, *C12P21/08*(2006.01)i

According to International Patent Classification (IPC) or to both national classification and IPC

B. FIELDS SEARCHED

Minimum documentation searched (classification system followed by classification symbols)
C07K16/28, A61K39/395, A61K45/00, C12N5/10, C12N15/09, C12P21/08

Documentation searched other than minimum documentation to the extent that such documents are included in the fields searched
| | | | |
| --- | --- | --- | --- |
| Jitsuyo Shinan Koho | 1922–1996 | Jitsuyo Shinan Toroku Koho | 1996–2011 |
| Kokai Jitsuyo Shinan Koho | 1971–2011 | Toroku Jitsuyo Shinan Koho | 1994–2011 |

Electronic data base consulted during the international search (name of data base and, where practicable, search terms used)
CA/BIOSIS/MEDLINE/WPIDS(STN), JSTPlus/JMEDPlus/JST7580(JDreamII),
GenBank/EMBL/DDBJ/GeneSeq, UniProt/GeneSeq, SwissProt/PIR/GeneSeq

C. DOCUMENTS CONSIDERED TO BE RELEVANT

| Category* | Citation of document, with indication, where appropriate, of the relevant passages | Relevant to claim No. |
| --- | --- | --- |
| Y/A | FORNARO, M. et al, Cloning of the gene encoding TROP-2, a cell-surface glycoprotein expressed by human carcinomas., International Journal of Cancer, 1995, Vol. 62, No. 5, p. 610-618 | 1-4,6-9, 12-21,25-28/ 5,10,11 |
| Y/A | WO 97/14796 A1 (BRISTOL-MYERS SQIBB CO.), 24 April 1997 (24.04.1997), a whole article & JP 2001-501801 A    & US 5840854 A & EP 856054 A | 1-4,6-9, 12-21,25-28/ 5,10,11 |

| ☒ Further documents are listed in the continuation of Box C. | ☐ See patent family annex. |
| --- | --- |

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 30 August, 2011 (30.08.11) | 06 September, 2011 (06.09.11) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

**INTERNATIONAL SEARCH REPORT**

| International application No. |
|---|
| PCT/JP2011/063294 |

C (Continuation). DOCUMENTS CONSIDERED TO BE RELEVANT

| Category* | Citation of document, with indication, where appropriate, of the relevant passages | Relevant to claim No. |
|---|---|---|
| Y/A | WO 2003/74566 A2 (IMMUNOMEDICS, INC.),<br>12 September 2003 (12.09.2003),<br>a whole article<br>& US 2004/0001825 A1 & US 2007/0212350 A1<br>& US 2010/0221177 A1 & US 2008/0131363 A1<br>& US 2011/0070156 A & EP 1483295 A<br>& EP 2287201 A2 & WO 2003/074566 A2 | 1-4,6-9,<br>12-21,25-28/<br>5,10,11 |
| Y/A | WO 2007/95748 A1 (ARIUS RESEARCH, INC.),<br>30 August 2007 (30.08.2007),<br>a whole article<br>& JP 2009-528995 A & US 2007/0202043 A1<br>& EP 1996700 A | 1-4,6-9,<br>12-21,25-28/<br>5,10,11 |
| Y/A | WO 2007/95749 A1 (ARIUS RESEARCH, INC.),<br>30 August 2007 (30.08.2007),<br>a whole article<br>& JP 2009-527230 A & US 2007/0202043 A1<br>& EP 1996701 A | 1-4,6-9,<br>12-21,25-28/<br>5,10,11 |
| Y/A | WO 2008/144891 A1 (ARIUS RESEARCH, INC.),<br>04 December 2008 (04.12.2008),<br>a whole article<br>& JP 2010-528056 A & US 2008/0131428 A1<br>& EP 2155791 A | 1-4,6-9,<br>12-21,25-28/<br>5,10,11 |

Form PCT/ISA/210 (continuation of second sheet) (July 2009)

## INTERNATIONAL SEARCH REPORT

International application No.

PCT/JP2011/063294

| Box No. II | Observations where certain claims were found unsearchable (Continuation of item 2 of first sheet) |
| --- | --- |

This international search report has not been established in respect of certain claims under Article 17(2)(a) for the following reasons:

1. ☒ Claims Nos.: 22-24
because they relate to subject matter not required to be searched by this Authority, namely:
The inventions set forth in claims 22 to 24 pertain to diagnostic methods to be practiced on the human body and thus relate to a subject matter on which the International Searching Authority is not required to carry out a search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

| Box No. III | Observations where unity of invention is lacking (Continuation of item 3 of first sheet) |
| --- | --- |

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9714796 A
- WO 2003074566 A
- WO 2007095748 A
- WO 2007095749 A
- WO 2008144891 A
- JP 61178926 A
- JP 56023587 A
- JP 2117920 A
- JP 58110600 A
- JP 60221091 A
- US 4686191 A
- US 4939094 A
- US 5160735 A
- JP 3022979 A
- JP 2227075 A
- WO 9710354 A

- JP 63000299 A
- JP 5336963 A
- WO 9423021 A
- JP 2257891 A
- WO 0535586 A
- WO 0231140 A
- WO 2005035586 A
- WO 200231140 A
- WO 0061739 A
- US 20070148165 A
- US 6737056 B
- US 7297775 B
- US 7317091 B
- US 7420040 B
- US 61353430 A

### Non-patent literature cited in the description

- *Proc. Natl. Acad. Sci. USA,* 1981, vol. 8, 5147
- *Int. J. Cancer,* 1995, vol. 62, 610
- *Mod. Pathol.,* 2008, vol. 21, 186
- *Clin. Cancer Res.,* 2006, vol. 12, 3057
- *Br. J. Cancer,* 2008, vol. 99, 1290
- *J. Clin. Pathol.,* 2009, vol. 62, 152
- *Eur. J. Cancer,* 2010, vol. 46, 944
- *Cancer Res.,* 2002, vol. 62, 5325
- *Lab. Invest.,* 2004, vol. 84, 320
- *Mol. Cancer Ther.,* 2008, vol. 7, 280
- *Int. J. Cancer,* 1987, vol. 39, 297
- *Cancer Res.,* 1990, vol. 50, 1330
- *Hybridoma,* 1992, vol. 11, 539
- *Breast Cancer Res. Treat.,* 2004, vol. 84, 173
- *Mol. Cell. Biol.,* 2001, vol. 21, 2570
- *Clin. Cancer Res.,* 2011, vol. 17, 3157
- Molecular Cloning, A Laboratory Manual. Cold Spring Harbor Laboratory Press, 1989
- Current Protocols in Molecular Biology. John Wiley & Sons, 1987
- *Nucleic Acids Research,* 1982, vol. 10, 6487
- *Proc. Natl. Acad. Sci. USA,* 1982, vol. 79, 6409
- *Gene,* 1985, vol. 34, 315
- *Nucleic Acids Research,* 1985, vol. 13, 4431

- *Proc. Natl. Acad. Sci. USA,* 1985, vol. 82, 488
- Molecular Cloning, A Laboratory Manual. Cold Spring Harbor Lab. Press, 1989
- DNA Cloning 1: Core Techniques, A Practical Approach. 1995
- *J. Mol. Biol.,* 1990, vol. 215, 403
- *Nucleic Acids Res.,* 1997, vol. 25, 33-89
- *Genome Res.,* 1997, vol. 7, 649, http://www.ncbi.nlm.nih.gov/Education/BLASTinfo/information3.html
- *Cancer Immunol. Immunother.,* 1993, vol. 36, 373
- Monoclonal Antibodies - Principles and practice. Academic Press, 1996
- Antibodies - A Laboratory Manual. Cold Spring Harbor Laboratory, 1988
- Monoclonal Antibody Experimental Manual. Kodan-sha Scientific, 1987
- Sequences of Proteins of Immunological Interest. US Dept. Health and Human Services, 1991
- Molecular Cloning. Cold Spring Harbor Laboratory Press, 1989
- *Protein Engineering,* 1994, vol. 7, 697
- Antibody Engineering Handbook. Chijin Shokan, 1994
- Rinsho Syuyo-gaku. *Gan to Kagaguryoho-Sha,* 1996

- Ensho to Kouensho-Ryoho. Ishiyaku Shuppann, 1982
- Bioconjugate Drug. Hirokawa Shoten, 1993
- *Agricultural Biological Chemistry,* 1984, vol. 48, 669
- *Agric. Biol. Chem.,* 1989, vol. 53, 277
- *Proc. Natl. Acad. Sci. USA,* 1985, vol. 82, 4306
- *J. Bacteriol.,* 1990, vol. 172, 2392
- *Proc. Natl. Acad. Sci. USA,* 1972, vol. 69, 2110
- *Gene,* 1982, vol. 17, 107
- *Molecular & General Genetics,* 1979, vol. 168, 111
- *Cytotechnology,* 1990, vol. 3, 133
- *Nature,* 1987, vol. 329, 840
- *J. Biochemistry,* 1987, vol. 101, 1307
- *Journal of Experimental Medicine,* 1958, vol. 108, 945
- *Proc .Natl. Acad. Sci. USA,* 1968, vol. 60, 1275
- *Genetics,* 1968, vol. 55, 513
- *Chromosoma,* 1973, vol. 41, 129
- *Methods in Cell Science,* 1996, vol. 18, 115
- *Radiation Research,* 1997, vol. 148, 260
- *Proc. Natl. Acad. Sci. USA,* 1980, vol. 77, 4216
- *Proc. Natl. Acad. Sci.,* 1968, vol. 60, 1275
- *Cell,* 1975, vol. 6, 121
- *Molecular Cell Genetics, Appendix I, II,* 883-900
- *Proc. Natl. Acad. Sci. USA,* 1987, vol. 84, 7413
- *The Journal of the American Medical Association,* 1967, vol. 199, 519
- *Science,* 1952, vol. 122, 501
- *Virology,* 1959, vol. 8, 396
- *Proceeding of the Society for the Biological Medicine,* 1950, vol. 73, 1
- **PAULSON et al.** *J. Biol. Chem.,* 1989, vol. 264, 17619
- **LOWE et al.** *Proc. Natl. Acad. Sci. USA,* 1989, vol. 86, 8227
- *Genes Develop.,* 1990, vol. 4, 1288
- Antibodies-A Laboratory Manual. Cold Spring Harbor Laboratory, 1988
- *Current Topics in Microbiology and Immunology,* 1978, vol. 18, 1
- *European J. Immunology,* 1976, vol. 6, 511
- *Nature,* 1978, vol. 276, 269
- *J. Immunology,* 1979, vol. 123, 1548
- *Nature,* 1975, vol. 256, 495
- *The Prostane,* 2007, vol. 67, 1163
- *Cytotechnol.,* 1990, vol. 3, 133
- *J. Biochem.,* 1987, vol. 101, 1307
- *Gene,* 1984, vol. 27, 223
- *Proc. Natl. Acad. Sci. USA,* 1981, vol. 78, 1527
- *Cytotechnol.,* 1990, vol. 4, 173
- *Cytotechnol.,* 1993, vol. 13, 79
- *Biochem. Biophys. Res. Commun.,* 1987, vol. 149, 960
- *Cell,* 1985, vol. 41, 479
- *Cell,* 1983, vol. 33, 717
- *J. Immunol. Methods,* 1994, vol. 167, 271
- *Hybridoma,* 1998, vol. 17, 559
- *Methods in Enzymol.,* 1987, vol. 154, 3
- Current Protocols in Molecular Biology. John Wiley & Sons, 1987, vol. 1
- *Strategies,* 1992, vol. 5, 58
- *Nucleic Acids Research,* 1989, vol. 17, 9494
- *DNA Cloning: A Practical Approach,* 1985, vol. I, 49
- *Mol. Cell. Biol.,* 1983, vol. 3, 280
- *Gene,* 1985, vol. 33, 103
- *Strategies,* 1992, vol. 5, 81
- *Genetics,* 1954, vol. 39, 440
- *Science,* 1983, vol. 222, 778
- *J. Mol. Biol.,* 1983, vol. 166, 1
- *J. Mol. Biol.,* 1966, vol. 16, 118
- *Gene,* 1985, vol. 38, 275
- *Proc. Natl. Acad. Sci. USA,* 1977, vol. 74, 5463
- Sequence of Proteins of Immunological Interest. US Dept. Health and Human Services, 1991
- *BIO/TECHNOLOGY,* 1991, vol. 9, 266
- *J. Mol. Biol.,* 1977, vol. 112, 535
- *Protein Engineering,* 1994, vol. 7, 1501
- Methods in Nucleic Acids Res. CRC Press, 1991, 283
- Monoclonal Antibodies-Principles and practice. Academic Press, 1996
- Monoclonal Antibody Experiment Manual. Kodansha Scientific, 1987
- *Proc. Natl. Acad. Sci. U.S.A.,* 1980, vol. 77, 4216
- *Somatic Cell and Molecular genetics,* 1986, vol. 12, 55
- *Nature,* 1970, vol. 227, 680
- Enzyme Immunoassay. Igaku Shoin, 1987
- Manual for Monoclonal Antibody Experiments. Kodansha Scientific, 1987
- **AKIRA KAWAO.** Fluorescent Immunoassay. *Soft Science,* 1983
- **RINSHO KENSA.** Bioluminescence and Chemical Luminescence. Hirokawa Shoten, 1998, vol. 42
- Handbook of Clinical Test Methods. Kanehara Shuppan, 1998
- Manual for Experiments of Monoclonal Antibodies. Kodansha Scientific. 1987
- *The Prostate,* 2007, vol. 67, 1163
- **W. H. FREEMAN.** *Antibody Engineering, A Practical Guide,* 1992
- SEQUENCES of Proteins of Immunological Interest. US Dept. Health and Human Services, 1991
- *Nucleic Acids Res.,* 1997, vol. 25, 3389

SEQUENCE LISTING

<110> KYOWA HAKKO KIRIN CO., LTD.

<120> Anti-Trop-2 antibody

<130> W502515

<150> US61/353430
<151> 2010-06-10

<160> 49

<170> PatentIn Ver. 3.3

<210> 1
<211> 323
<212> PRT
<213> Homo sapiens

<400> 1
```
Met Ala Arg Gly Pro Gly Leu Ala Pro Pro Pro Leu Arg Leu Pro Leu
1               5                   10                  15
Leu Leu Leu Val Leu Ala Ala Val Thr Gly His Thr Ala Ala Gln Asp
            20                  25                  30
Asn Cys Thr Cys Pro Thr Asn Lys Met Thr Val Cys Ser Pro Asp Gly
        35                  40                  45
Pro Gly Gly Arg Cys Gln Cys Arg Ala Leu Gly Ser Gly Met Ala Val
    50                  55                  60
Asp Cys Ser Thr Leu Thr Ser Lys Cys Leu Leu Leu Lys Ala Arg Met
65                  70                  75                  80
Ser Ala Pro Lys Asn Ala Arg Thr Leu Val Arg Pro Ser Glu His Ala
                85                  90                  95
Leu Val Asp Asn Asp Gly Leu Tyr Asp Pro Asp Cys Asp Pro Glu Gly
            100                 105                 110
Arg Phe Lys Ala Arg Gln Cys Asn Gln Thr Ser Val Cys Trp Cys Val
        115                 120                 125
Asn Ser Val Gly Val Arg Arg Thr Asp Lys Gly Asp Leu Ser Leu Arg
    130                 135                 140
Cys Asp Glu Leu Val Arg Thr His His Ile Leu Ile Asp Leu Arg His
145                 150                 155                 160
Arg Pro Thr Ala Gly Ala Phe Asn His Ser Asp Leu Asp Ala Glu Leu
                165                 170                 175
Arg Arg Leu Phe Arg Glu Arg Tyr Arg Leu His Pro Lys Phe Val Ala
            180                 185                 190
Ala Val His Tyr Glu Gln Pro Thr Ile Gln Ile Glu Leu Arg Gln Asn
        195                 200                 205
Thr Ser Gln Lys Ala Ala Gly Asp Val Asp Ile Gly Asp Ala Ala Tyr
    210                 215                 220
Tyr Phe Glu Arg Asp Ile Lys Gly Glu Ser Leu Phe Gln Gly Arg Gly
225                 230                 235                 240
Gly Leu Asp Leu Arg Val Arg Gly Glu Pro Leu Gln Val Glu Arg Thr
                245                 250                 255
Leu Ile Tyr Tyr Leu Asp Glu Ile Pro Pro Lys Phe Ser Met Lys Arg
            260                 265                 270
Leu Thr Ala Gly Leu Ile Ala Val Ile Val Val Val Val Val Ala Leu
        275                 280                 285
Val Ala Gly Met Ala Val Leu Val Ile Thr Asn Arg Arg Lys Ser Gly
    290                 295                 300
Lys Tyr Lys Lys Val Glu Ile Lys Glu Leu Gly Glu Leu Arg Lys Glu
305                 310                 315                 320

Pro Ser Leu
```

50

<210> 2
<211> 972
<212> DNA
<213> Homo sapiens

<220>
<221> CDS
<222> (1)..(972)

<400> 2

```
atg gct cgg ggc ccc ggc ctc gcg ccg cca ccg ctg cgg ctg ccg ctg     48
Met Ala Arg Gly Pro Gly Leu Ala Pro Pro Pro Leu Arg Leu Pro Leu
1               5                   10                  15

ctg ctg ctg gtg ctg gcg gcg gtg acc ggc cac acg gcc gcg cag gac     96
Leu Leu Leu Val Leu Ala Ala Val Thr Gly His Thr Ala Ala Gln Asp
            20                  25                  30

aac tgc acg tgt ccc acc aac aag atg acc gtg tgc agc ccc gac ggc    144
Asn Cys Thr Cys Pro Thr Asn Lys Met Thr Val Cys Ser Pro Asp Gly
        35                  40                  45

ccc ggc ggc cgc tgc cag tgc cgc gcg ctg ggc tcg ggc atg gcg gtc    192
Pro Gly Gly Arg Cys Gln Cys Arg Ala Leu Gly Ser Gly Met Ala Val
    50                  55                  60

gac tgc tcc acg ctg acc tcc aag tgt ctg ctg ctc aag gcg cgc atg    240
Asp Cys Ser Thr Leu Thr Ser Lys Cys Leu Leu Leu Lys Ala Arg Met
65                  70                  75                  80

agc gcc ccc aag aac gcc cgc acg ctg gtg cgg ccg agt gag cac gcg    288
Ser Ala Pro Lys Asn Ala Arg Thr Leu Val Arg Pro Ser Glu His Ala
                85                  90                  95

ctc gtg gac aac gat ggc ctc tac gac ccc gac tgc gac ccc gag ggc    336
Leu Val Asp Asn Asp Gly Leu Tyr Asp Pro Asp Cys Asp Pro Glu Gly
            100                 105                 110

cgc ttc aag gcg cgc cag tgc aac cag acg tcg gtg tgc tgg tgc gtg    384
Arg Phe Lys Ala Arg Gln Cys Asn Gln Thr Ser Val Cys Trp Cys Val
        115                 120                 125

aac tcg gtg ggc gtg cgc cgc acg gac aag ggc gac ctg agc cta cgc    432
Asn Ser Val Gly Val Arg Arg Thr Asp Lys Gly Asp Leu Ser Leu Arg
    130                 135                 140

tgc gat gag ctg gtg cgc acc cac cac atc ctc att gac ctg cgc cac    480
Cys Asp Glu Leu Val Arg Thr His His Ile Leu Ile Asp Leu Arg His
145                 150                 155                 160

cgc ccc acc gcc ggc gcc ttc aac cac tca gac ctg gac gcc gag ctg    528
Arg Pro Thr Ala Gly Ala Phe Asn His Ser Asp Leu Asp Ala Glu Leu
                165                 170                 175

agg cgg ctc ttc cgc gag cgc tat cgg ctg cac ccc aag ttc gtg gcg    576
Arg Arg Leu Phe Arg Glu Arg Tyr Arg Leu His Pro Lys Phe Val Ala
            180                 185                 190

gcc gtg cac tac gag cag ccc acc atc cag atc gag ctg cgg cag aac    624
Ala Val His Tyr Glu Gln Pro Thr Ile Gln Ile Glu Leu Arg Gln Asn
        195                 200                 205
```

```
acg tct cag aag gcc gcc ggt gac gtg gat atc ggc gat gcc gcc tac      672
Thr Ser Gln Lys Ala Ala Gly Asp Val Asp Ile Gly Asp Ala Ala Tyr
    210             215             220

tac ttc gag agg gac atc aag ggc gag tct cta ttc cag ggc cgc ggc      720
Tyr Phe Glu Arg Asp Ile Lys Gly Glu Ser Leu Phe Gln Gly Arg Gly
225             230             235             240

ggc ctg gac ttg cgc gtg cgc gga gaa ccc ctg cag gtg gag cgc acg      768
Gly Leu Asp Leu Arg Val Arg Gly Glu Pro Leu Gln Val Glu Arg Thr
        245             250             255

ctc atc tat tac ctg gac gag att ccc ccg aag ttc tcc atg aag cgc      816
Leu Ile Tyr Tyr Leu Asp Glu Ile Pro Pro Lys Phe Ser Met Lys Arg
        260             265             270

ctc acc gcc ggc ctc atc gcc gtc atc gtg gtg gtc gtg gtg gcc ctc      864
Leu Thr Ala Gly Leu Ile Ala Val Ile Val Val Val Val Val Ala Leu
        275             280             285

gtc gcc ggc atg gcc gtc ctg gtg atc acc aac cgg aga aag tcg ggg      912
Val Ala Gly Met Ala Val Leu Val Ile Thr Asn Arg Arg Lys Ser Gly
        290             295             300

aag tac aag aag gtg gag atc aag gaa ctg ggg gag ttg aga aag gaa      960
Lys Tyr Lys Lys Val Glu Ile Lys Glu Leu Gly Glu Leu Arg Lys Glu
305             310             315             320

ccg agc ttg tag                                                       972
Pro Ser Leu

<210> 3
<211> 476
<212> DNA
<213> Mus musculus

<220>
<221> CDS
<222> (1)..(476)

<400> 3
aaggaaaaaa gcggccgctg aacacactga ctctaacc atg aga gtg ctg att ctt     56
                                            Met Arg Val Leu Ile Leu
                                            1               5

ttg tgg ctg ttc aca gcc ttt cct ggt atc ctg tct cag atc cag ttg      104
Leu Trp Leu Phe Thr Ala Phe Pro Gly Ile Leu Ser Gln Ile Gln Leu
            10              15              20

gtg cag tct gga cct gag ctg aag aag cct gga gag aca gtc aag atc      152
Val Gln Ser Gly Pro Glu Leu Lys Lys Pro Gly Glu Thr Val Lys Ile
        25              30              35

tcc tgc aag gct tct ggg tat acc ttc aca aac tat gga atg aac tgg      200
Ser Cys Lys Ala Ser Gly Tyr Thr Phe Thr Asn Tyr Gly Met Asn Trp
        40              45              50

gtg aag cag gct cca gga aag ggt tta aag tgg atg ggc tgg ata aac      248
Val Lys Gln Ala Pro Gly Lys Gly Leu Lys Trp Met Gly Trp Ile Asn
 55                 60              65                  70

acc aaa act gga gag cca aca tat gct gaa gag ttc aag gga cgg ttt      296
Thr Lys Thr Gly Glu Pro Thr Tyr Ala Glu Glu Phe Lys Gly Arg Phe
```

```
                    75                      80                      85
gcc ttc tct ttg gaa acc tct gcc agc act gcc tat ttg cag atc aac      344
Ala Phe Ser Leu Glu Thr Ser Ala Ser Thr Ala Tyr Leu Gln Ile Asn
        90                      95                     100

aac ctc aaa aaa gag gac acg gct aca tat ttc tgt gga aga ggg ggc      392
Asn Leu Lys Lys Glu Asp Thr Ala Thr Tyr Phe Cys Gly Arg Gly Gly
        105                     110                     115

tac ggt agt agc tac tgg tac ttc gat gtc tgg ggc gca ggg acc acg      440
Tyr Gly Ser Ser Tyr Trp Tyr Phe Asp Val Trp Gly Ala Gly Thr Thr
        120                     125                     130

gtc acc gtc tcc tca gcc tcc acc aag ggc cca tcg                      476
Val Thr Val Ser Ser Ala Ser Thr Lys Gly Pro Ser
135                     140                     145


<210> 4
<211> 428
<212> DNA
<213> Mus musculus

<220>
<221> CDS
<222> (1)..(428)

<400> 4
ccggaattca gacaggcagg ggaagcaag atg cat ttt caa gtg cag att ttc       53
                                Met His Phe Gln Val Gln Ile Phe
                                 1               5

agc ttc ctg cta atc agt gcc tca gtc ata atg tcc aga gga gac att      101
Ser Phe Leu Leu Ile Ser Ala Ser Val Ile Met Ser Arg Gly Asp Ile
        10                      15                      20

gtg atg acc cag tct cac aaa ttc atg tcc aca tca gta gga gac agg      149
Val Met Thr Gln Ser His Lys Phe Met Ser Thr Ser Val Gly Asp Arg
 25                      30                      35              40

gtc agc atc acc tgc aag gcc agt cag gat gtg agt att gct gta gcc      197
Val Ser Ile Thr Cys Lys Ala Ser Gln Asp Val Ser Ile Ala Val Ala
                45                      50                      55

tgg tat caa cag aaa cca gga caa tct cct aaa gtg ctg att tac tcg      245
Trp Tyr Gln Gln Lys Pro Gly Gln Ser Pro Lys Val Leu Ile Tyr Ser
            60                      65                      70

gca tcc tac cgg tac act gga gtc cct gat cgc ttc act ggc agt gga      293
Ala Ser Tyr Arg Tyr Thr Gly Val Pro Asp Arg Phe Thr Gly Ser Gly
        75                      80                      85

tct ggg acg gat ttc act ttc acc atc agc agg gtg cag gct gaa gac      341
Ser Gly Thr Asp Phe Thr Phe Thr Ile Ser Arg Val Gln Ala Glu Asp
        90                      95                     100

ctg gca gtt tat tac tgt cag caa cat tat att act ccg ctc acg ttc      389
Leu Ala Val Tyr Tyr Cys Gln Gln His Tyr Ile Thr Pro Leu Thr Phe
105                     110                     115                 120

ggt gct ggg acc aag ctg gag ctg aaa cgt acg gtg gct                  428
Gly Ala Gly Thr Lys Leu Glu Leu Lys Arg Thr Val Ala
            125                     130
```

53

```
<210> 5
<211> 30
<212> DNA
<213> Artificial Sequence

<220>
<223> Description of Artificial Sequence: synthetic DNA

<400> 5
ctgcccaaac taactccatg gtgaccctgg                                    30

<210> 6
<211> 30
<212> DNA
<213> Artificial Sequence

<220>
<223> Description of Artificial Sequence: synthetic DNA

<400> 6
gatctgctgc ccaaactaac tccatggtga                                    30

<210> 7
<211> 32
<212> DNA
<213> Artificial Sequence

<220>
<223> Description of Artificial Sequence: synthetic DNA

<400> 7
ttgtcaagag cttcaacagg aatgagtgtt ag                                 32

<210> 8
<211> 30
<212> DNA
<213> Artificial Sequence

<220>
<223> Description of Artificial Sequence: synthetic DNA

<400> 8
acatctggag gtgcctcagt cgtgtgcttc                                    30

<210> 9
<211> 405
<212> DNA
<213> Mus musculus

<220>
<221> CDS
<222> (1)..(405)

<400> 9
atg gaa tgg agc ggg gtc ttt atc ttt ctc ttg tca gta act gca gat      48
Met Glu Trp Ser Gly Val Phe Ile Phe Leu Leu Ser Val Thr Ala Asp
  1               5                  10                  15

gtc cac tcc cag gtc cag ttg cag cag tct gga cct gag ctg gta agg      96
Val His Ser Gln Val Gln Leu Gln Gln Ser Gly Pro Glu Leu Val Arg
              20                  25                  30
```

```
cct ggg act tca gtg agg ata tcc tgc aag gct tct ggc tac acc ttc      144
Pro Gly Thr Ser Val Arg Ile Ser Cys Lys Ala Ser Gly Tyr Thr Phe
        35                  40                  45

act att tac tgg cta ggt tgg gta aag cag aga cct gga cat gga ctt      192
Thr Ile Tyr Trp Leu Gly Trp Val Lys Gln Arg Pro Gly His Gly Leu
        50                  55                  60

gaa tgg att gga aat att ttc cct gga agt gct tac att aac tac aat      240
Glu Trp Ile Gly Asn Ile Phe Pro Gly Ser Ala Tyr Ile Asn Tyr Asn
    65                  70                  75                  80

gag aag ttc aag ggc aag gcc aca ctg act gca gac aca tcc tcc agc      288
Glu Lys Phe Lys Gly Lys Ala Thr Leu Thr Ala Asp Thr Ser Ser Ser
                85                  90                  95

act gcc tat atg cag ctc agt agc ctg aca tct gag gac tct gct gtc      336
Thr Ala Tyr Met Gln Leu Ser Ser Leu Thr Ser Glu Asp Ser Ala Val
        100                 105                 110

tat ttc tgt gca aga gag ggt agt aat tcc ggc tac tgg ggt caa ggc      384
Tyr Phe Cys Ala Arg Glu Gly Ser Asn Ser Gly Tyr Trp Gly Gln Gly
        115                 120                 125

acc act ctc aca gtc tcc tca                                          405
Thr Thr Leu Thr Val Ser Ser
        130                 135


<210> 10
<211> 135
<212> PRT
<213> Mus musculus

<400> 10
Met Glu Trp Ser Gly Val Phe Ile Phe Leu Leu Ser Val Thr Ala Asp
 1               5                   10                  15
Val His Ser Gln Val Gln Leu Gln Gln Ser Gly Pro Glu Leu Val Arg
            20                  25                  30
Pro Gly Thr Ser Val Arg Ile Ser Cys Lys Ala Ser Gly Tyr Thr Phe
        35                  40                  45
Thr Ile Tyr Trp Leu Gly Trp Val Lys Gln Arg Pro Gly His Gly Leu
    50                  55                  60
Glu Trp Ile Gly Asn Ile Phe Pro Gly Ser Ala Tyr Ile Asn Tyr Asn
65                  70                  75                  80
Glu Lys Phe Lys Gly Lys Ala Thr Leu Thr Ala Asp Thr Ser Ser Ser
                85                  90                  95
Thr Ala Tyr Met Gln Leu Ser Ser Leu Thr Ser Glu Asp Ser Ala Val
        100                 105                 110
Tyr Phe Cys Ala Arg Glu Gly Ser Asn Ser Gly Tyr Trp Gly Gln Gly
        115                 120                 125
Thr Thr Leu Thr Val Ser Ser
        130                 135


<210> 11
<211> 399
<212> DNA
<213> Mus musculus

<220>
<221> CDS
<222> (1)..(399)

<400> 11
```

55

```
atg gaa tca cag act cag gtc ctc atc tcc ttg ctg ttc tgg gta tct          48
Met Glu Ser Gln Thr Gln Val Leu Ile Ser Leu Leu Phe Trp Val Ser
  1               5                  10                  15

ggt acc tgt ggg gac att gtg atg aca cag tct cca tcc tcc ctg agt          96
Gly Thr Cys Gly Asp Ile Val Met Thr Gln Ser Pro Ser Ser Leu Ser
                20                  25                  30

gtg tca gca gga gag aag gtc act atg acc tgc aag tcc agt cag agt         144
Val Ser Ala Gly Glu Lys Val Thr Met Thr Cys Lys Ser Ser Gln Ser
        35                  40                  45

ctg tta aac agt gga aat caa cag aac tac ttg gcc tgg tac cag cag         192
Leu Leu Asn Ser Gly Asn Gln Gln Asn Tyr Leu Ala Trp Tyr Gln Gln
        50                  55                  60

aaa cca ggg cag cct cct aaa ctg ttg atc tac ggg gca tcc act agg         240
Lys Pro Gly Gln Pro Pro Lys Leu Leu Ile Tyr Gly Ala Ser Thr Arg
 65                  70                  75                  80

gag tct ggg gtc cct gat cgc ttc aca ggc agt gga tct gga acc gat         288
Glu Ser Gly Val Pro Asp Arg Phe Thr Gly Ser Gly Ser Gly Thr Asp
                85                  90                  95

ttc act ctc acc atc aac agt gtg cag gct gaa gac ctg gca gtt tat         336
Phe Thr Leu Thr Ile Asn Ser Val Gln Ala Glu Asp Leu Ala Val Tyr
               100                 105                 110

tac tgt cag agt gat cat att tat ccg tac acg ttc gga ggg ggg acc         384
Tyr Cys Gln Ser Asp His Ile Tyr Pro Tyr Thr Phe Gly Gly Gly Thr
           115                 120                 125

aag ctg gaa ata aaa                                                      399
Lys Leu Glu Ile Lys
       130
```

```
<210> 12
<211> 133
<212> PRT
<213> Mus musculus

<400> 12
Met Glu Ser Gln Thr Gln Val Leu Ile Ser Leu Leu Phe Trp Val Ser
  1               5                  10                  15
Gly Thr Cys Gly Asp Ile Val Met Thr Gln Ser Pro Ser Ser Leu Ser
                20                  25                  30
Val Ser Ala Gly Glu Lys Val Thr Met Thr Cys Lys Ser Ser Gln Ser
        35                  40                  45
Leu Leu Asn Ser Gly Asn Gln Gln Asn Tyr Leu Ala Trp Tyr Gln Gln
        50                  55                  60
Lys Pro Gly Gln Pro Pro Lys Leu Leu Ile Tyr Gly Ala Ser Thr Arg
 65                  70                  75                  80
Glu Ser Gly Val Pro Asp Arg Phe Thr Gly Ser Gly Ser Gly Thr Asp
                85                  90                  95
Phe Thr Leu Thr Ile Asn Ser Val Gln Ala Glu Asp Leu Ala Val Tyr
               100                 105                 110
Tyr Cys Gln Ser Asp His Ile Tyr Pro Tyr Thr Phe Gly Gly Gly Thr
           115                 120                 125
Lys Leu Glu Ile Lys
       130

<210> 13
<211> 5
```

<212> PRT
<213> Mus musculus

<400> 13
Ile Tyr Trp Leu Gly
1               5

<210> 14
<211> 17
<212> PRT
<213> Mus musculus

<400> 14
Asn Ile Phe Pro Gly Ser Ala Tyr Ile Asn Tyr Asn Glu Lys Phe Lys
1               5               10              15
Gly

<210> 15
<211> 7
<212> PRT
<213> Mus musculus

<400> 15
Glu Gly Ser Asn Ser Gly Tyr
1               5

<210> 16
<211> 17
<212> PRT
<213> Mus musculus

<400> 16
Lys Ser Ser Gln Ser Leu Leu Asn Ser Gly Asn Gln Gln Asn Tyr Leu
1               5               10              15
Ala

<210> 17
<211> 7
<212> PRT
<213> Mus musculus

<400> 17
Gly Ala Ser Thr Arg Glu Ser
1               5

<210> 18
<211> 9
<212> PRT
<213> Mus musculus

<400> 18
Gln Ser Asp His Ile Tyr Pro Tyr Thr
1               5

<210> 19
<211> 39
<212> DNA
<213> Artificial Sequence

<220>
<223> Description of Artificial Sequence: synthetic DNA

<400> 19

57

EP 2 594 589 A1

aaggaaaaaa gcggccgcac catggaatgg agcggggtc                                      39

<210> 20
<211> 43
<212> DNA
<213> Artificial Sequence

<220>
<223> Description of Artificial Sequence: synthetic DNA

<400> 20
cgatgggccc ttggtggagg ctgaggagac tgtgagagtg gtg                                 43

<210> 21
<211> 35
<212> DNA
<213> Artificial Sequence

<220>
<223> Description of Artificial Sequence: synthetic DNA

<400> 21
ccggaattcc aagatggaat cacagactca ggtcc                                          35

<210> 22
<211> 31
<212> DNA
<213> Artificial Sequence

<220>
<223> Description of Artificial Sequence: synthetic DNA

<400> 22
agccaccgta cgttttattt ccagcttggt c                                              31

<210> 23
<211> 348
<212> DNA
<213> Mus musculus

<220>
<221> CDS
<222> (1)..(348)

<400> 23
```
cag gtc cag ttg gtg cag tct ggc gct gag gtg aag aag cct ggg gct           48
Gln Val Gln Leu Val Gln Ser Gly Ala Glu Val Lys Lys Pro Gly Ala
1               5                   10                  15

tca gtg aag gtg tcc tgc aag gct tct ggc tac acc ttc act att tac           96
Ser Val Lys Val Ser Cys Lys Ala Ser Gly Tyr Thr Phe Thr Ile Tyr
            20                  25                  30


tgg ctg ggt tgg gta cgg cag gcc cct ggt caa ggg ctt gaa tgg atg          144
Trp Leu Gly Trp Val Arg Gln Ala Pro Gly Gln Gly Leu Glu Trp Met
        35                  40                  45

ggc aat att ttc cct ggc agt gct tac att aac tac aat gag aag ttc          192
Gly Asn Ile Phe Pro Gly Ser Ala Tyr Ile Asn Tyr Asn Glu Lys Phe
        50                  55                  60

aag ggc cgg gtc aca atc act gca gac aca tcc acc agc act gcc tat          240
```

58

```
Lys Gly Arg Val Thr Ile Thr Ala Asp Thr Ser Thr Ser Thr Ala Tyr
65                  70              75                  80


atg gaa ctc agt agc ctg cga tct gag gac act gct gtc tat tac tgt      288

Met Glu Leu Ser Ser Leu Arg Ser Glu Asp Thr Ala Val Tyr Tyr Cys
                    85              90                  95

gca cga gag ggt agt aat tcc ggc tac tgg ggt caa ggc acc ctg gtc      336
Ala Arg Glu Gly Ser Asn Ser Gly Tyr Trp Gly Gln Gly Thr Leu Val
                100              105             110

aca gtc tcc tca                                                      348

Thr Val Ser Ser
            115
```

```
<210> 24
<211> 116
<212> PRT
<213> Mus musculus

<400> 24
Gln Val Gln Leu Val Gln Ser Gly Ala Glu Val Lys Lys Pro Gly Ala
1               5               10                  15
Ser Val Lys Val Ser Cys Lys Ala Ser Gly Tyr Thr Phe Thr Ile Tyr
            20                  25                  30
Trp Leu Gly Trp Val Arg Gln Ala Pro Gly Gln Gly Leu Glu Trp Met
        35                  40                  45
Gly Asn Ile Phe Pro Gly Ser Ala Tyr Ile Ans Tyr Asn Glu Lys Phe
        50                  55                  60
Lys Gly Arg Val Thr Ile Thr Ala Asp Thr Ser Thr Ser Thr Ala Tyr
65                  70              75                  80
Met Glu Leu Ser Ser Leu Arg Ser Glu Asp Thr Ala Val Tyr Tyr Cys
                    85              90                  95
Ala Arg Glu Gly Ser Asn Ser Gly Tyr Trp Gly Gln Gly Thr Leu Vla
                100              105             110
Thr Val Ser Ser
            116
```

```
<210> 25
<211> 339
<212> DNA
<213> Mus musculus

<220>
<221> CDS
<222> (1)..(339)

<400> 25
gac att gtg atg aca cag tct cca gac tcc ctg gct gtg tca ctg ggt      48
Asp Ile Val Met Thr Gln Ser Pro Asp Ser Leu Ala Val Ser Leu Gly
1               5               10                  15

gag cga gcc act atc aac tgc aag tcc agt cag agt ctg tta aac agt      96

Glu Arg Ala Thr Ile Asn Cys Lys Ser Ser Gln Ser Leu Leu Asn Ser
            20                  25                  30

ggc aat caa cag aac tac ttg gcc tgg tac cag cag aaa cca ggg cag      144
Gly Asn Gln Gln Asn Tyr Leu Ala Trp Tyr Gln Gln Lys Pro Gly Gln
            35                  40                  45
```

```
cct cct aaa ctg ttg atc tac ggg gca tcc act cgg gag tct ggg gtc     192
Pro Pro Lys Leu Leu Ile Tyr Gly Ala Ser Thr Arg Glu Ser Gly Val
    50                  55                  60

cct gat cgc ttc tca ggc agt ggc tct ggt acc gat ttc act ctc acc     240
Pro Asp Arg Phe Ser Gly Ser Gly Ser Gly Thr Asp Phe Thr Leu Thr
65                  70                  75                  80

atc tca agt ttg cag gct gaa gac gtg gca gtt tat tac tgt cag agt     288
Ile Ser Ser Leu Gln Ala Glu Asp Val Ala Val Tyr Tyr Cys Gln Ser
                85                  90                  95

gat cat att tat ccg tac acg ttc ggt caa ggg acc aag ctg gaa ata     336
Asp His Ile Tyr Pro Tyr Thr Phe Gly Gln Gly Thr Lys Leu Glu Ile
                100                 105                 110

aaa                                                                  339
Lys


<210> 26
<211> 113
<212> PRT
<213> Mus musculus

<400> 26
Asp Ile Val Met Thr Gln Ser Pro Asp Ser Leu Ala Val Ser Leu Gly
1               5                   10                  15
Glu Arg Ala Thr Ile Asn Cys Lys Ser Ser Gln Ser Leu Leu Asn Ser
                20                  25                  30
Gly Asn Gln Gln Asn Tyr Leu Ala Trp Tyr Gln Gln Lys Pro Gly Gln
        35                  40                  45
Pro Pro Lys Leu Leu Ile Tyr Gly Ala Ser Thr Arg Glu Ser Gly Val
        50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Val Tyr Tyr Cys Gln Ser
                85                  90                  95
Asp His Ile Tyr Pro Tyr Thr Phe Gly Gln Gly Thr Lys Leu Glu Ile
                100                 105                 110
Lys
113


<210> 27
<211> 348
<212> DNA
<213> Artificial Sequence

<220>
<221> CDS
<220> (1)..(348)
<223> Description of Artificial Sequence: HV3a sequence

<400> 27
cag gtc cag ttg gtg cag tct gga cct gag gtg aag aag cct ggg gct      48
Gln Val Gln Leu Val Gln Ser Gly Pro Glu Val Lys Lys Pro Gly Ala
 1               5                   10                  15

tca gtg aag gta tcc tgc aag gct tct ggc tac acc ttc act att tac      96
Ser Val Lys Val Ser Cys Lys Ala Ser Gly Tyr Thr Phe Thr Ile Tyr
                20                  25                  30

tgg cta ggt tgg gta cgg cag gca cct gga cag gga ctt gaa tgg atg     144
Trp Leu Gly Trp Val Arg Gln Ala Pro Gly Gln Gly Leu Glu Trp Met
```

```
                35                      40                      45

gga aat att ttc cct gga agt gct tac att aac tac aat gag aag ttc        192
Gly Asn Ile Phe Pro Gly Ser Ala Tyr Ile Asn Tyr Asn Glu Lys Phe
        50                      55                      60

aag ggc aag gtc aca atc act gca gac aca tcc acc agc act gcc tat        240
Lys Gly Lys Val Thr Ile Thr Ala Asp Thr Ser Thr Ser Thr Ala Tyr
65                      70                      75                      80

atg gag ctc agt agc ctg cga tct gag gac act gct gtc tat ttt tgt        288
Met Glu Leu Ser Ser Leu Arg Ser Glu Asp Thr Ala Val Tyr Phe Cys
                        85                      90                      95

gca aga gag ggt agt aat tcc ggc tac tgg ggt caa ggc acc ctt gtc        336
Ala Arg Glu Gly Ser Asn Ser Gly Tyr Trp Gly Gln Gly Thr Leu Val
            100                     105                     110

aca gtc tcc tca                                                        348
Thr Val Ser Ser
            115
```

<210> 28
<211> 116
<212> PRT
<213> Artificial Sequence

<220>
<221> PEPTIDE
<220> (1)..(116)
<223> Description of Artificial Sequence: HV3a sequence

<400> 28

```
Gln Val Gln Leu Val Gln Ser Gly Pro Glu Val Lys Lys Pro Gly Ala
1               5                   10                  15
Ser Val Lys Val Ser Cys Lys Ala Ser Gly Tyr Thr Phe Thr Ile Tyr
                20                  25                  30
Trp Leu Gly Trp Val Arg Gln Ala Pro Gly Gln Gly Leu Glu Trp Met
            35                  40                  45
Gly Asn Ile Phe Pro Gly Ser Ala Tyr Ile Asn Tyr Asn Glu Lys Phe
        50                  55                  60
Lys Gly Lys Val Thr Ile Thr Ala Asp Thr Ser Thr Ser Thr Ala Tyr
65                  70                  75                  80
Met Glu Leu Ser Ser Leu Arg Ser Glu Asp Thr Ala Val Tyr Phe Cys
                85                  90                  95
Ala Arg Glu Gly Ser Asn Ser Gly Tyr Trp Gly Gln Gly Thr Leu Val
            100                 105                 110
Thr Val Ser Ser
            115
```

<210> 29
<211> 348
<212> DNA
<213> Artificial Sequence

<220>
<221> CDS
<220> (1)..(348)
<223> Description of Artificial Sequence: HV4 sequence

<400> 29

```
cag gtc cag ttg gtg cag tct gga cct gag gtg aag aag cct ggg gct         48
Gln Val Gln Leu Val Gln Ser Gly Pro Glu Val Lys Lys Pro Gly Ala
```

```
              1                    5                   10                  15

     tca gtg aag gta tcc tgc aag gct tct ggc tac acc ttc act att tac          96
     Ser Val Lys Val Ser Cys Lys Ala Ser Gly Tyr Thr Phe Thr Ile Tyr
              20                  25                  30

     tgg cta ggt tgg gta aag cag gca cct gga cag gga ctt gaa tgg att         144
     Trp Leu Gly Trp Val Lys Gln Ala Pro Gly Gln Gly Leu Glu Trp Ile
              35                  40                  45

     gga aat att ttc cct gga agt gct tac att aac tac aat gag aag ttc         192
     Gly Asn Ile Phe Pro Gly Ser Ala Tyr Ile Asn Tyr Asn Glu Lys Phe
              50                  55                  60

     aag ggc cgg gtc aca atc act gca gac aca tcc acc agc act gcc tat         240
     Lys Gly Arg Val Thr Ile Thr Ala Asp Thr Ser Thr Ser Thr Ala Tyr
              65                  70                  75                  80

     atg gag ctc agt agc ctg cga tct gag gac act gct gtc tat ttt tgt         288
     Met Glu Leu Ser Ser Leu Arg Ser Glu Asp Thr Ala Val Tyr Phe Cys
                          85                  90                  95

     gca aga gag ggt agt aat tcc ggc tac tgg ggt caa ggc acc ctt gtc         336
     Ala Arg Glu Gly Ser Asn Ser Gly Tyr Trp Gly Gln Gly Thr Leu Val
                      100                 105                 110

     aca gtc tcc tca                                                          348
     Thr Val Ser Ser
                  115
```

<210> 30
<211> 116
<212> PRT
<213> Artificial Sequence

<220>
<221> PEPTIDE
<220> (1)..(116)
<223> Description of Artificial Sequence: HV4 sequence

<400> 30
```
Gln Val Gln Leu Val Gln Ser Gly Pro Glu Val Lys Lys Pro Gly Ala
  1               5                   10                  15
Ser Val Lys Val Ser Cys Lys Ala Ser Gly Tyr Thr Phe Thr Ile Tyr
              20                  25                  30
Trp Leu Gly Trp Val Lys Gln Ala Pro Gly Gln Gly Leu Glu Trp Ile
          35                  40                  45
Gly Asn Ile Phe Pro Gly Ser Ala Tyr Ile Asn Tyr Asn Glu Lys Phe
          50                  55                  60
Lys Gly Arg Val Thr Ile Thr Ala Asp Thr Ser Thr Ser Thr Ala Tyr
  65                  70                  75                  80
Met Glu Leu Ser Ser Leu Arg Ser Glu Asp Thr Ala Val Tyr Phe Cys
                  85                  90                  95
Ala Arg Glu Gly Ser Asn Ser Gly Tyr Trp Gly Gln Gly Thr Leu Val
              100                 105                 110
Thr Val Ser Ser
              115
```

<210> 31
<211> 348
<212> DNA
<213> Artificial Sequence

<220>
<221> CDS
<220> (1)..(348)
<223> Description of Artificial Sequence: HV5 sequence

<400> 31

```
cag gtc cag ttg gtg cag tct gga gct gag gtg aag aag cct ggg gct        48
Gln Val Gln Leu Val Gln Ser Gly Ala Glu Val Lys Lys Pro Gly Ala
1               5                   10                  15

tca gtg aag gta tcc tgc aag gct tct ggc tac acc ttc act att tac        96
Ser Val Lys Val Ser Cys Lys Ala Ser Gly Tyr Thr Phe Thr Ile Tyr
            20                  25                  30

tgg cta ggt tgg gta aag cag gca cct gga cag gga ctt gaa tgg att       144
Trp Leu Gly Trp Val Lys Gln Ala Pro Gly Gln Gly Leu Glu Trp Ile
        35                  40                  45

gga aat att ttc cct gga agt gct tac att aac tac aat gag aag ttc       192
Gly Asn Ile Phe Pro Gly Ser Ala Tyr Ile Asn Tyr Asn Glu Lys Phe
    50                  55                  60

aag ggc aag gcc aca atc act gca gac aca tcc acc agc act gcc tat       240
Lys Gly Lys Ala Thr Ile Thr Ala Asp Thr Ser Thr Ser Thr Ala Tyr
65                  70                  75                  80

atg gag ctc agt agc ctg cga tct gag gac act gct gtc tat ttt tgt       288
Met Glu Leu Ser Ser Leu Arg Ser Glu Asp Thr Ala Val Tyr Phe Cys
                85                  90                  95

gca aga gag ggt agt aat tcc ggc tac tgg ggt caa ggc acc ctt gtc       336
Ala Arg Glu Gly Ser Asn Ser Gly Tyr Trp Gly Gln Gly Thr Leu Val
                100                 105                 110

aca gtc tcc tca                                                       348
Thr Val Ser Ser
            115
```

<210> 32
<211> 116
<212> PRT
<213> Artificial Sequence

<220>
<221> PEPTIDE
<220> (1)..(116)
<223> Description of Artificial Sequence: HV5 sequence

<400> 32

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ile Tyr
            20                  25                  30
Trp Leu Gly Trp Val Lys Gln Ala Pro Gly Gln Gly Leu Glu Trp Ile
        35                  40                  45
Gly Asn Ile Phe Pro Gly Ser Ala Tyr Ile Asn Tyr Asn Glu Lys Phe
    50                  55                  60
Lys Gly Lys Ala Thr Ile Thr Ala Asp Thr Ser Thr Ser Thr Ala Tyr
65                  70                  75                  80
Met Glu Leu Ser Ser Leu Arg Ser Glu Asp Thr Ala Val Tyr Phe Cys
                85                  90                  95
Ala Arg Glu Gly Ser Asn Ser Gly Tyr Trp Gly Gln Gly Thr Leu Val
                100                 105                 110
```

Thr Val Ser Ser
        115


<210> 33
<211> 348
<212> DNA
<213> Artificial Sequence


<220>
<221> CDS
<220> (1)..(348)
<223> Description of Artificial Sequence: HV10 sequence


<400> 33
cag gtc cag ttg gtg cag tct gga cct gag gtg gta aag cct ggg gct     48
Gln Val Gln Leu Val Gln Ser Gly Pro Glu Val Val Lys Pro Gly Ala
1               5                   10                  15

tca gtg aag ata tcc tgc aag gct tct ggc tac acc ttc act att tac     96
Ser Val Lys Ile Ser Cys Lys Ala Ser Gly Tyr Thr Phe Thr Ile Tyr
            20                  25                  30

tgg cta ggt tgg gta aag cag gca cct gga cag gga ctt gaa tgg att    144
Trp Leu Gly Trp Val Lys Gln Ala Pro Gly Gln Gly Leu Glu Trp Ile
        35                  40                  45

gga aat att ttc cct gga agt gct tac att aac tac aat gag aag ttc    192
Gly Asn Ile Phe Pro Gly Ser Ala Tyr Ile Asn Tyr Asn Glu Lys Phe
    50                  55                  60

aag ggc aag gcc aca ctg act gca gac aca tcc acc agc act gcc tat    240
Lys Gly Lys Ala Thr Leu Thr Ala Asp Thr Ser Thr Ser Thr Ala Tyr
65                  70                  75                  80

atg gag ctc agt agc ctg cga tct gag gac act gct gtc tat ttt tgt    288
Met Glu Leu Ser Ser Leu Arg Ser Glu Asp Thr Ala Val Tyr Phe Cys
                85                  90                  95

gca aga gag ggt agt aat tcc ggc tac tgg ggt caa ggc acc ctt ctc    336
Ala Arg Glu Gly Ser Asn Ser Gly Tyr Trp Gly Gln Gly Thr Leu Leu
            100                 105                 110

aca gtc tcc tca                                                     348
Thr Val Ser Ser
        115


<210> 34
<211> 116
<212> PRT
<213> Artificial Sequence


<220>
<221> PEPTIDE
<220> (1)..(116)
<223> Description of Artificial Sequence: HV10 sequence


<400> 34
Gln Val Gln Leu Val Gln Ser Gly Pro Glu Val Val Lys Pro Gly Ala
1               5                   10                  15
Ser Val Lys Ile Ser Cys Lys Ala Ser Gly Tyr Thr Phe Thr Ile Tyr
            20                  25                  30
Trp Leu Gly Trp Val Lys Gln Ala Pro Gly Gln Gly Leu Glu Trp Ile
        35                  40                  45

```
Gly Asn Ile Phe Pro Gly Ser Ala Tyr Ile Asn Tyr Asn Glu Lys Phe
     50              55              60
Lys Gly Lys Ala Thr Leu Thr Ala Asp Thr Ser Thr Ser Thr Ala Tyr
 65              70              75              80
Met Glu Leu Ser Ser Leu Arg Ser Glu Asp Thr Ala Val Tyr Phe Cys
             85              90              95
Ala Arg Glu Gly Ser Asn Ser Gly Tyr Trp Gly Gln Gly Thr Leu Leu
         100             105             110
Thr Val Ser Ser
         115
```

```
<210> 35
<211> 339
<212> DNA
<213> Artificial Sequence

<220>
<221> CDS
<220> (1)..(339)
<223> Description of Artificial Sequence: LV2 sequence

<400> 35
gac att gtg atg aca cag tct cca gac tcc ctg gct gtg tca tta gga      48
Asp Ile Val Met Thr Gln Ser Pro Asp Ser Leu Ala Val Ser Leu Gly
 1               5                   10                  15

gag cgg gtc act atc aac tgc aag tcc agt cag agt ctg tta aac agt      96
Glu Arg Val Thr Ile Asn Cys Lys Ser Ser Gln Ser Leu Leu Asn Ser
             20                  25                  30

gga aat caa cag aac tac ttg gcc tgg tac cag cag aaa cca ggg cag     144
Gly Asn Gln Gln Asn Tyr Leu Ala Trp Tyr Gln Gln Lys Pro Gly Gln
         35                  40                  45

cct cct aaa ctg ttg atc tac ggg gca tcc act agg gag tct ggg gtc     192
Pro Pro Lys Leu Leu Ile Tyr Gly Ala Ser Thr Arg Glu Ser Gly Val
     50                  55                  60

cct gat cgc ttc tca ggc agt gga tct gga acc gat ttc act ctc acc     240
Pro Asp Arg Phe Ser Gly Ser Gly Ser Gly Thr Asp Phe Thr Leu Thr
 65                  70                  75                  80

atc agc agt gtg cag gct gaa gac gtg gca gtt tat tac tgt cag agt     288
Ile Ser Ser Val Gln Ala Glu Asp Val Ala Val Tyr Tyr Cys Gln Ser
                 85                  90                  95

gat cat att tat ccg tac acg ttc gga cag ggg acc aag ctg gaa ata     336
Asp His Ile Tyr Pro Tyr Thr Phe Gly Gln Gly Thr Lys Leu Glu Ile
             100                 105                 110

aaa                                                                 339
Lys
```

```
<210> 36
<211> 113
<212> PRT
<213> Artificial Sequence

<220>
<221> PEPTIDE
<220> (1)..(113)
<223> Description of Artificial Sequence: LV2 sequence
```

<400> 36

```
Asp Ile Val Met Thr Gln Ser Pro Asp Ser Leu Ala Val Ser Leu Gly
 1               5                   10                  15
Glu Arg Val Thr Ile Asn Cys Lys Ser Ser Gln Ser Leu Leu Asn Ser
             20                  25                  30
Gly Asn Gln Gln Asn Tyr Leu Ala Trp Tyr Gln Gln Lys Pro Gly Gln
         35                  40                  45
Pro Pro Lys Leu Leu Ile Tyr Gly Ala Ser Thr Arg Glu Ser Gly Val
     50                  55                  60
Pro Asp Arg Phe Ser Gly Ser Gly Ser Gly Thr Asp Phe Thr Leu Thr
 65                  70                  75                  80
Ile Ser Ser Val Gln Ala Glu Asp Val Ala Val Tyr Tyr Cys Gln Ser
                 85                  90                  95
Asp His Ile Tyr Pro Tyr Thr Phe Gly Gln Gly Thr Lys Leu Glu Ile
                100                 105                 110
Lys
```

<210> 37
<211> 339
<212> DNA
<213> Artificial Sequence

<220>
<221> CDS
<220> (1)..(339)
<223> Description of Artificial Sequence: LV4 sequence

<400> 37

```
gac att gtg atg aca cag tct cca gac tcc ctg gct gtg tca gca gga     48
Asp Ile Val Met Thr Gln Ser Pro Asp Ser Leu Ala Val Ser Ala Gly
 1               5                   10                  15

gag cgg gtc act atg aac tgc aag tcc agt cag agt ctg tta aac agt     96
Glu Arg Val Thr Met Asn Cys Lys Ser Ser Gln Ser Leu Leu Asn Ser
             20                  25                  30

gga aat caa cag aac tac ttg gcc tgg tac cag cag aaa cca ggg cag    144
Gly Asn Gln Gln Asn Tyr Leu Ala Trp Tyr Gln Gln Lys Pro Gly Gln
         35                  40                  45

cct cct aaa ctg ttg atc tac ggg gca tcc act agg gag tct ggg gtc    192
Pro Pro Lys Leu Leu Ile Tyr Gly Ala Ser Thr Arg Glu Ser Gly Val
     50                  55                  60

cct gat cgc ttc tca ggc agt gga tct gga acc gat ttc act ctc acc    240
Pro Asp Arg Phe Ser Gly Ser Gly Ser Gly Thr Asp Phe Thr Leu Thr
 65                  70                  75                  80

atc agc agt gtg cag gct gaa gac gtg gca gtt tat tac tgt cag agt    288
Ile Ser Ser Val Gln Ala Glu Asp Val Ala Val Tyr Tyr Cys Gln Ser
                 85                  90                  95

gat cat att tat ccg tac acg ttc gga cag ggg acc aag ctg gaa ata    336
Asp His Ile Tyr Pro Tyr Thr Phe Gly Gln Gly Thr Lys Leu Glu Ile
                100                 105                 110

aaa                                                                 339
Lys
```

<210> 38
<211> 113
<212> PRT
<213> Artificial Sequence

<220>
<221> PEPTIDE
<220> (1)..(113)
<223> Description of Artificial Sequence: LV4 sequence

<400> 38
Asp Ile Val Met Thr Gln Ser Pro Asp Ser Leu Ala Val Ser Ala Gly
1               5                   10                  15
Glu Arg Val Thr Met Asn Cys Lys Ser Ser Gln Ser Leu Leu Asn Ser
            20                  25                  30
Gly Asn Gln Gln Asn Tyr Leu Ala Trp Tyr Gln Gln Lys Pro Gly Gln
        35                  40                  45
Pro Pro Lys Leu Leu Ile Tyr Gly Ala Ser Thr Arg Glu Ser Gly Val
        50                  55                  60
Pro Asp Arg Phe Ser Gly Ser Gly Ser Gly Thr Asp Phe Thr Leu Thr
65                  70                  75                  80
Ile Ser Ser Val Gln Ala Glu Asp Val Ala Val Tyr Tyr Cys Gln Ser
                85                  90                  95
Asp His Ile Tyr Pro Tyr Thr Phe Gly Gln Gly Thr Lys Leu Glu Ile
            100                 105                 110
Lys

<210> 39
<211> 720
<212> DNA
<213> Artificial Sequence

<220>
<221> CDS
<220> (1)..(720)
<223> Description of Artificial Sequence: FLAG/Fc sequence

<400> 39
tct aga gca gac tac aag gac gac gat gac aag act agt gac aaa act          48
Ser Arg Ala Asp Tyr Lys Asp Asp Asp Asp Lys Thr Ser Asp Lys Thr
  1               5                   10                  15

cac aca tgc cca ccg tgc cca gca cct gaa ctc ctg ggg gga ccg tca          96
His Thr Cys Pro Pro Cys Pro Ala Pro Glu Leu Leu Gly Gly Pro Ser
                20                  25                  30

gtc ttc ctc ttc ccc cca aaa ccc aag gac acc ctc atg atc tcc cgg         144
Val Phe Leu Phe Pro Pro Lys Pro Lys Asp Thr Leu Met Ile Ser Arg
            35                  40                  45

acc cct gag gtc aca tgc gtg gtg gtg gac gtg agc cac gaa gac cct         192
Thr Pro Glu Val Thr Cys Val Val Val Asp Val Ser His Glu Asp Pro
        50                  55                  60

gag gtc aag ttc aac tgg tac gtg gac ggc gtg gag gtg cat aat gcc         240
Glu Val Lys Phe Asn Trp Tyr Val Asp Gly Val Glu Val His Asn Ala
65                  70                  75                  80

aag aca aag ccg cgg gag gag cag tac aac agc acg tac cgt gtg gtc         288
Lys Thr Lys Pro Arg Glu Glu Gln Tyr Asn Ser Thr Tyr Arg Val Val
                85                  90                  95

agc gtc ctc acc gtc ctg cac cag gac tgg ctg aat ggc aag gag tac         336
Ser Val Leu Thr Val Leu His Gln Asp Trp Leu Asn Gly Lys Glu Tyr
            100                 105                 110

aag tgc aag gtc tcc aac aaa gcc ctc cca gcc ccc atc gag aaa acc         384

```
Lys Cys Lys Val Ser Asn Lys Ala Leu Pro Ala Pro Ile Glu Lys Thr
        115                 120                 125

atc tcc aaa gcc aaa ggg cag ccc cga gaa cca cag gtg tac acc ctg        432
Ile Ser Lys Ala Lys Gly Gln Pro Arg Glu Pro Gln Val Tyr Thr Leu
        130                 135                 140

ccc cca tcc cgg gat gag ctg acc aag aac cag gtc agc ctg acc tgc        480
Pro Pro Ser Arg Asp Glu Leu Thr Lys Asn Gln Val Ser Leu Thr Cys
145                 150                 155                 160

ctg gtc aaa ggc ttc tat ccc agc gac atc gcc gtg gag tgg gag agc        528
Leu Val Lys Gly Phe Tyr Pro Ser Asp Ile Ala Val Glu Trp Glu Ser
                165                 170                 175

aat ggg cag ccg gag aac aac tac aag acc acg cct ccc gtg ctg gac        576
Asn Gly Gln Pro Glu Asn Asn Tyr Lys Thr Thr Pro Pro Val Leu Asp
                180                 185                 190

tcc gac ggc tcc ttc ttc ctc tac agc aag ctc acc gtg gac aag agc        624
Ser Asp Gly Ser Phe Phe Leu Tyr Ser Lys Leu Thr Val Asp Lys Ser
                195                 200                 205

agg tgg cag cag ggg aac gtc ttc tca tgc tcc gtg atg cat gag gct        672
Arg Trp Gln Gln Gly Asn Val Phe Ser Cys Ser Val Met His Glu Ala
        210                 215                 220

ctg cac aac cac tac acg cag aag agc ctc tcc ctg tct ccg ggt aaa        720
Leu His Asn His Tyr Thr Gln Lys Ser Leu Ser Leu Ser Pro Gly Lys
225                 230                 235                 240
```

```
<210> 40
<211> 798
<212> DNA
<213> Artificial Sequence

<220>
<221> CDS
<220> (1)..(798)
<223> Description of Artificial Sequence: Trop-2-signal sequence

<400> 40
atg aga gtg ctg att ctt ttg tgg ctg ttc aca gcc ttt cct ggt atc         48
Met Arg Val Leu Ile Leu Leu Trp Leu Phe Thr Ala Phe Pro Gly Ile
  1                 5                   10                  15

ctg tct cac acg gcc gcg cag gac aac tgc acg tgt ccc acc aac aag         96
Leu Ser His Thr Ala Ala Gln Asp Asn Cys Thr Cys Pro Thr Asn Lys
                20                  25                  30

atg acc gtg tgc agc ccc gac ggc ccc ggc ggc cgc tgc cag tgc cgc        144
Met Thr Val Cys Ser Pro Asp Gly Pro Gly Gly Arg Cys Gln Cys Arg
        35                  40                  45

gcg ctg ggc tcg ggc atg gcg gtc gac tgc tcc acg ctg acc tcc aag        192
Ala Leu Gly Ser Gly Met Ala Val Asp Cys Ser Thr Leu Thr Ser Lys
        50                  55                  60

tgt ctg ctg ctc aag gcg cgc atg agc gcc ccc aag aac gcc cgc acg        240
Cys Leu Leu Leu Lys Ala Arg Met Ser Ala Pro Lys Asn Ala Arg Thr
 65                  70                  75                  80

ctg gtg cgg ccg agt gag cac gcg ctc gtg gac aac gat ggc ctc tac        288
```

```
        Leu Val Arg Pro Ser Glu His Ala Leu Val Asp Asn Asp Gly Leu Tyr
                        85                  90                  95


        gac ccc gac tgc gac ccc gag ggc cgc ttc aag gcg cgc cag tgc aac    336
        Asp Pro Asp Cys Asp Pro Glu Gly Arg Phe Lys Ala Arg Gln Cys Asn
                    100                 105                 110

        cag acg tcg gtg tgc tgg tgc gtg aac tcg gtg ggc gtg cgc cgc acg    384
        Gln Thr Ser Val Cys Trp Cys Val Asn Ser Val Gly Val Arg Arg Thr
                115                 120                 125

        gac aag ggc gac ctg agc cta cgc tgc gat gag ctg gtg cgc acc cac    432
        Asp Lys Gly Asp Leu Ser Leu Arg Cys Asp Glu Leu Val Arg Thr His
            130                 135                 140

        cac atc ctc att gac ctg cgc cac cgc ccc acc gcc ggc gcc ttc aac    480
        His Ile Leu Ile Asp Leu Arg His Arg Pro Thr Ala Gly Ala Phe Asn
        145                 150                 155                 160

        cac tca gac ctg gac gcc gag ctg agg cgg ctc ttc cgc gag cgc tat    528
        His Ser Asp Leu Asp Ala Glu Leu Arg Arg Leu Phe Arg Glu Arg Tyr
                        165                 170                 175

        cgg ctg cac ccc aag ttc gtg gcg gcc gtg cac tac gag cag ccc acc    576
        Arg Leu His Pro Lys Phe Val Ala Ala Val His Tyr Glu Gln Pro Thr
                    180                 185                 190

        atc cag atc gag ctg cgg cag aac acg tct cag aag gcc gcc ggt gac    624
        Ile Gln Ile Glu Leu Arg Gln Asn Thr Ser Gln Lys Ala Ala Gly Asp
                195                 200                 205

        gtg gat atc ggc gat gcc gcc tac tac ttc gag agg gac atc aag ggc    672
        Val Asp Ile Gly Asp Ala Ala Tyr Tyr Phe Glu Arg Asp Ile Lys Gly
            210                 215                 220

        gag tct cta ttc cag ggc cgc ggc ggc ctg gac ttg cgc gtg cgc gga    720
        Glu Ser Leu Phe Gln Gly Arg Gly Gly Leu Asp Leu Arg Val Arg Gly
        225                 230                 235                 240

        gaa ccc ctg cag gtg gag cgc acg ctc atc tat tac ctg gac gag att    768
        Glu Pro Leu Gln Val Glu Arg Thr Leu Ile Tyr Tyr Leu Asp Glu Ile
                        245                 250                 255

        ccc ccg aag ttc tcc atg aag cgc ctc acc                            798
        Pro Pro Lys Phe Ser Met Lys Arg Leu Thr
        260         265


        <210> 41
        <211> 681
        <212> DNA
        <213> Artificial Sequence

        <220>
        <221> CDS
        <220> (1)..(681)
        <223> Description of Artificial Sequence

        <400> 41
        atg aga gtg ctg att ctt ttg tgg ctg ttc aca gcc ttt cct ggt atc     48
        Met Arg Val Leu Ile Leu Leu Trp Leu Phe Thr Ala Phe Pro Gly Ile
        1                   5                   10                  15

        ctg tct cac acg gcc gcg cag gac aac tgt ctg ctg ctc aag gcg cgc     96
```

```
Leu Ser His Thr Ala Ala Gln Asp Asn Cys Leu Leu Leu Lys Ala Arg
        20              25              30

atg agc gcc ccc aag aac gcc cgc acg ctg gtg cgg ccg agt gag cac    144
Met Ser Ala Pro Lys Asn Ala Arg Thr Leu Val Arg Pro Ser Glu His
        35              40              45

gcg ctc gtg gac aac gat ggc ctc tac gac ccc gac tgc gac ccc gag    192
Ala Leu Val Asp Asn Asp Gly Leu Tyr Asp Pro Asp Cys Asp Pro Glu
        50              55              60

ggc cgc ttc aag gcg cgc cag tgc aac cag acg tcg gtg tgc tgg tgc    240
Gly Arg Phe Lys Ala Arg Gln Cys Asn Gln Thr Ser Val Cys Trp Cys
 65              70              75              80

gtg aac tcg gtg ggc gtg cgc cgc acg gac aag ggc gac ctg agc cta    288
Val Asn Ser Val Gly Val Arg Arg Thr Asp Lys Gly Asp Leu Ser Leu
                85              90              95

cgc tgc gat gag ctg gtg cgc acc cac cac atc ctc att gac ctg cgc    336
Arg Cys Asp Glu Leu Val Arg Thr His His Ile Leu Ile Asp Leu Arg
            100             105             110

cac cgc ccc acc gcc ggc gcc ttc aac cac tca gac ctg gac gcc gag    384
His Arg Pro Thr Ala Gly Ala Phe Asn His Ser Asp Leu Asp Ala Glu
            115             120             125

ctg agg cgg ctc ttc cgc gag cgc tat cgg ctg cac ccc aag ttc gtg    432
Leu Arg Arg Leu Phe Arg Glu Arg Tyr Arg Leu His Pro Lys Phe Val
        130             135             140

gcg gcc gtg cac tac gag cag ccc acc atc cag atc gag ctg cgg cag    480
Ala Ala Val His Tyr Glu Gln Pro Thr Ile Gln Ile Glu Leu Arg Gln
145             150             155             160

aac acg tct cag aag gcc gcc ggt gac gtg gat atc ggc gat gcc gcc    528
Asn Thr Ser Gln Lys Ala Ala Gly Asp Val Asp Ile Gly Asp Ala Ala
            165             170             175

tac tac ttc gag agg gac atc aag ggc gag tct cta ttc cag ggc cgc    576
Tyr Tyr Phe Glu Arg Asp Ile Lys Gly Glu Ser Leu Phe Gln Gly Arg
            180             185             190

ggc ggc ctg gac ttg cgc gtg cgc gga gaa ccc ctg cag gtg gag cgc    624
Gly Gly Leu Asp Leu Arg Val Arg Gly Glu Pro Leu Gln Val Glu Arg
        195             200             205

acg ctc atc tat tac ctg gac gag att ccc ccg aag ttc tcc atg aag    672
Thr Leu Ile Tyr Tyr Leu Asp Glu Ile Pro Pro Lys Phe Ser Met Lys
        210             215             220

cgc ctc acc                                                         681
Arg Leu Thr
225
```

<210> 42
<211> 462
<212> DNA
<213> Artificial Sequence

<220>
<221> CDS
<220> (1)..(462)

<223> Description of Artificial Sequence

<400> 42
```
atg aga gtg ctg att ctt ttg tgg ctg ttc aca gcc ttt cct ggt atc          48
Met Arg Val Leu Ile Leu Leu Trp Leu Phe Thr Ala Phe Pro Gly Ile
1               5                   10                  15

ctg tct cac acg gcc gcg cag gac aac gat gag ctg gtg cgc acc cac          96
Leu Ser His Thr Ala Ala Gln Asp Asn Asp Glu Leu Val Arg Thr His
                20                  25                  30

cac atc ctc att gac ctg cgc cac cgc ccc acc gcc ggc gcc ttc aac          144
His Ile Leu Ile Asp Leu Arg His Arg Pro Thr Ala Gly Ala Phe Asn
            35                  40                  45

cac tca gac ctg gac gcc gag ctg agg cgg ctc ttc cgc gag cgc tat          192
His Ser Asp Leu Asp Ala Glu Leu Arg Arg Leu Phe Arg Glu Arg Tyr
        50                  55                  60

cgg ctg cac ccc aag ttc gtg gcg gcc gtg cac tac gag cag ccc acc          240
Arg Leu His Pro Lys Phe Val Ala Ala Val His Tyr Glu Gln Pro Thr
65                  70                  75                  80

atc cag atc gag ctg cgg cag aac acg tct cag aag gcc gcc ggt gac          288
Ile Gln Ile Glu Leu Arg Gln Asn Thr Ser Gln Lys Ala Ala Gly Asp
                85                  90                  95

gtg gat atc ggc gat gcc gcc tac tac ttc gag agg gac atc aag ggc          336
Val Asp Ile Gly Asp Ala Ala Tyr Tyr Phe Glu Arg Asp Ile Lys Gly
            100                 105                 110

gag tct cta ttc cag ggc cgc ggc ggc ctg gac ttg cgc gtg cgc gga          384
Glu Ser Leu Phe Gln Gly Arg Gly Gly Leu Asp Leu Arg Val Arg Gly
        115                 120                 125

gaa ccc ctg cag gtg gag cgc acg ctc atc tat tac ctg gac gag att          432
Glu Pro Leu Gln Val Glu Arg Thr Leu Ile Tyr Tyr Leu Asp Glu Ile
    130                 135                 140

ccc ccg aag ttc tcc atg aag cgc ctc acc                                  462
Pro Pro Lys Phe Ser Met Lys Arg Leu Thr
145                 150
```

<210> 43
<211> 363
<212> DNA
<213> Artificial Sequence

<220>
<221> CDS
<220> (1)..(363)
<223> Description of Artificial Sequence

<400> 43
```
atg aga gtg ctg att ctt ttg tgg ctg ttc aca gcc ttt cct ggt atc          48
Met Arg Val Leu Ile Leu Leu Trp Leu Phe Thr Ala Phe Pro Gly Ile
1               5                   10                  15

ctg tct cac acg gcc gcg cag gac aac ctc ttc cgc gag cgc tat cgg          96
Leu Ser His Thr Ala Ala Gln Asp Asn Leu Phe Arg Glu Arg Tyr Arg
                20                  25                  30

cta cac ccc aag ttc gtg gcg gcc gtg cac tac gag cag ccc acc atc          144
```

```
        Leu His Pro Lys Phe Val Ala Ala Val His Tyr Glu Gln Pro Thr Ile
                35                  40                  45


        cag atc gag ctg cgg cag aac acg tct cag aag gcc gcc ggt gac gtg        192
        Gln Ile Glu Leu Arg Gln Asn Thr Ser Gln Lys Ala Ala Gly Asp Val
                50                  55                  60


        gat atc ggc gat gcc gcc tac tac ttc gag agg gac atc aag ggc gag        240
        Asp Ile Gly Asp Ala Ala Tyr Tyr Phe Glu Arg Asp Ile Lys Gly Glu
        65                  70                  75                  80


        tct cta ttc cag ggc cgc ggc ggc ctg gac ttg cgc gtg cgc gga gaa        288
        Ser Leu Phe Gln Gly Arg Gly Gly Leu Asp Leu Arg Val Arg Gly Glu
                        85                  90                  95


        ccc ctg cag gtg gag cgc acg ctc atc tat tac ctg gac gag att ccc        336
        Pro Leu Gln Val Glu Arg Thr Leu Ile Tyr Tyr Leu Asp Glu Ile Pro
                        100                 105                 110


        ccg aag ttc tcc atg aag cgc ctc acc                                    363
        Pro Lys Phe Ser Met Lys Arg Leu Thr
                        115                 120


        <210> 44
        <211> 506
        <212> PRT
        <213> Artificial Sequence


        <220>
        <221> PEPTIDE
        <220> (1)..(506)
        <223> Description of Artificial Sequence: Trop-2/FLAG/Fc sequence


        <400> 44
        Met Arg Val Leu Ile Leu Leu Trp Leu Phe Thr Ala Phe Pro Gly Ile
        1               5                   10                  15
        Leu Ser His Thr Ala Ala Gln Asp Asn Cys Thr Cys Pro Thr Asn Lys
                        20                  25                  30
        Met Thr Val Cys Ser Pro Asp Gly Pro Gly Gly Arg Cys Gln Cys Arg
                        35                  40                  45
        Ala Leu Gly Ser Gly Met Ala Val Asp Cys Ser Thr Leu Thr Ser Lys
                50                  55                  60
        Cys Leu Leu Leu Lys Ala Arg Met Ser Ala Pro Lys Asn Ala Arg Thr
        65                  70                  75                  80
        Leu Val Arg Pro Ser Glu His Ala Leu Val Asp Asn Asp Gly Leu Tyr
                        85                  90                  95
        Asp Pro Asp Cys Asp Pro Glu Gly Arg Phe Lys Ala Arg Gln Cys Asn
                        100                 105                 110
        Gln Thr Ser Val Cys Trp Cys Val Asn Ser Val Gly Val Arg Arg Thr
                        115                 120                 125
        Asp Lys Gly Asp Leu Ser Leu Arg Cys Asp Glu Leu Val Arg Thr His
                130                 135                 140
        His Ile Leu Ile Asp Leu Arg His Arg Pro Thr Ala Gly Ala Phe Asn
        145                 150                 155                 160
        His Ser Asp Leu Asp Ala Glu Leu Arg Arg Leu Phe Arg Glu Arg Tyr
                        165                 170                 175
        Arg Leu His Pro Lys Phe Val Ala Ala Val His Tyr Glu Gln Pro Thr
                        180                 185                 190
        Ile Gln Ile Glu Leu Arg Gln Asn Thr Ser Gln Lys Ala Ala Gly Asp
                        195                 200                 205
        Val Asp Ile Gly Asp Ala Ala Tyr Tyr Phe Glu Arg Asp Ile Lys Gly
                210                 215                 220
        Glu Ser Leu Phe Gln Gly Arg Gly Gly Leu Asp Leu Arg Val Arg Gly
```

```
225                   230                   235                   240
Glu Pro Leu Gln Val Glu Arg Thr Leu Ile Tyr Tyr Leu Asp Glu Ile
                245                   250                   255
Pro Pro Lys Phe Ser Met Lys Arg Leu Thr Ser Arg Ala Asp Tyr Lys
                260                   265                   270
Asp Asp Asp Asp Lys Thr Ser Asp Lys Thr His Thr Cys Pro Pro Cys
                275                   280                   285
Pro Ala Pro Glu Leu Leu Gly Gly Pro Ser Val Phe Leu Phe Pro Pro
        290                   295                   300
Lys Pro Lys Asp Thr Leu Met Ile Ser Arg Thr Pro Glu Val Thr Cys
305                   310                   315                   320
Val Val Val Asp Val Ser His Glu Asp Pro Glu Val Lys Phe Asn Trp
                325                   330                   335
Tyr Val Asp Gly Val Glu Val His Asn Ala Lys Thr Lys Pro Arg Glu
                340                   345                   350
Glu Gln Tyr Asn Ser Thr Tyr Arg Val Val Ser Val Leu Thr Val Leu
                355                   360                   365
His Gln Asp Trp Leu Asn Gly Lys Glu Tyr Lys Cys Lys Val Ser Asn
        370                   375                   380
Lys Ala Leu Pro Ala Pro Ile Glu Lys Thr Ile Ser Lys Ala Lys Gly
        385                   390                   395                   400
Gln Pro Arg Glu Pro Gln Val Tyr Thr Leu Pro Pro Ser Arg Asp Glu
                405                   410                   415
Leu Thr Lys Asn Gln Val Ser Leu Thr Cys Leu Val Lys Gly Phe Tyr
                420                   425                   430
Pro Ser Asp Ile Ala Val Glu Trp Glu Ser Asn Gly Gln Pro Glu Asn
                435                   440                   445
Asn Tyr Lys Thr Thr Pro Pro Val Leu Asp Ser Asp Gly Ser Phe Phe
        450                   455                   460
Leu Tyr Ser Lys Leu Thr Val Asp Lys Ser Arg Trp Gln Gln Gly Asn
465                   470                   475                   480
Val Phe Ser Cys Ser Val Met His Glu Ala Leu His Asn His Tyr Thr
                485                   490                   495
Gln Lys Ser Leu Ser Leu Ser Pro Gly Lys
                500                   505
```

<210> 45
<211> 467
<212> PRT
<213> Artificial Sequence

<220>
<221> PEPTIDE
<220> (1)..(467)
<223> Description of Artificial Sequence: mutant A sequence

<400> 45

```
Met Arg Val Leu Ile Leu Leu Trp Leu Phe Thr Ala Phe Pro Gly Ile
1               5                   10                  15
Leu Ser His Thr Ala Ala Gln Asp Asn Cys Leu Leu Leu Lys Ala Arg
            20                  25                  30
Met Ser Ala Pro Lys Asn Ala Arg Thr Leu Val Arg Pro Ser Glu His
        35                  40                  45
Ala Leu Val Asp Asn Asp Gly Leu Tyr Asp Pro Asp Cys Asp Pro Glu
        50                  55                  60
Gly Arg Phe Lys Ala Arg Gln Cys Asn Gln Thr Ser Val Cys Trp Cys
65                  70                  75                  80
Val Asn Ser Val Gly Val Arg Arg Thr Asp Lys Gly Asp Leu Ser Leu
                85                  90                  95
Arg Cys Asp Glu Leu Val Arg Thr His His Ile Leu Ile Asp Leu Arg
            100                 105                 110
His Arg Pro Thr Ala Gly Ala Phe Asn His Ser Asp Leu Asp Ala Glu
            115                 120                 125
```

```
Leu Arg Arg Leu Phe Arg Glu Arg Tyr Arg Leu His Pro Lys Phe Val
    130                 135             140
Ala Ala Val His Tyr Glu Gln Pro Thr Ile Gln Ile Glu Leu Arg Gln
145                 150             155                 160
Asn Thr Ser Gln Lys Ala Ala Gly Asp Val Asp Ile Gly Asp Ala Ala
                165             170             175
Tyr Tyr Phe Glu Arg Asp Ile Lys Gly Glu Ser Leu Phe Gln Gly Arg
            180             185             190
Gly Gly Leu Asp Leu Arg Val Arg Gly Glu Pro Leu Gln Val Glu Arg
            195             200             205
Thr Leu Ile Tyr Tyr Leu Asp Glu Ile Pro Pro Lys Phe Ser Met Lys
    210             215             220
Arg Leu Thr Ser Arg Ala Asp Tyr Lys Asp Asp Asp Lys Thr Ser
225             230             235             240
Asp Lys Thr His Thr Cys Pro Pro Cys Pro Ala Pro Glu Leu Leu Gly
                245             250             255
Gly Pro Ser Val Phe Leu Phe Pro Pro Lys Pro Lys Asp Thr Leu Met
            260             265             270
Ile Ser Arg Thr Pro Glu Val Thr Cys Val Val Val Asp Val Ser His
        275             280             285
Glu Asp Pro Glu Val Lys Phe Asn Trp Tyr Val Asp Gly Val Glu Val
    290             295             300
His Asn Ala Lys Thr Lys Pro Arg Glu Glu Gln Tyr Asn Ser Thr Tyr
305             310             315                 320
Arg Val Val Ser Val Leu Thr Val Leu His Gln Asp Trp Leu Asn Gly
            325             330             335
Lys Glu Tyr Lys Cys Lys Val Ser Asn Lys Ala Leu Pro Ala Pro Ile
            340             345             350
Glu Lys Thr Ile Ser Lys Ala Lys Gly Gln Pro Arg Glu Pro Gln Val
        355             360             365
Tyr Thr Leu Pro Pro Ser Arg Asp Glu Leu Thr Lys Asn Gln Val Ser
    370             375             380
Leu Thr Cys Leu Val Lys Gly Phe Tyr Pro Ser Asp Ile Ala Val Glu
385             390             395                 400
Trp Glu Ser Asn Gly Gln Pro Glu Asn Asn Tyr Lys Thr Thr Pro Pro
            405             410             415
Val Leu Asp Ser Asp Gly Ser Phe Phe Leu Tyr Ser Lys Leu Thr Val
            420             425             430
Asp Lys Ser Arg Trp Gln Gln Gly Asn Val Phe Ser Cys Ser Val Met
    435             440             445
His Glu Ala Leu His Asn His Tyr Thr Gln Lys Ser Leu Ser Leu Ser
    450             455             460
Pro Gly Lys
465
```

```
<210> 46
<211> 394
<212> PRT
<213> Artificial Sequence

<220>
<221> PEPTIDE
<220> (1)..(394)
<223> Description of Artificial Sequence: mutant B sequence

<400> 46
Met Arg Val Leu Ile Leu Leu Trp Leu Phe Thr Ala Phe Pro Gly Ile
  1           5               10              15
Leu Ser His Thr Ala Ala Gln Asp Asn Asp Glu Leu Val Arg Thr His
            20              25              30
His Ile Leu Ile Asp Leu Arg His Arg Pro Thr Ala Gly Ala Phe Asn
        35              40              45
His Ser Asp Leu Asp Ala Glu Leu Arg Arg Leu Phe Arg Glu Arg Tyr
```

```
                50                      55                      60
Arg Leu His Pro Lys Phe Val Ala Ala Val His Tyr Glu Gln Pro Thr
65                      70                      75                      80
Ile Gln Ile Glu Leu Arg Gln Asn Thr Ser Gln Lys Ala Ala Gly Asp
                        85                      90                      95
Val Asp Ile Gly Asp Ala Ala Tyr Tyr Phe Glu Arg Asp Ile Lys Gly
                100                     105                     110
Glu Ser Leu Phe Gln Gly Arg Gly Gly Leu Asp Leu Arg Val Arg Gly
                115                     120                     125
Glu Pro Leu Gln Val Glu Arg Thr Leu Ile Tyr Tyr Leu Asp Glu Ile
        130                     135                     140
Pro Pro Lys Phe Ser Met Lys Arg Leu Thr Ser Arg Ala Asp Tyr Lys
145                     150                     155                     160
Asp Asp Asp Asp Lys Thr Ser Asp Lys Thr His Thr Cys Pro Pro Cys
                165                     170                     175
Pro Ala Pro Glu Leu Leu Gly Gly Pro Ser Val Phe Leu Phe Pro Pro
                180                     185                     190
Lys Pro Lys Asp Thr Leu Met Ile Ser Arg Thr Pro Glu Val Thr Cys
                195                     200                     205
Val Val Asp Val Ser His Glu Asp Pro Glu Val Lys Phe Asn Trp
        210                     215                     220
Tyr Val Asp Gly Val Glu Val His Asn Ala Lys Thr Lys Pro Arg Glu
225                     230                     235                     240
Glu Gln Tyr Asn Ser Thr Tyr Arg Val Val Ser Val Leu Thr Val Leu
                245                     250                     255
His Gln Asp Trp Leu Asn Gly Lys Glu Tyr Lys Cys Lys Val Ser Asn
                260                     265                     270
Lys Ala Leu Pro Ala Pro Ile Glu Lys Thr Ile Ser Lys Ala Lys Gly
        275                     280                     285
Gln Pro Arg Glu Pro Gln Val Tyr Thr Leu Pro Pro Ser Arg Asp Glu
        290                     295                     300
Leu Thr Lys Asn Gln Val Ser Leu Thr Cys Leu Val Lys Gly Phe Tyr
305                     310                     315                     320
Pro Ser Asp Ile Ala Val Glu Trp Glu Ser Asn Gly Gln Pro Glu Asn
                325                     330                     335
Asn Tyr Lys Thr Thr Pro Pro Val Leu Asp Ser Asp Gly Ser Phe Phe
                340                     345                     350
Leu Tyr Ser Lys Leu Thr Val Asp Lys Ser Arg Trp Gln Gln Gly Asn
                355                     360                     365
Val Phe Ser Cys Ser Val Met His Glu Ala Leu His Asn His Tyr Thr
        370                     375                     380
Gln Lys Ser Leu Ser Leu Ser Pro Gly Lys
385                     390
```

```
<210> 47
<211> 361
<212> PRT
<213> Artificial Sequence

<220>
<221> PEPTIDE
<220> (1)..(361)
<223> Description of Artificial Sequence: mutant C sequence

<400> 47
Met Arg Val Leu Ile Leu Leu Trp Leu Phe Thr Ala Phe Pro Gly Ile
1               5                       10                      15
Leu Ser His Thr Ala Ala Gln Asp Asn Leu Phe Arg Glu Arg Tyr Arg
                20                      25                      30
Leu His Pro Lys Phe Val Ala Ala Val His Tyr Glu Gln Pro Thr Ile
        35                      40                      45
Gln Ile Glu Leu Arg Gln Asn Thr Ser Gln Lys Ala Ala Gly Asp Val
        50                      55                      60
```

```
Asp Ile Gly Asp Ala Ala Tyr Tyr Phe Glu Arg Asp Ile Lys Gly Glu
65              70              75              80
Ser Leu Phe Gln Gly Arg Gly Gly Leu Asp Leu Arg Val Arg Gly Glu
                85              90              95
Pro Leu Gln Val Glu Arg Thr Leu Ile Tyr Tyr Leu Asp Glu Ile Pro
            100             105             110
Pro Lys Phe Ser Met Lys Arg Leu Thr Ser Arg Ala Asp Tyr Lys Asp
        115             120             125
Asp Asp Asp Lys Thr Ser Asp Lys Thr His Thr Cys Pro Pro Cys Pro
        130             135             140
Ala Pro Glu Leu Leu Gly Gly Pro Ser Val Phe Leu Phe Pro Pro Lys
145             150             155             160
Pro Lys Asp Thr Leu Met Ile Ser Arg Thr Pro Glu Val Thr Cys Val
                165             170             175
Val Val Asp Val Ser His Glu Asp Pro Glu Val Lys Phe Asn Trp Tyr
            180             185             190
Val Asp Gly Val Glu Val His Asn Ala Lys Thr Lys Pro Arg Glu Glu
            195             200             205
Gln Tyr Asn Ser Thr Tyr Arg Val Val Ser Val Leu Thr Val Leu His
    210             215             220
Gln Asp Trp Leu Asn Gly Lys Glu Tyr Lys Cys Lys Val Ser Asn Lys
225             230             235             240
Ala Leu Pro Ala Pro Ile Glu Lys Thr Ile Ser Lys Ala Lys Gly Gln
                245             250             255
Pro Arg Glu Pro Gln Val Tyr Thr Leu Pro Pro Ser Arg Asp Glu Leu
            260             265             270
Thr Lys Asn Gln Val Ser Leu Thr Cys Leu Val Lys Gly Phe Tyr Pro
            275             280             285
Ser Asp Ile Ala Val Glu Trp Glu Ser Asn Gly Gln Pro Glu Asn Asn
        290             295             300
Tyr Lys Thr Thr Pro Pro Val Leu Asp Ser Asp Gly Ser Phe Phe Leu
305             310             315             320
Tyr Ser Lys Leu Thr Val Asp Lys Ser Arg Trp Gln Gln Gly Asn Val
                325             330             335
Phe Ser Cys Ser Val Met His Glu Ala Leu His Asn His Tyr Thr Gln
                340             345             350
Lys Ser Leu Ser Leu Ser Pro Gly Lys
        355             360
```

```
<210> 48
<211> 344
<212> PRT
<213> Artificial Sequence

<220>
<221> PEPTIDE
<220> (1)..(344)
<223> Description of Artificial Sequence: myc/His-tagged Trop-2 sequence

<400> 48
Met Ala Arg Gly Pro Gly Leu Ala Pro Pro Pro Leu Arg Leu Pro Leu
1               5               10              15
Leu Leu Leu Val Leu Ala Ala Val Thr Gly His Thr Ala Ala Gln Asp
            20              25              30
Asn Cys Thr Cys Pro Thr Asn Lys Met Thr Val Cys Ser Pro Asp Gly
        35              40              45
Pro Gly Gly Arg Cys Gln Cys Arg Ala Leu Gly Ser Gly Met Ala Val
    50              55              60
Asp Cys Ser Thr Leu Thr Ser Lys Cys Leu Leu Leu Lys Ala Arg Met
65              70              75              80
Ser Ala Pro Lys Asn Ala Arg Thr Leu Val Arg Pro Ser Glu His Ala
                85              90              95
Leu Val Asp Asn Asp Gly Leu Tyr Asp Pro Asp Cys Asp Pro Glu Gly
```

```
            100                    105                    110
Arg Phe Lys Ala Arg Gln Cys Asn Gln Thr Ser Val Cys Trp Cys Val
        115                    120                    125
Asn Ser Val Gly Val Arg Arg Thr Asp Lys Gly Asp Leu Ser Leu Arg
    130                    135                    140
Cys Asp Glu Leu Val Arg Thr His His Ile Leu Ile Asp Leu Arg His
145                    150                    155                    160
Arg Pro Thr Ala Gly Ala Phe Asn His Ser Asp Leu Asp Ala Glu Leu
                165                    170                    175
Arg Arg Leu Phe Arg Glu Arg Tyr Arg Leu His Pro Lys Phe Val Ala
                180                    185                    190
Ala Val His Tyr Glu Gln Pro Thr Ile Gln Ile Glu Leu Arg Gln Asn
            195                    200                    205
Thr Ser Gln Lys Ala Ala Gly Asp Val Asp Ile Gly Asp Ala Ala Tyr
    210                    215                    220
Tyr Phe Glu Arg Asp Ile Lys Gly Glu Ser Leu Phe Gln Gly Arg Gly
225                    230                    235                    240
Gly Leu Asp Leu Arg Val Arg Gly Glu Pro Leu Gln Val Glu Arg Thr
                245                    250                    255
Leu Ile Tyr Tyr Leu Asp Glu Ile Pro Pro Lys Phe Ser Met Lys Arg
                260                    265                    270
Leu Thr Ala Gly Leu Ile Ala Val Ile Val Val Val Val Val Ala Leu
            275                    280                    285
Val Ala Gly Met Ala Val Leu Val Ile Thr Asn Arg Arg Lys Ser Gly
    290                    295                    300
Lys Tyr Lys Lys Val Glu Ile Lys Glu Leu Gly Glu Leu Arg Lys Glu
305                    310                    315                    320
Pro Ser Leu Glu Gln Lys Leu Ile Ser Glu Glu Asp Leu Asn Met His
                325                    330                    335
Thr Gly His His His His His His
                340


<210> 49
<211> 1032
<212> DNA
<213> Artificial Sequence

<220>
<221> CDS
<220> (1)..(1032)
<223> Description of Artificial Sequence: myc/His-tagged Trop-2 sequence

<400> 49
atg gct cgg ggc ccc ggc ctc gcg ccg cca ccg ctg cgg ctg ccg ctg       48
Met Ala Arg Gly Pro Gly Leu Ala Pro Pro Pro Leu Arg Leu Pro Leu
  1               5                  10                  15

ctg ctg ctg gtg ctg gcg gcg gtg acc ggc cac acg gcc gcg cag gac       96
Leu Leu Leu Val Leu Ala Ala Val Thr Gly His Thr Ala Ala Gln Asp
                20                  25                  30

aac tgc acg tgt ccc acc aac aag atg acc gtg tgc agc ccc gac ggc      144
Asn Cys Thr Cys Pro Thr Asn Lys Met Thr Val Cys Ser Pro Asp Gly
            35                  40                  45

ccc ggc ggc cgc tgc cag tgc cgc gcg ctg ggc tcg ggc atg gcg gtc      192
Pro Gly Gly Arg Cys Gln Cys Arg Ala Leu Gly Ser Gly Met Ala Val
        50                  55                  60

gac tgc tcc acg ctg acc tcc aag tgt ctg ctg ctc aag gcg cgc atg      240
Asp Cys Ser Thr Leu Thr Ser Lys Cys Leu Leu Leu Lys Ala Arg Met
    65                  70                  75                  80
```

```
agc gcc ccc aag aac gcc cgc acg ctg gtg cgg ccg agt gag cac gcg        288
Ser Ala Pro Lys Asn Ala Arg Thr Leu Val Arg Pro Ser Glu His Ala
            85              90              95

ctc gtg gac aac gat ggc ctc tac gac ccc gac tgc gac ccc gag ggc        336
Leu Val Asp Asn Asp Gly Leu Tyr Asp Pro Asp Cys Asp Pro Glu Gly
            100             105             110

cgc ttc aag gcg cgc cag tgc aac cag acg tcg gtg tgc tgg tgc gtg        384
Arg Phe Lys Ala Arg Gln Cys Asn Gln Thr Ser Val Cys Trp Cys Val
            115             120             125

aac tcg gtg ggc gtg cgc cgc acg gac aag ggc gac ctg agc cta cgc        432
Asn Ser Val Gly Val Arg Arg Thr Asp Lys Gly Asp Leu Ser Leu Arg
    130             135             140

tgc gat gag ctg gtg cgc acc cac cac atc ctc att gac ctg cgc cac        480
Cys Asp Glu Leu Val Arg Thr His His Ile Leu Ile Asp Leu Arg His
145             150             155             160

cgc ccc acc gcc ggc gcc ttc aac cac tca gac ctg gac gcc gag ctg        528
Arg Pro Thr Ala Gly Ala Phe Asn His Ser Asp Leu Asp Ala Glu Leu
            165             170             175

agg cgg ctc ttc cgc gag cgc tat cgg ctg cac ccc aag ttc gtg gcg        576
Arg Arg Leu Phe Arg Glu Arg Tyr Arg Leu His Pro Lys Phe Val Ala
            180             185             190

gcc gtg cac tac gag cag ccc acc atc cag atc gag ctg cgg cag aac        624
Ala Val His Tyr Glu Gln Pro Thr Ile Gln Ile Glu Leu Arg Gln Asn
            195             200             205

acg tct cag aag gcc gcc ggt gac gtg gat atc ggc gat gcc gcc tac        672
Thr Ser Gln Lys Ala Ala Gly Asp Val Asp Ile Gly Asp Ala Ala Tyr
    210             215             220

tac ttc gag agg gac atc aag ggc gag tct cta ttc cag ggc cgc ggc        720
Tyr Phe Glu Arg Asp Ile Lys Gly Glu Ser Leu Phe Gln Gly Arg Gly
225             230             235             240

ggc ctg gac ttg cgc gtg cgc gga gaa ccc ctg cag gtg gag cgc acg        768
Gly Leu Asp Leu Arg Val Arg Gly Glu Pro Leu Gln Val Glu Arg Thr
            245             250             255

ctc atc tat tac ctg gac gag att ccc ccg aag ttc tcc atg aag cgc        816
Leu Ile Tyr Tyr Leu Asp Glu Ile Pro Pro Lys Phe Ser Met Lys Arg
            260             265             270

ctc acc gcc ggc ctc atc gct gtc atc gtg gtg gtc gtg gtg gcc ctc        864
Leu Thr Ala Gly Leu Ile Ala Val Ile Val Val Val Val Val Ala Leu
            275             280             285

gtc gcc ggc atg gcc gtc ctg gtg atc acc aac cgg aga aag tcg ggg        912
Val Ala Gly Met Ala Val Leu Val Ile Thr Asn Arg Arg Lys Ser Gly
            290             295             300

aag tac aag aag gtg gag atc aag gaa ctg ggg gag ttg aga aag gaa        960
Lys Tyr Lys Lys Val Glu Ile Lys Glu Leu Gly Glu Leu Arg Lys Glu
305             310             315             320

ccg agc ttg gaa caa aaa ctc atc tca gaa gag gat ctg aat atg cat        1008
Pro Ser Leu Glu Gln Lys Leu Ile Ser Glu Glu Asp Leu Asn Met His
            325             330             335
```

78

```
acc ggt cat cat cac cat cac cat                                              1032
Thr Gly His His His His His His
              340
```